# EUROPEAN PATENT SPECIFICATION

(11) **EP 4 148 057 B1**
(45) Date of publication and mention of the grant of the patent: **26.11.2025**
(21) Application number: 22187114.8
(22) Date of filing: 27.07.2022
(51) Int. Cl.: C07D 491/048, H10K 50/15, H10K 85/60

(54) **BENZOFUROCARBAZOLE COMPOUND AND ORGANIC LIGHT EMITTING DEVICE**
BENZOFUROCATBAZOL-VERBINDUNG UND ORGANISCHE LICHTEMITTIERENDE VORRICHTUNG
COMPOSÉ DE BENZOFUROCARBAZOLE ET DISPOSITIF ÉLECTROLUMINESCENT ORGANIQUE

(30) Priority: 08.09.2021 KR 20210119582
(43) Date of publication of application: 15.03.2023
(73) Proprietor: LT Materials Co., Ltd., Yongin-City 17118 (KR)
(72) Inventor: KIM, Su-Yeon, 17118 Yongin-City, Gyeonggi-Do (KR); KIM, Na-Yeong, 17118 Yongin-City, Gyeonggi-Do (KR); HAM, Hyo-Kyun, 17118 Yongin-City, Gyeonggi-Do (KR); JEONG, Won-Jang, 17118 Yongin-City, Gyeonggi-Do (KR); KIM, Dong-Jun, 17118 Yongin-City, Gyeonggi-Do (KR)
(74) Representative: Goddar, Heinz J.

(56) References cited:
- WO-A1-2015/126081
- WO-A1-2018/169260
- CN-A- 108 341 795
- CN-A- 110 317 206
- JP-B2- 5 499 972
- JP-B2- 5 868 652
- US-A1- 2018 047 914

## Description

### TECHNICAL FIELD

The present application relates to a heterocyclic compound and an organic light emitting device using the same.

### BACKGROUND ART

An electroluminescence device is a kind of selfemitting type display device, and has an advantage in that the viewing angle is wide, the contrast is excellent, and the response speed is fast.

An organic light emitting device has a structure in which an organic thin film is disposed between two electrodes. When a voltage is applied to an organic light emitting device having the structure, electrons and holes injected from the two electrodes combine with each other in an organic thin film to make a pair, and then, emit light while being extinguished. The organic thin film may be composed of a single layer or multi layers, if necessary.

A material for the organic thin film may have a light emitting function, if necessary. For example, as the material for the organic thin film, it is also possible to use a compound, which may itself constitute a light emitting layer alone, or it is also possible to use a compound, which may serve as a host or a dopant of a hostdopant-based light emitting layer. In addition, as a material for the organic thin film, it is also possible to use a compound, which may perform a function such as hole injection, hole transport, electron blocking, hole blocking, electron transport or electron injection.

In order to improve the performance, service life, or efficiency of the organic light emitting device, there is a continuous need for developing a material for an organic thin film.

### [Related Art Document]

### [Patent Document]

US Patent No. 4,356,429
US 2018/047914 A1 discloses a compound represented by the following Chemical Formula 1: 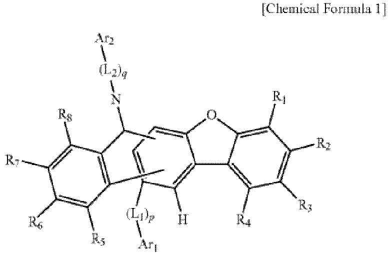
wherein, in Chemical Formula 1, Ar1 is a substituted or unsubstituted biphenyl group; a substituted or unsubstituted terphenyl group; a substituted or unsubstituted quaterphenyl group; a substituted or unsubstituted multicyclic aryl group; a substituted or unsubstituted arylamine group; or a substituted or unsubstituted heterocyclic group;L1 and L2 are the same as or different from each other, and
each independently a direct bond; a substituted or unsubstituted arylene group; or a substituted or unsubstituted heteroarylene group;p and q are the same as or different from each other, and each independently an integer of 0 to 5; when p and q are 2 or greater, structures in the parentheses are the same as or different from each other; and Ar2 and R1 to R8 are the same as or different from each other, and each independently hydrogen; deuterium; a halogen group; a nitrile group; a nitro group; a hydroxyl group; a carbonyl group; an ester group; an imide group; an amino group; a substituted or unsubstituted silyl group; a substituted or unsubstituted alkyl group; a substituted or unsubstituted cycloalkyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted alkylsulfoxy group; a substituted or unsubstituted arylsulfoxy group; a substituted or unsubstituted alkenyl group; a substituted or unsubstituted aralkyl group; a substituted or unsubstituted aralkenyl group; a substituted or unsubstituted alkylaryl group; a substituted or unsubstituted aralkylamine group; a substituted or unsubstituted heteroarylamine group; a substituted or unsubstituted arylamine group; a substituted or unsubstituted arylphosphine group; a substituted or unsubstituted phosphine oxide group; a substituted or unsubstituted aryl group; or a substituted or unsubstituted heterocyclic group, or adjacent groups bond to each other to form a substituted or unsubstituted ring.
WO 2015/126081 A1 discloses an organic optoelectric device comprising: an anode; a cathode facing the anode; a light emitting layer positioned between the anode and the cathode; a hole transport layer positioned between the anode and the light emitting layer; and a hole transport assist layer positioned between the hole transport layer and the light emitting layer, wherein the light emitting layer comprises at least one first compound represented by chemical formula 1 and at least one second compound represented by chemical formula 2, and the hole transport assist layer comprises a third compound represented by a combination of a moiety represented by chemical formula 3, a moiety represented by chemical formula 4 and a moiety represented by chemical formula 5.

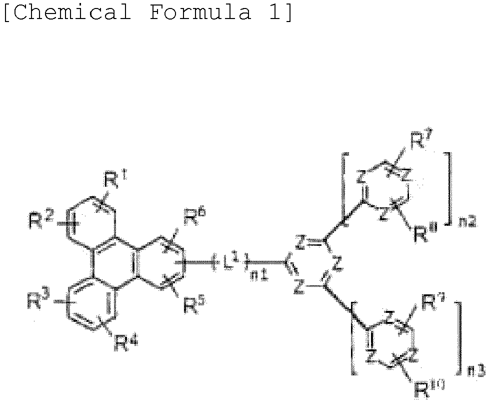

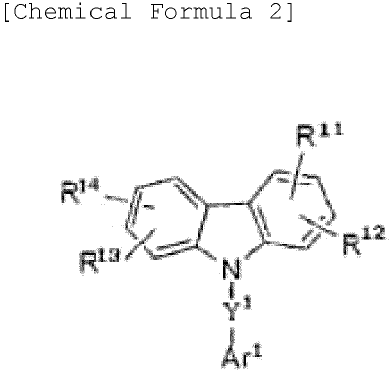

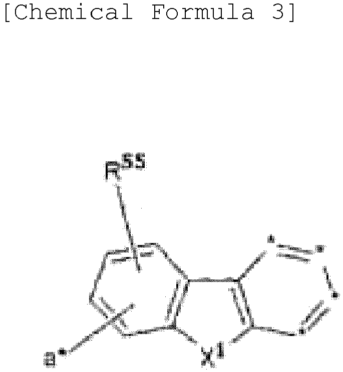

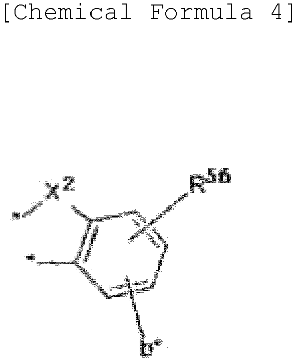

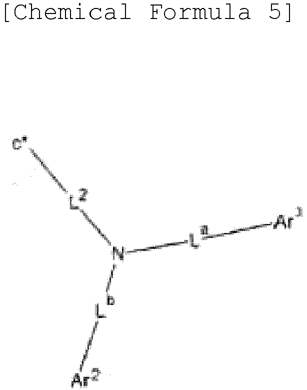

### SUMMARY OF THE INVENTION

The present invention has been made in an effort to provide a heterocyclic compound and an organic light emitting device including the same.

An exemplary embodiment of the present application provides a heterocyclic compound represented by any one of the following Chemical Formulae 2 to 9. 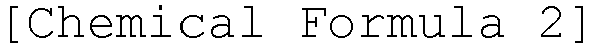 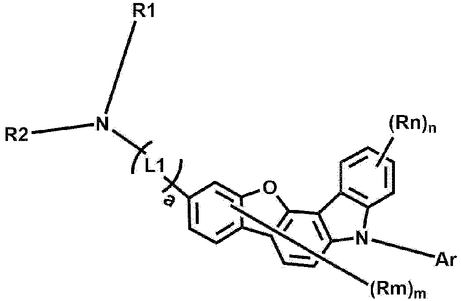 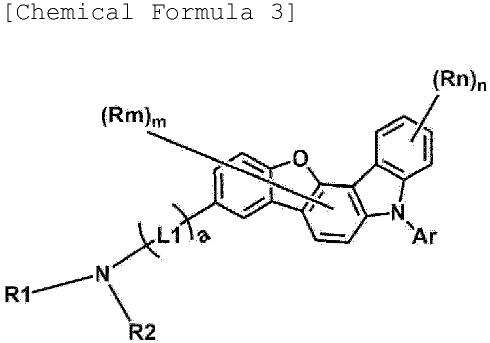 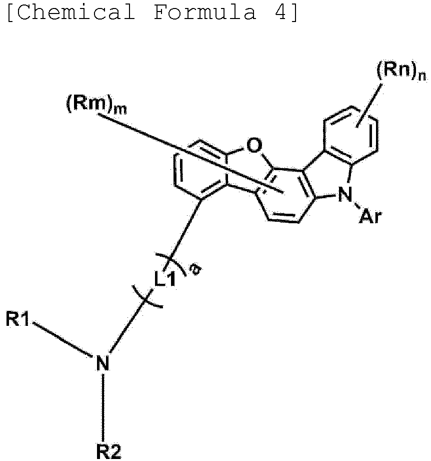 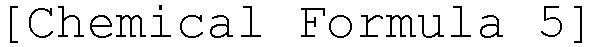 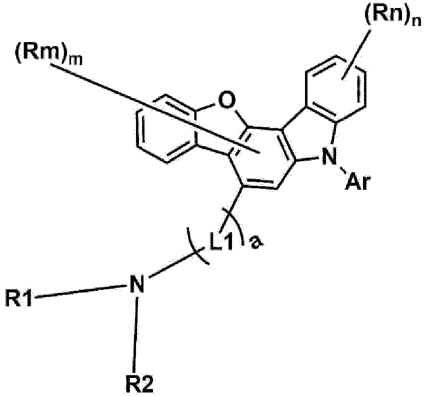 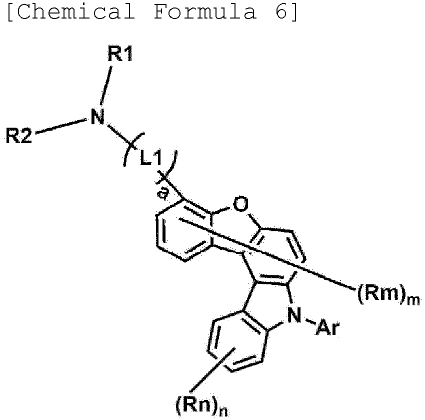 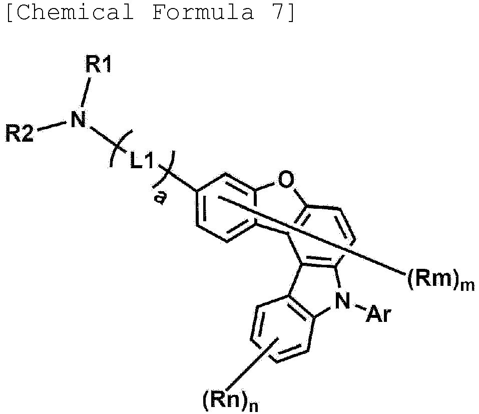 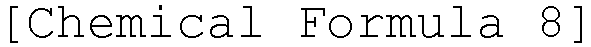 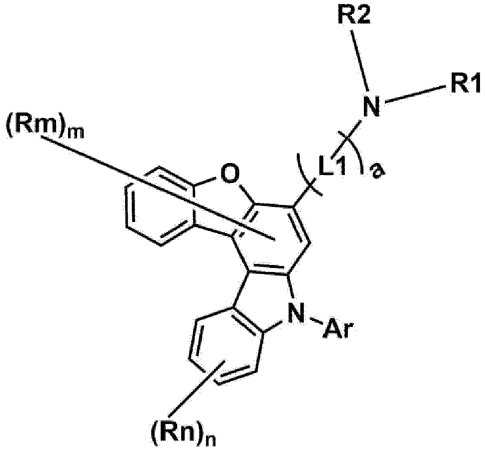 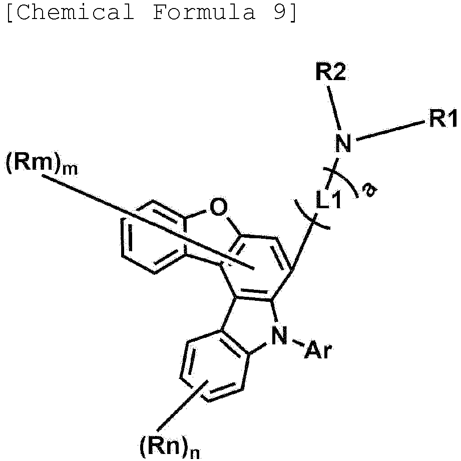 In Chemical Formulae 2 to 9,
L1 is a direct bond; or a substituted or unsubstituted arylene group having 6 to 60 carbon atoms, a is an integer from 0 to 3, and when a is 2 or higher, L1's in the parenthesis are the same as or different from each other,
R1 is a phenyl group unsubstituted or substituted with one or more deuteriums; a biphenyl group unsubstituted or substituted with one or more deuteriums; a terphenyl group unsubstituted or substituted with one or more deuteriums; a naphthyl group unsubstituted or substituted with one or more deuteriums; a phenanthrene group unsubstituted or substituted with one or more deuteriums; a pyrene group; a fluorene group unsubstituted or substituted with one or more substituents selected from the group consisting of deuterium and a methyl group; a triphenylenyl group unsubstituted or substituted with one or more deuteriums; a dibenzofuran group unsubstituted or substituted with one or more deuteriums; or a dibenzothiophene group unsubstituted or substituted with one or more deuteriums,
R2 is a phenyl group unsubstituted or substituted with one or more deuteriums; a biphenyl group unsubstituted or substituted with one or more deuteriums; a terphenyl group unsubstituted or substituted with one or more deuteriums; a naphthyl group unsubstituted or substituted with one or more deuteriums; a phenanthrene group unsubstituted or substituted with one or more deuteriums; a dibenzofuran group unsubstituted or substituted with one or more deuteriums; or a dibenzothiophene group unsubstituted or substituted with one or more deuteriums,
Rm and Rn are the same as or different from each other, and are each hydrogen; or deuterium,
m is an integer from 0 to 5, and when m is 2 or higher, Rm's in the parenthesis are the same as or different from each other,
n is an integer from 0 to 4, and when n is 2 or higher, Rn's in the parenthesis are the same as or different from each other, and
Ar is a substituted or unsubstituted aryl group having 6 to 60 carbon atoms.

An exemplary embodiment of the present application provides an organic light emitting device including a first electrode, a second electrode and an organic material layer having one or more layers provided between the first electrode and the second electrode, in which one or more layers of the organic material layer include the heterocyclic compound of the invention.

A heterocyclic compound according to an exemplary embodiment of the present application can be used as a material for an organic material layer of an organic light emitting device. The heterocyclic compound can be used as a material for a hole injection layer, a hole transport layer, a light emitting layer, an electron transport layer, an electron injection layer, a charge generation layer, and the like in an organic light emitting device. In particular, the heterocyclic compound represented by any of Chemical Formulae 2 to 9 can be used as a material for a hole transport layer or electron blocking layer of an organic light emitting device. In addition, when the heterocyclic compound represented by any of Chemical Formulae 2 to 9 is used for an organic light emitting device, the driving voltage of the device can be lowered, the light efficiency of the device can be improved, and the service life characteristics of the device can be improved due to the thermal stability of the compound.
Further embodiments are disclosed in the dependent claims.

### BRIEF DESCRIPTION OF THE DRAWINGS

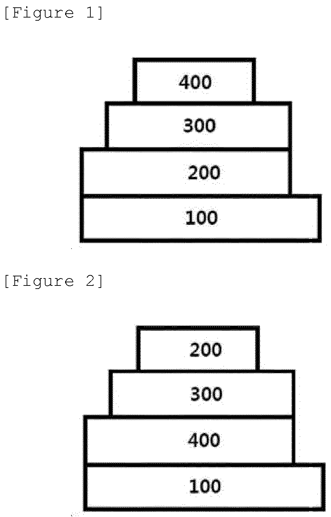

FIGS. 1 to 3 each are views schematically illustrating a stacking structure of an organic light emitting device according to an exemplary embodiment of the present application.

### DETAILED DESCRIPTION

Hereinafter, the present application will be described in detail.

The present application provides a heterocyclic compound represented by any one of the following Chemical Formulae 2 to 9.

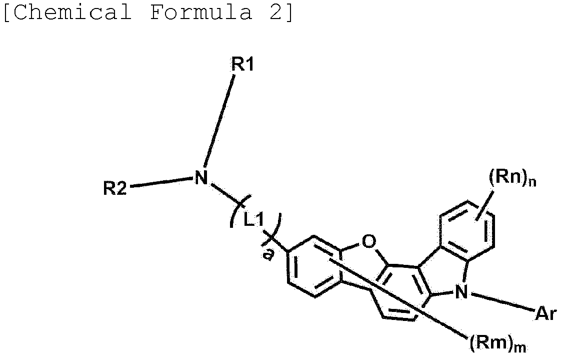

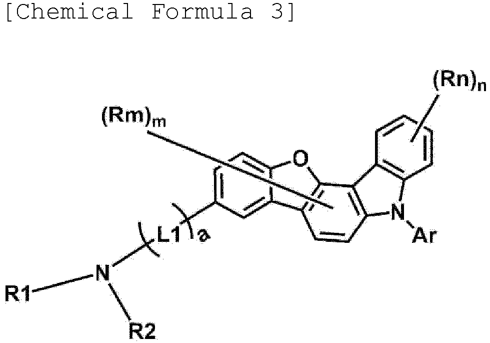

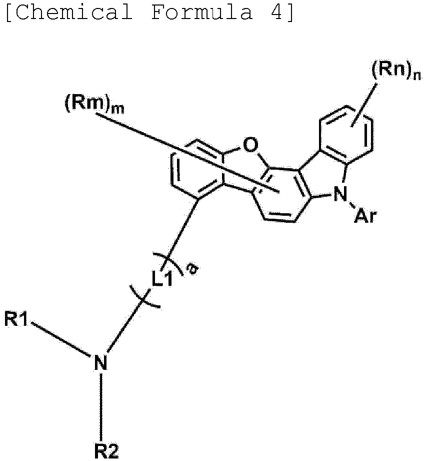

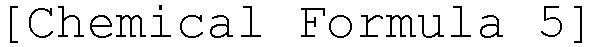

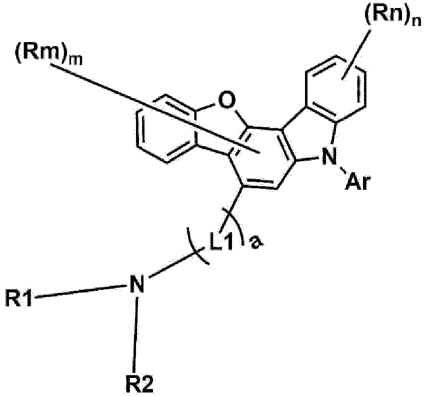

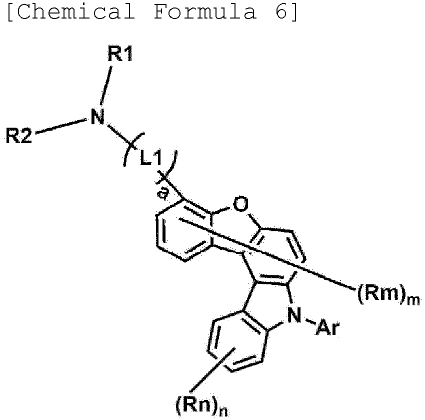

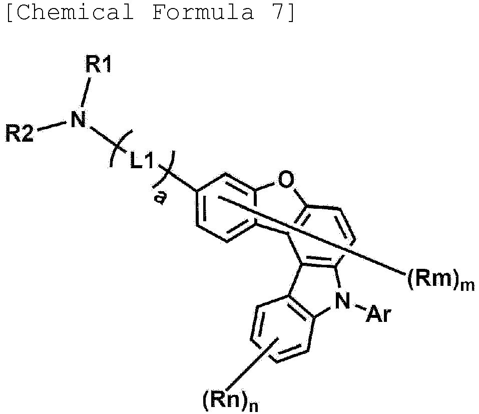

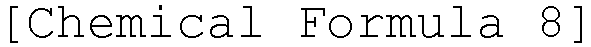

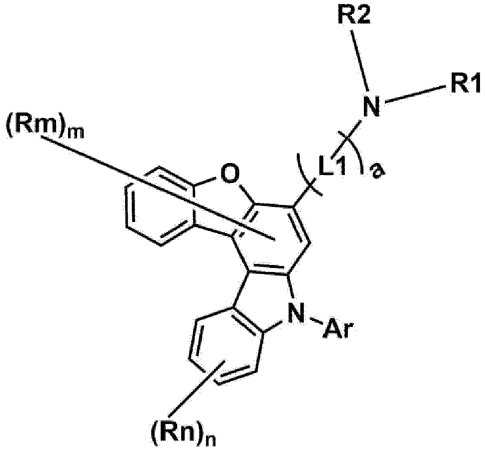

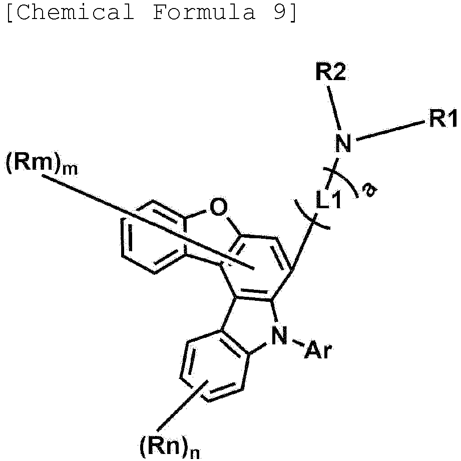

In Chemical Formulae 2 to 9,
L1 is a direct bond; or a substituted or unsubstituted arylene group having 6 to 60 carbon atoms, a is an integer from 0 to 3, and when a is 2 or higher, L1's in the parenthesis are the same as or different from each other,
R1 is a phenyl group unsubstituted or substituted with one or more deuteriums; a biphenyl group unsubstituted or substituted with one or more deuteriums; a terphenyl group unsubstituted or substituted with one or more deuteriums; a naphthyl group unsubstituted or substituted with one or more deuteriums; a phenanthrene group unsubstituted or substituted with one or more deuteriums; a pyrene group; a fluorene group unsubstituted or substituted with one or more substituents selected from the group consisting of deuterium and a methyl group; a triphenylenyl group unsubstituted or substituted with one or more deuteriums; a dibenzofuran group unsubstituted or substituted with one or more deuteriums; or a dibenzothiophene group unsubstituted or substituted with one or more deuteriums,
R2 is a phenyl group unsubstituted or substituted with one or more deuteriums; a biphenyl group unsubstituted or substituted with one or more deuteriums; a terphenyl group unsubstituted or substituted with one or more deuteriums; a naphthyl group unsubstituted or substituted with one or more deuteriums; a phenanthrene group unsubstituted or substituted with one or more deuteriums; a dibenzofuran group unsubstituted or substituted with one or more deuteriums; or a dibenzothiophene group unsubstituted or substituted with one or more deuteriums,
Rm and Rn are the same as or different from each other, and are each independently hydrogen; or deuterium,
m is an integer from 0 to 5, and when m is 2 or higher, Rm's in the parenthesis are the same as or different from each other,
n is an integer from 0 to 4, and when n is 2 or higher, Rn's in the parenthesis are the same as or different from each other, and
Ar is a substituted or unsubstituted aryl group having 6 to 60 carbon atoms.

The compound represented by any of Chemical Formulae 2 to 9 has a structure in which benzofurocarbazole is substituted with two types of substituents, that is, an amine group and an aryl group. As a result, the compound represented by any of Chemical Formulae 2 to 9 may delocalize the highest occupied molecular orbital (HOMO) energy level to increase the hole transport ability and stabilize the HOMO energy.

Therefore, when the heterocyclic compound of any of Chemical Formulae 2 to 9 is used as a material for the hole transport layer in the organic light emitting device, an appropriate energy level and an appropriate band gap are formed to increase excitons in the light emitting region. Increasing excitons in the light emitting region means having an effect of reducing the driving voltage of the device and an effect of increasing efficiency.

That is, the compound represented by any of Chemical Formulae 2 to 9 has an excellent hole transport ability, so that when the compound represented by any of Chemical Formulae 2 to 9 is used as a material for an organic material layer of an organic light emitting device, the efficiency and service life of the device can be improved.

In the present specification, the term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more substituents are substituted, the two or more substituents may be the same as or different from each other.

In the present specification, "substituted or unsubstituted" means being unsubstituted or substituted with one or more substituents selected from the group consisting of deuterium; a cyano group; a halogen group; a straight-chained or branched alkyl having 1 to 60 carbon atoms; a straight-chained or branched alkenyl having 2 to 60 carbon atoms; a straight-chained or branched alkynyl having 2 to 60 carbon atoms; a monocyclic or polycyclic cycloalkyl having 3 to 60 carbon atoms; a monocyclic or polycyclic heterocycloalkyl having 2 to 60 carbon atoms; a monocyclic or polycyclic aryl having 6 to 60 carbon atoms; a monocyclic or polycyclic heteroaryl having 2 to 60 carbon atoms; -SiRR'R"; -P(=O)RR'; an alkylamine having 1 to 20 carbon atoms; a monocyclic or polycyclic arylamine having 6 to 60 carbon atoms; and a monocyclic or polycyclic heteroarylamine having 2 to 60 carbon atoms, or being unsubstituted or substituted with a substituent to which two or more substituents selected from the above exemplified substituents are linked, and means that R, R' and R" are the same as or different from each other, and are each independently a substituted or unsubstituted alkyl having 1 to 60 carbon atoms; a substituted or unsubstituted aryl having 6 to 60 carbon atoms; or a substituted or unsubstituted heteroaryl having 2 to 60 carbon atoms.

In the present specification, "when a substituent is not indicated in the structure of a chemical formula or compound" means that a hydrogen atom is bonded to a carbon atom. However, since deuterium (²H) is an isotope of hydrogen, some hydrogen atoms may be deuterium.

In an exemplary embodiment of the present application, "when a substituent is not indicated in the structure of a chemical formula or compound" may mean that all the positions that may be reached by the substituent are hydrogen or deuterium. That is, deuterium is an isotope of hydrogen, and some hydrogen atoms may be deuterium which is an isotope, and in this case, the content of deuterium may be 0% to 100%.

In an exemplary embodiment of the present application, in "the case where a substituent is not indicated in the structure of a chemical formula or compound", when the content of deuterium is 0%, the content of hydrogen is 100%, and all the substituents do not explicitly exclude deuterium such as hydrogen, hydrogen and deuterium may be mixed and used in the compound.

In an exemplary embodiment of the present application, deuterium is one of the isotopes of hydrogen, is an element that has a deuteron composed of one proton and one neutron as a nucleus, and may be represented by hydrogen-2, and the element symbol may also be expressed as D or 2H.

In an exemplary embodiment of the present application, the isotope means an atom with the same atomic number (Z), but different mass numbers (A), and the isotope may be interpreted as an element which has the same number of protons, but different number of neutrons.

In an exemplary embodiment of the present application, when the total number of substituents of a basic compound is defined as T1 and the number of specific substituents among the substituents is defined as T2, the content T% of the specific substituent may be defined as T2/T1×100 = T%.

That is, in an example, the deuterium content of 20% in a phenyl group represented by 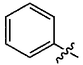 may be represented by 20% when the total number of substituents that the phenyl group can have is 5 (T1 in the formula) and the number of deuteriums among the substituents is 1 (T2 in the formula). That is, a deuterium content of 20% in the phenyl group may be represented by the following structural formula.

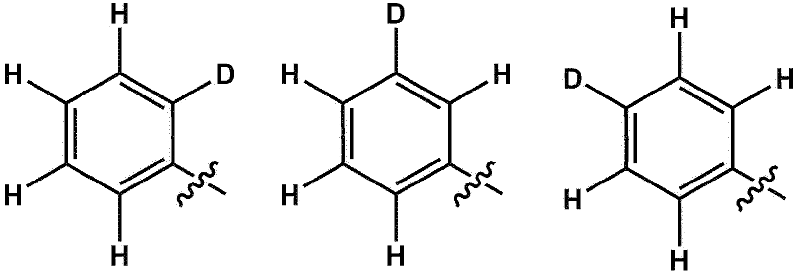

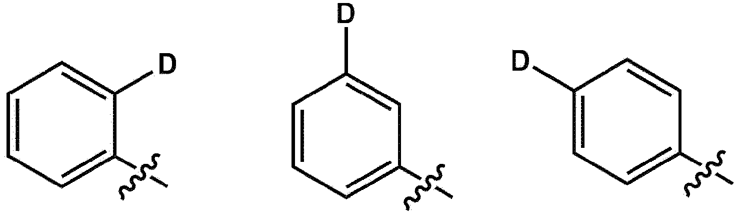

Further, in an exemplary embodiment of the present application, "a phenyl group having a deuterium content of 0%" may mean a phenyl group that does not include a deuterium atom, that is, has five hydrogen atoms.

In the present specification, the halogen may be fluorine, chlorine, bromine or iodine.

In the present specification, the alkyl group includes a straight-chain or branched-chain having 1 to 60 carbon atoms, and may be additionally substituted with another substituent. The number of carbon atoms of the alkyl group may be 1 to 60, specifically 1 to 40, and more specifically 1 to 20. Specific examples thereof include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group, and the like, but are not limited thereto.

In the present specification, the alkenyl group includes a straight-chain or branched-chain having 2 to 60 carbon atoms, and may be additionally substituted with another substituent. The number of carbon atoms of the alkenyl group may be 2 to 60, specifically 2 to 40, and more specifically 2 to 20. Specific examples thereof include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like, but are not limited thereto.

In the present specification, the alkynyl group includes a straight-chain or branched-chain having 2 to 60 carbon atoms, and may be additionally substituted with another substituent. The number of carbon atoms of the alkynyl group may be 2 to 60, specifically 2 to 40, and more specifically 2 to 20.

In the present specification, an alkoxy group may be straight-chained, branched, or cyclic. The number of carbon atoms of the alkoxy group is not particularly limited, but is preferably 1 to 20. Specific examples thereof include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and the like, but are not limited thereto.
In the present specification, the cycloalkyl group includes a monocycle or polycycle having 3 to 60 carbon atoms, and may be additionally substituted with another substituent. Here, the polycycle means a group in which a cycloalkyl group is directly linked to or fused with another cyclic group. Here, another cyclic group may also be a cycloalkyl group, but may also be another kind of cyclic group, for example, a heterocycloalkyl group, an aryl group, a heteroaryl group, and the like. The number of carbon atoms of the cycloalkyl group may be 3 to 60, specifically 3 to 40, and more specifically 5 to 20. Specific examples thereof include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group, and the like, but are not limited thereto.

In the present specification, the heterocycloalkyl group includes O, S, Se, N, or Si as a heteroatom, includes a monocycle or polycycle having 2 to 60 carbon atoms, and may be additionally substituted with another substituent. Here, the polycycle means a group in which a heterocycloalkyl group is directly linked to or fused with another cyclic group. Here, another cyclic group may also be a heterocycloalkyl group, but may also be another kind of cyclic group, for example, a cycloalkyl group, an aryl group, a heteroaryl group, and the like. The number of carbon atoms of the heterocycloalkyl group may be 2 to 60, specifically 2 to 40, and more specifically 3 to 20.

In the present specification, the aryl group includes a monocycle or polycycle having 6 to 60 carbon atoms, and may be additionally substituted with another substituent. Here, the polycycle means a group in which an aryl group is directly linked to or fused with another cyclic group. Here, another cyclic group may also be an aryl group, but may also be another kind of cyclic group, for example, a cycloalkyl group, a heterocycloalkyl group, a heteroaryl group, and the like. The aryl group includes a spiro group. The number of carbon atoms of the aryl group may be 6 to 60, specifically 6 to 40, and more specifically 6 to 25. Specific examples of the aryl group include a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused cyclic group thereof, and the like, but are not limited thereto.

In the present specification, a phosphine oxide group is represented by -P(=O)R101R102, and R101 and R102 are the same as or different from each other, and may be each independently a substituent composed of at least one of hydrogen; deuterium; a halogen group; an alkyl group; an alkenyl group; an alkoxy group; a cycloalkyl group; an aryl group; and a heterocyclic group. Specific examples of the phosphine oxide group include a diphenylphosphine oxide group, dinaphthylphosphine oxide, and the like, but are not limited thereto.

In the present specification, a silyl group includes Si and is a substituent to which the Si atom is directly linked as a radical, and is represented by -SiR104R105R106, and R104 to R106 are the same as or different from each other, and may be each independently a substituent composed of at least one of hydrogen; deuterium; a halogen group; an alkyl group; an alkenyl group; an alkoxy group; a cycloalkyl group; an aryl group; and a heterocyclic group. Specific examples of the silyl group include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but are not limited thereto.

In the present specification, the fluorenyl group may be substituted, and adjacent substituents may be bonded to each other to form a ring.

In the present specification, the spiro group is a group including a spiro structure, and may have 15 to 60 carbon atoms. For example, the spiro group may include a structure in which a 2,3-dihydro-1H-indene group or a cyclohexane group is spiro-bonded to a fluorenyl group. Specifically, the spiro group may include any one of the groups of the following structural formulae.

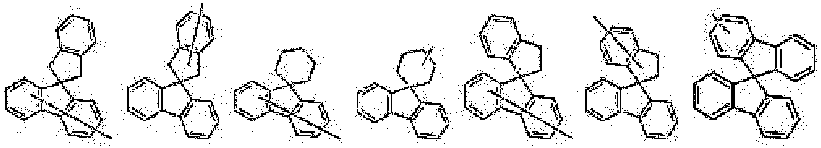

In the present specification, the heteroaryl group includes S, O, Se, N, or Si as a heteroatom, includes a monocycle or polycycle having 2 to 60 carbon atoms, and may be additionally substituted with another substituent. Here, the polycycle means a group in which a heteroaryl group is directly linked to or fused with another cyclic group. Here, another cyclic group may also be a heteroaryl group, but may also be another kind of cyclic group, for example, a cycloalkyl group, a heterocycloalkyl group, an aryl group, and the like. The number of carbon atoms of the heteroaryl group may be 2 to 60, specifically 2 to 40, and more specifically 3 to 25. Specific examples of the heteroaryl group include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a quinozolilyl group, a naphthyridyl group, an acridinyl group, a phenanthridinyl group, an imidazopyridinyl group, a diaza naphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi (dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepin group, a 9,10-dihydroacridinyl group, a phenanthrazinyl group, a phenothiazinyl group, a phthalazinyl group, a naphthylidinyl group, a phenanthrolinyl group, a benzo[c][1,2,5]thiadiazolyl group, a 5,10-dihydrodibenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group, and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and the number of carbon atoms thereof is not particularly limited, but is preferably 1 to 30. Specific examples of the amine group include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group, and the like, but are not limited thereto.

In the present specification, an arylene group means that there are two bonding positions in an aryl group, that is, a divalent group. The above-described description on the aryl group may be applied to the arylene group, except that the arylene groups are each a divalent group. Further, a heteroarylene group means that there are two bonding positions in a heteroaryl group, that is, a divalent group. The above-described description on the heteroaryl group may be applied to the heteroarylene group, except for a divalent heteroarylene group.

In the present specification, the "adjacent" group may mean a substituent substituted with an atom directly linked to an atom in which the corresponding substituent is substituted, a substituent disposed to be sterically closest to the corresponding substituent, or another substituent substituted with an atom in which the corresponding substituent is substituted. For example, two substituents substituted at the ortho position in a benzene ring and two substituents substituted with the same carbon in an aliphatic ring may be interpreted as groups which are "adjacent" to each other.

The heterocyclic compound represented by any of Chemical Formulae 2 to 9 according to an exemplary embodiment of the present application may be used as a material for the organic material layer of the organic light emitting device.

In an exemplary embodiment of the present application, the deuterium content of the heterocyclic compound represented by any of Chemical Formulae 2 to 9 1 may be 0% to 100%.

In an exemplary embodiment of the present application, the deuterium content of the heterocyclic compound represented by any of Chemical Formulae 2 to 9 1 may be 10% or more and 100% or less.

In an exemplary embodiment of the present application, the deuterium content of the heterocyclic compound represented by any of Chemical Formulae 2 to 9 may be 20% or more and 100% or less.

In an exemplary embodiment of the present application, the deuterium content of the heterocyclic compound represented by any of Chemical Formulae 2 to 9 may be 30% or more and 100% or less.

In an exemplary embodiment of the present application, the deuterium content of the heterocyclic compound represented by any of Chemical Formulae 2 to 9 may be 40% or more and 100% or less.

When a part or all of the heterocyclic compound represented by any of Chemical Formulae 2 to 9 is substituted with deuterium, there is an effect that the driving voltage, the light emitting efficiency and the service life are further improved due to the magnitude of the C-D dissociation energy.

In an exemplary embodiment of the present application, L1 of any of Chemical Formulae 2 to may be a direct bond; or a substituted or unsubstituted arylene group having 6 to 60 carbon atoms.
In an exemplary embodiment of the present application, L1 may be a direct bond; or a substituted or unsubstituted arylene group having 6 to 40 carbon atoms.

In an exemplary embodiment of the present application, L1 may be a direct bond; or a substituted or unsubstituted arylene group having 6 to 20 carbon atoms.

In an exemplary embodiment of the present application, L1 may be a direct bond; or a substituted or unsubstituted phenylene group.

In an exemplary embodiment of the present application, L1 may be a direct bond; or a phenylene group unsubstituted or substituted with one or more deuteriums.

In an exemplary embodiment of the present application, L1 is a direct bond.

In an exemplary embodiment of the present application, L1 is a substituted or unsubstituted phenylene group.

In an exemplary embodiment of the present application, L1 is a phenylene group unsubstituted or substituted with one or more deuteriums.

In an exemplary embodiment of the present application, m of any of Chemical Formulae 2 to 9 is 0. In an exemplary embodiment of the present application, m is 1. In an exemplary embodiment of the present application, m is 2. In an exemplary embodiment of the present application, m is 3. In an exemplary embodiment of the present application, m is 4. In an exemplary embodiment of the present application, m is 5.

In an exemplary embodiment of the present application, when m is 2 or higher, Rm's in the parenthesis are the same as or different from each other.

In an exemplary embodiment of the present application, n of any of Chemical Formulae 2 to 9 is 0. In an exemplary embodiment of the present application, n is 1. In an exemplary embodiment of the present application, n is 2. In an exemplary embodiment of the present application, n is 3. In an exemplary embodiment of the present application, n is 4.

In an exemplary embodiment of the present application, when n is 2 or higher, Rn's in the parenthesis are the same as or different from each other.

In an exemplary embodiment of the present application, Ar of any of Chemical Formulae 2 to 9 may be a substituted or unsubstituted aryl group having 6 to 60 carbon atoms.

In an exemplary embodiment of the present application, Ar may be a substituted or unsubstituted aryl group having 6 to 40 carbon atoms.

In an exemplary embodiment of the present application, Ar may be a substituted or unsubstituted aryl group having 6 to 20 carbon atoms.

In an exemplary embodiment of the present application, Ar may be a substituted or unsubstituted phenyl group; a substituted or unsubstituted biphenyl group; or a substituted or unsubstituted terphenyl group.

In an exemplary embodiment of the present application, Ar may be a phenyl group unsubstituted or substituted with one or more deuteriums; a biphenyl group unsubstituted or substituted with one or more deuteriums; or a terphenyl group unsubstituted or substituted with one or more deuteriums.

Chemical Formulae 2 to 5 each have an amine group as a substituent at positions Nos. 2 to 5 of benzofurocarbazole represented by the following Chemical Formula A to further increase the hole transport ability of the compound, and thus have an effect in which the driving, efficiency and service life of the device are improved using the same. In contrast, in the case of having an amine group as a substituent at position No. 1 of benzofurocarbazole represented by the following Chemical Formula A, a T1 value (energy level value in a triplet state) is lowered. This means that the driving voltage and efficiency of the device using the same may be enhanced and reduced, respectively.

In addition, in the case of having an amine group as a substituent at position No. 6 of benzofurocarbazole represented by the following Chemical Formula A, the benzofurocarbazole becomes structurally unstable due to steric hindrance between the substituents. This means that the service life and efficiency of the device using the same may be reduced.

Here, substitution positions Nos. 1 to 6 of benzofurocarbazole in the description of the compounds of Chemical Formulae 2 to 5 mean the portions represented by numbers in the following Chemical Formula A.

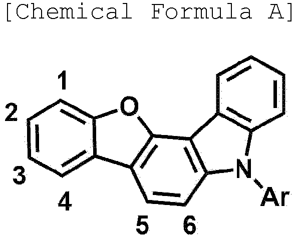

In Chemical Formula A, the definition of Ar is the same as that in any of Chemical Formulae 2 to 9.

Chemical Formulae 6 to 9 each have an amine group as a substituent at positions Nos. 3 to 6 of benzofurocarbazole represented by the following Chemical Formula B to further increase the hole transport ability of the compound, and thus have an effect in which the driving, efficiency and service life of the device are improved using the same.

In contrast, in the case of having an amine group as a substituent at position No. 1 of benzofurocarbazole represented by the following Chemical Formula B, the benzofurocarbazole becomes structurally unstable due to steric hindrance between the substituents. This means that the service life and efficiency of the device using the same may be reduced.

Furthermore, in the case of having an amine group as a substituent at position No. 2 of benzofurocarbazole represented by the following Chemical Formula B, the decomposition temperature (hereinafter, also represented by Td) is low and the benzofurocarbazole becomes structurally unstable. This means that the service life and efficiency of the device using the same may be reduced.

Here, substitution positions Nos. 1 to 6 of benzofurocarbazole in the description of the compounds of Chemical Formulae 6 to 9 mean the portions represented by numbers in the following Chemical Formula B.

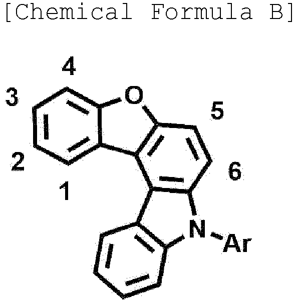

In Chemical Formula B, the definition of Ar is the same as that in any of Chemical Formulae 2 to 9.

According to an exemplary embodiment of the present application, Chemical Formulae 2 to 9 may be represented by any one of the following compounds, but is not limited thereto.

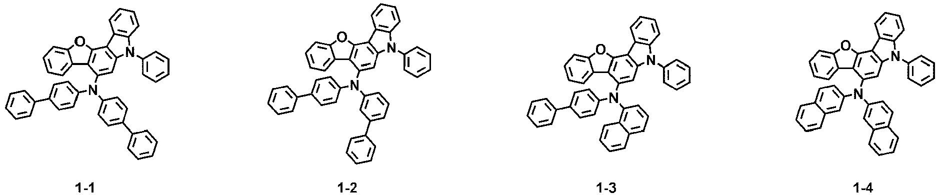

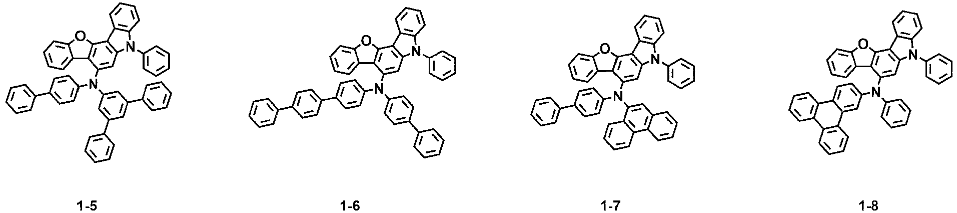

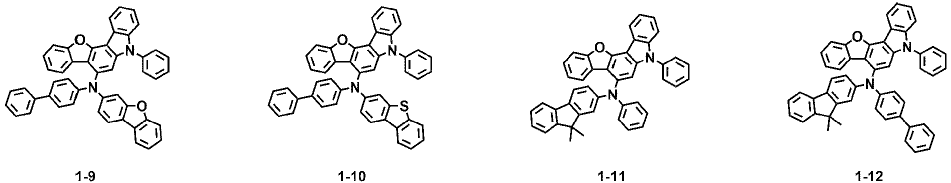

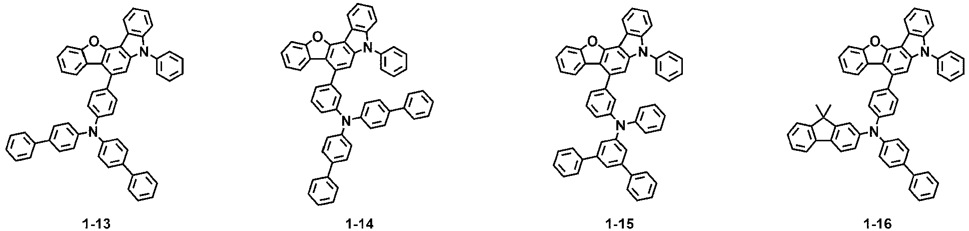

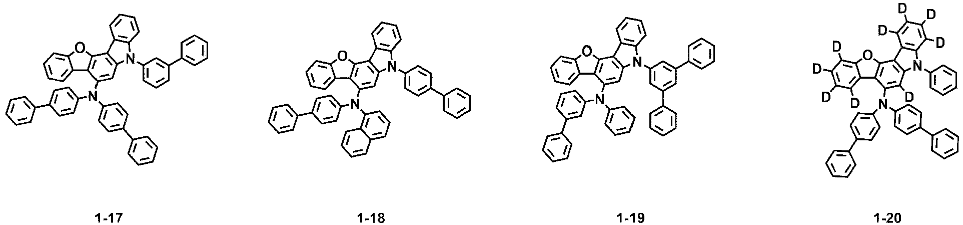

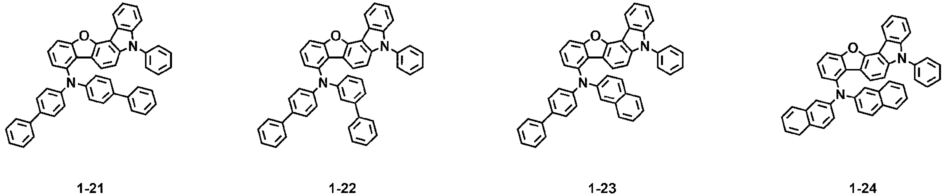

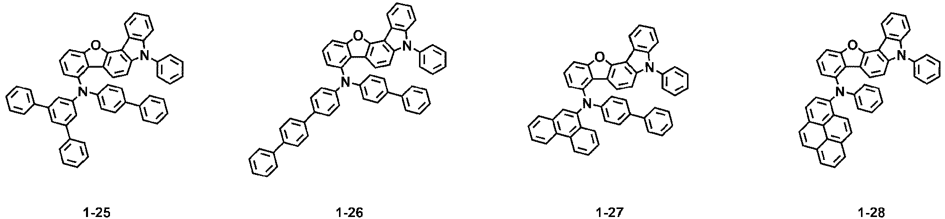

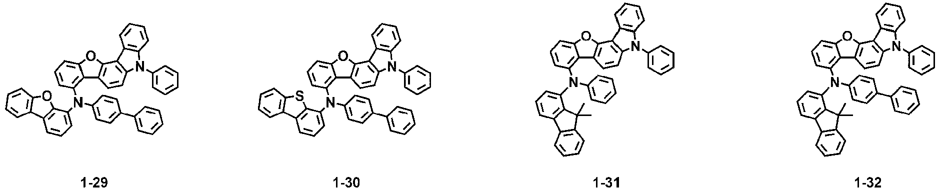

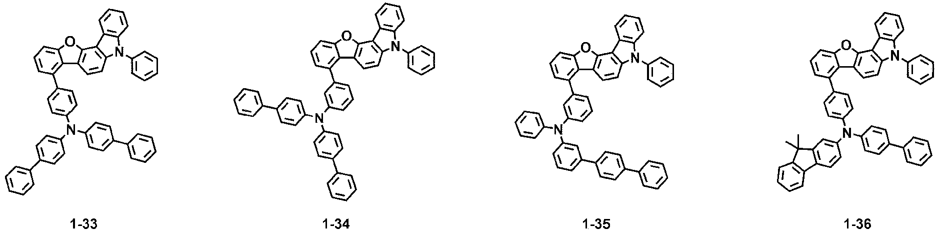

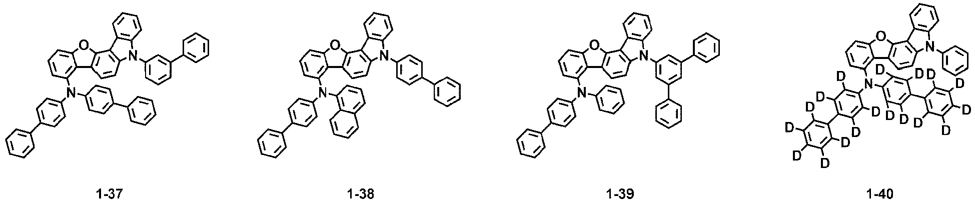

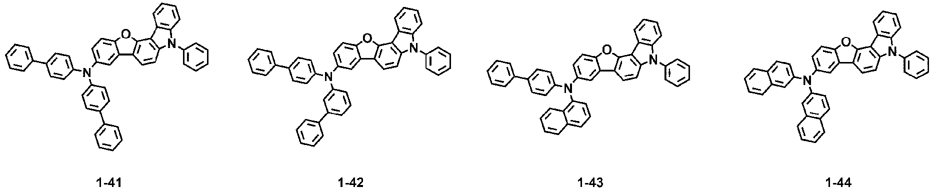

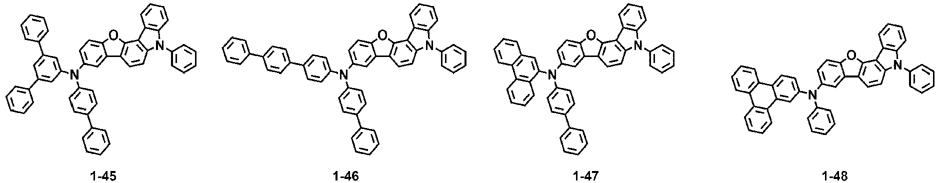

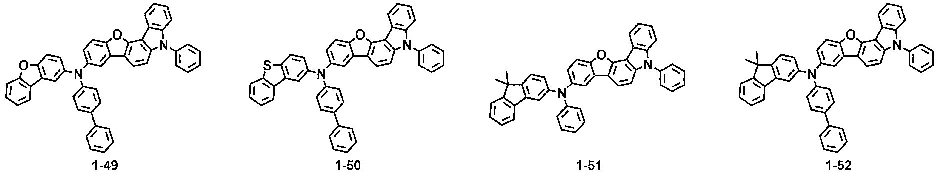

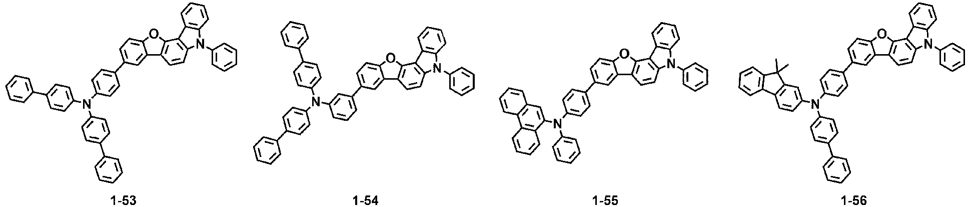

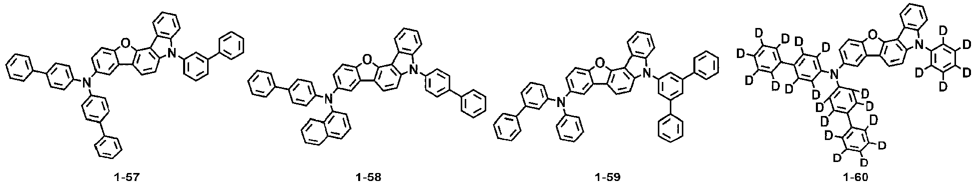

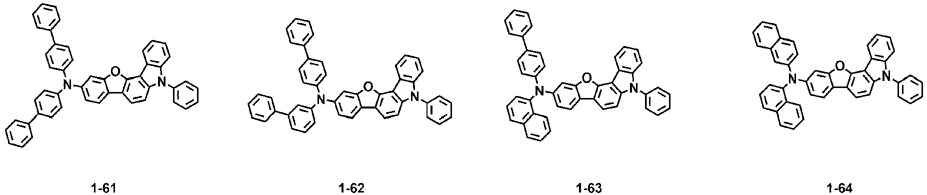

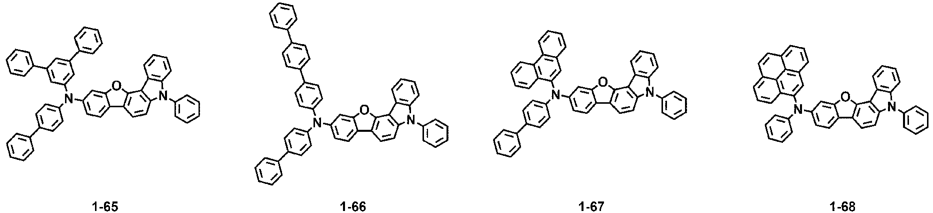

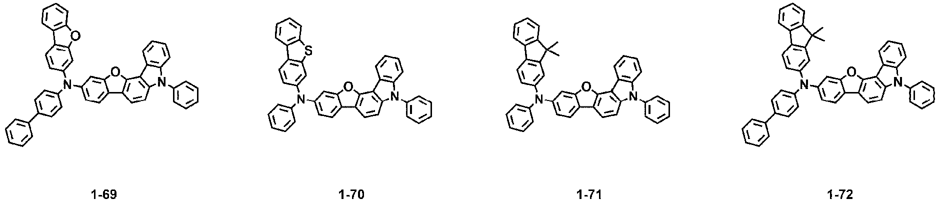

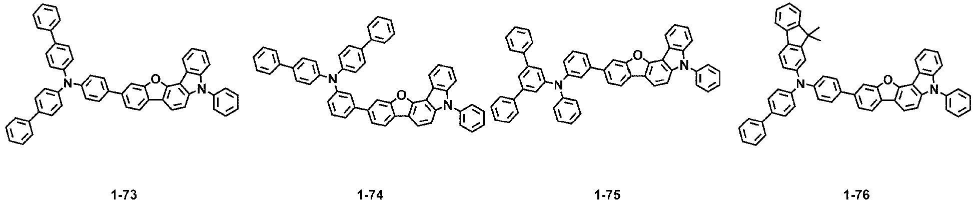

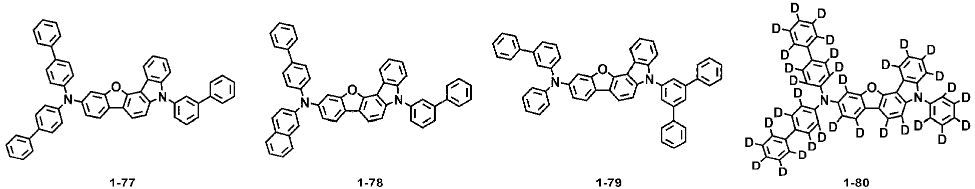

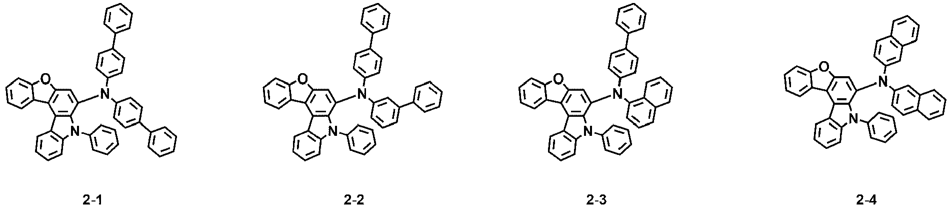

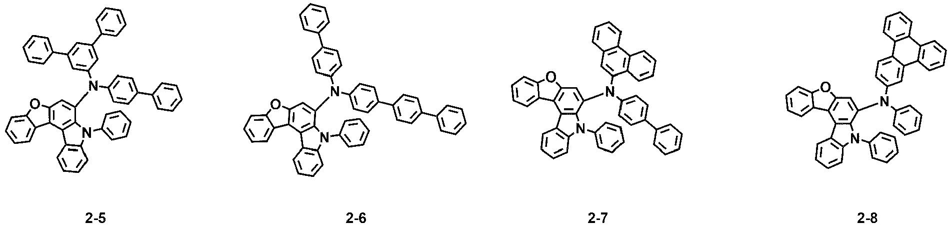

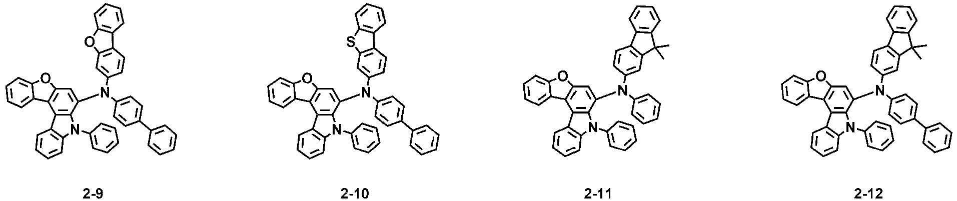

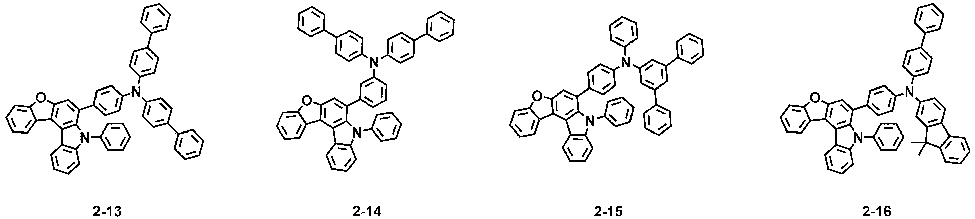

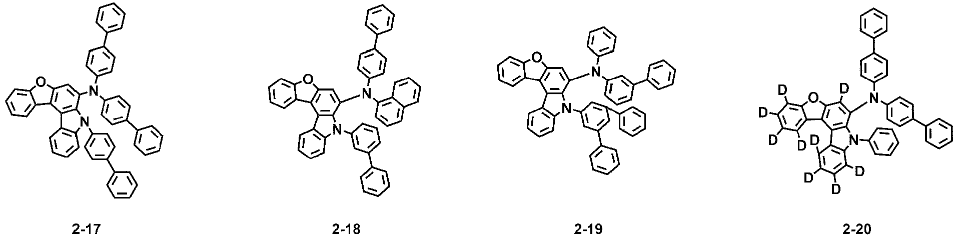

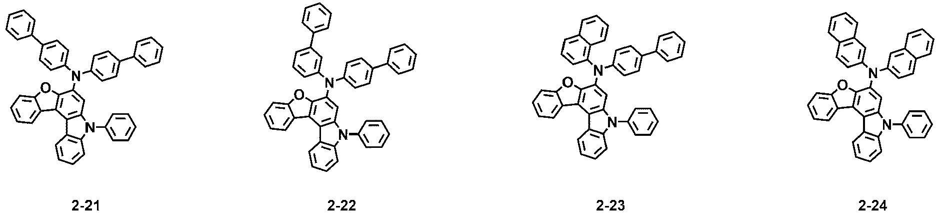

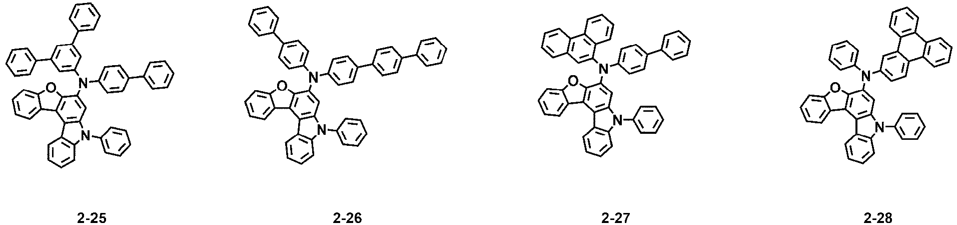

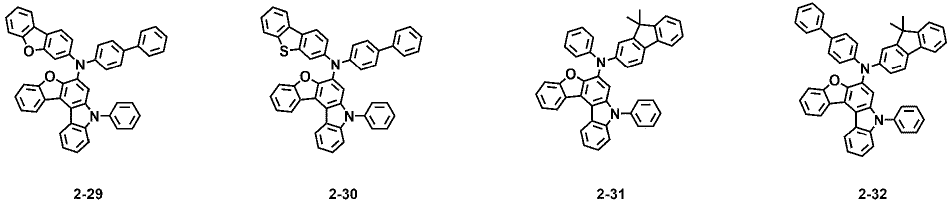

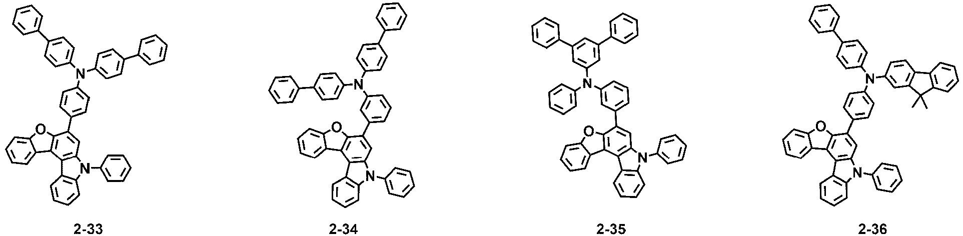

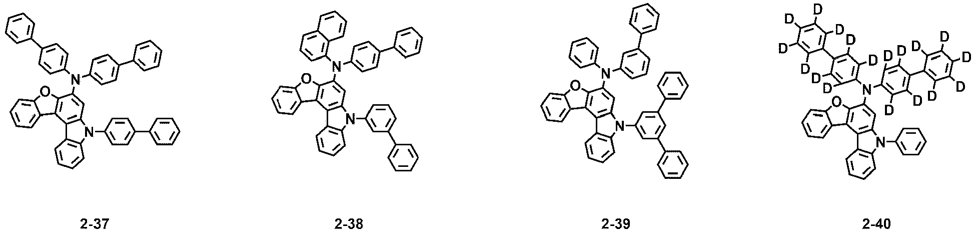

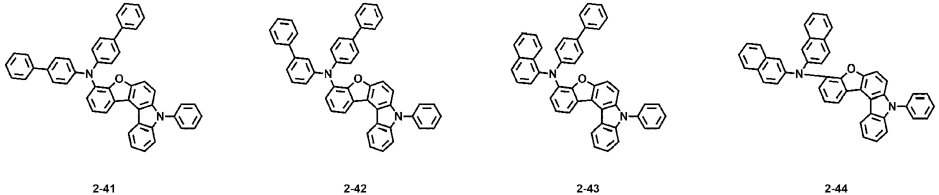

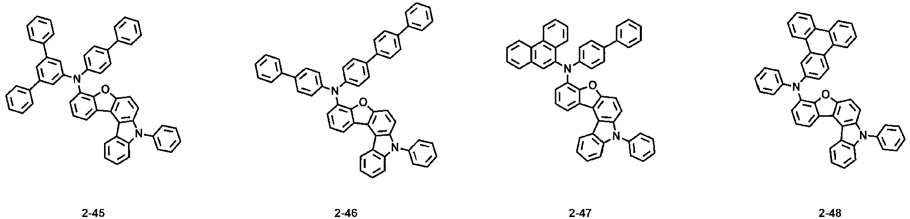

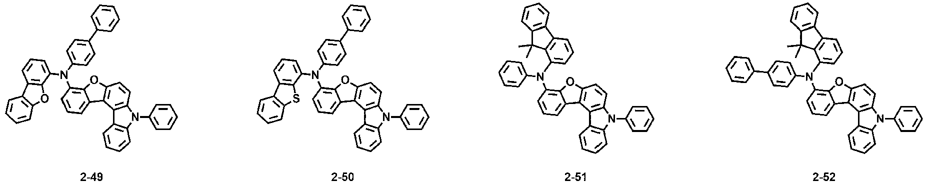

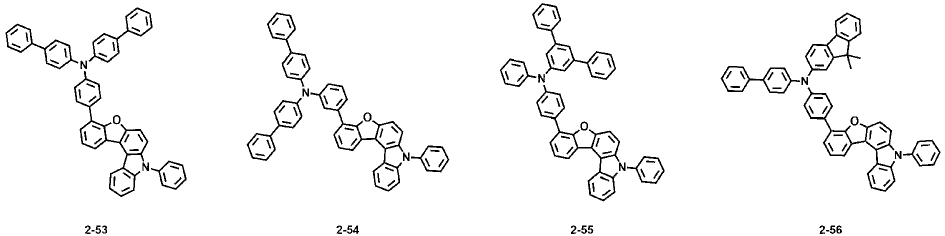

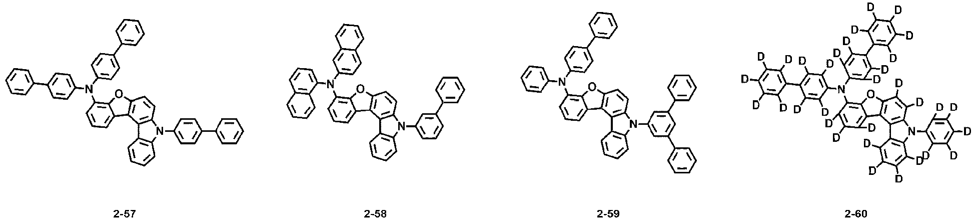

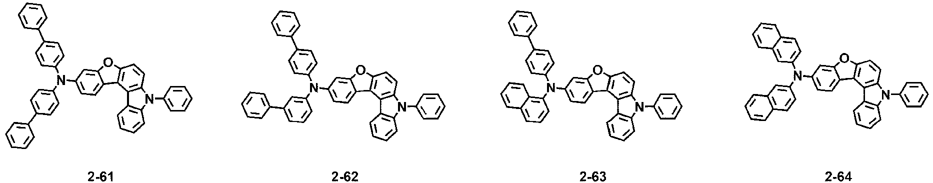

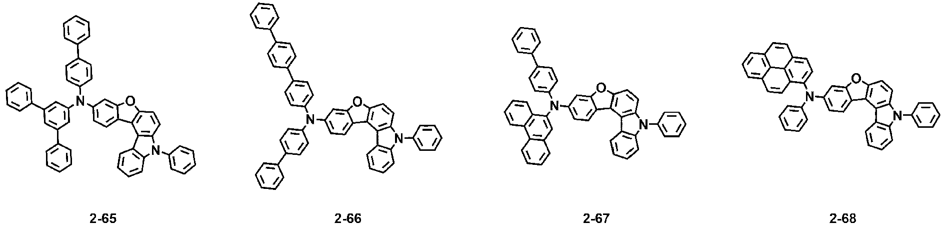

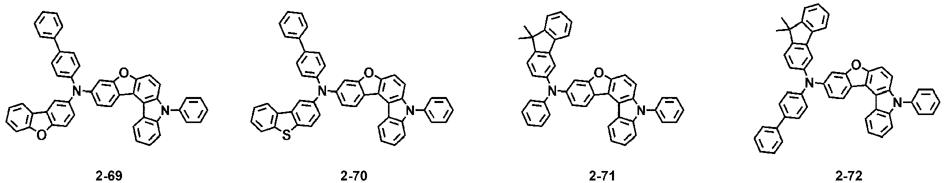

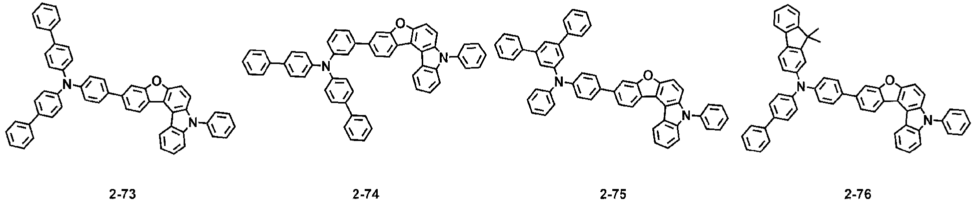

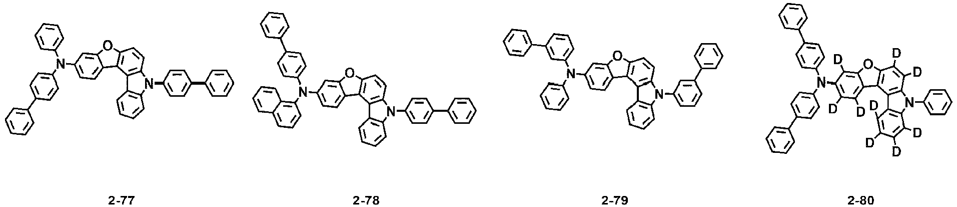

Further, various substituents may be introduced into the structure of any of Chemical Formulae 2 to 9 to synthesize a compound having inherent characteristics of a substituent introduced. For example, it is possible to synthesize a material which satisfies conditions required for each organic material layer by introducing a substituent usually used for a hole injection layer material, a material for transporting holes, a light emitting layer material, an electron transport layer material, and a charge generation layer material, which are used for preparing an organic light emitting device, into the core structure.

In addition, it is possible to finely adjust an energy band-gap by introducing various substituents into the structure of any of Chemical Formulae 2 to 9, and meanwhile, it is possible to improve characteristics at the interface between organic materials and diversify the use of the material.

Meanwhile, the heterocyclic compound of any of Chemical Formulae 2 to 9 has a high glass transition temperature (Tg) and thus has excellent thermal stability. The increase in thermal stability becomes an important factor for providing a device with driving stability.

The heterocyclic compound according to an exemplary embodiment of the present application may be prepared by a multi-step chemical reaction. Some intermediate compounds are first prepared, and any of the compounds of Chemical Formulae 2 to 9 may be prepared from the intermediate compounds. More specifically, the heterocyclic compound according to an exemplary embodiment of the present application may be prepared based on the Preparation Examples to be described below.

Specifically, the organic light emitting device according to an exemplary embodiment of the present application includes a first electrode, a second electrode and an organic material layer having one or more layers provided between the first electrode and the second electrode, in which one or more layers of the organic material layer include the heterocyclic compound represented by any of Chemical Formulae 2 to 9. When the heterocyclic compound represented by any of Chemical Formulae 2 to 9 is included in the organic material layer, the light emitting efficiency and service life of the organic light emitting device are excellent.

In an exemplary embodiment of the present application, the first electrode may be a positive electrode, and the second electrode may be a negative electrode.

In another exemplary embodiment, the first electrode may be a negative electrode, and the second electrode may be a positive electrode.

Another exemplary embodiment of the present application provides an organic light emitting device including the heterocyclic compound represented by any of Chemical Formulae 2 to 9. The "organic light emitting device" may be expressed by terms such as "organic light emitting diode", "organic light emitting diodes (OLEDs)", "OLED device", and "organic electroluminescence device".

The heterocyclic compound may be formed as an organic material layer by not only a vacuum deposition method, but also a solution application method when an organic light emitting device is manufactured. Here, the solution application method means spin coating, dip coating, inkjet printing, screen printing, a spray method, roll coating, and the like, but is not limited thereto.

The organic material layer of the organic light emitting device of the present invention may further include one layer or two or more layers selected from the group consisting of a light emitting layer, a hole injection layer, a hole transport layer, an electron injection layer, an electron transport layer, a hole auxiliary layer, and a hole blocking layer.

The organic material layer includes a hole transport layer having one or more layers, and the hole transport layer includes the heterocyclic compound represented by any of Chemical Formulae 2 to 9. When the heterocyclic compound represented by any of Chemical Formulae 2 to 9 is included in the light emitting layer among the organic material layers, the light emitting efficiency and service life of the organic light emitting device are better.

The organic light emitting device according to an exemplary embodiment of the present application may be manufactured by typical manufacturing methods and materials of the organic light emitting device, except that the above-described heterocyclic compound is used to form an organic material layer.

FIGS. 1 to 3 exemplify the stacking sequence of the electrodes and the organic material layer of the organic light emitting device according to an exemplary embodiment of the present application. However, the scope of the present application is not intended to be limited by these drawings, and the structure of the organic light emitting device known in the art may also be applied to the present application.

According to FIG. 1, an organic light emitting device in which a positive electrode 200, an organic material layer 300, and a negative electrode 400 are sequentially stacked on a substrate 100 is illustrated. However, the organic light emitting device is not limited only to such a structure, and as in FIG. 2, an organic light emitting device in which a negative electrode, an organic material layer, and a positive electrode are sequentially stacked on a substrate may also be implemented.

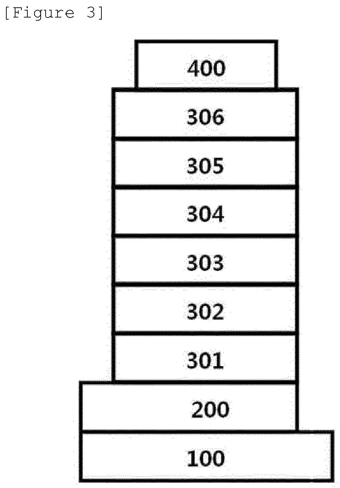

FIG. 3 exemplifies a case where an organic material layer is a multilayer. An organic light emitting device according to FIG. 3 includes a hole injection layer 301, a hole transport layer 302, a light emitting layer 303, a hole blocking layer 304, an electron transport layer 305, and an electron injection layer 306. However, the scope of the present application is not limited by the stacking structure as described above, and if necessary, the other layers except for the light emitting layer may be omitted, and another necessary functional layer may be further added.

In the organic light emitting device according to an exemplary embodiment of the present application, materials other than the heterocyclic compound of any of Chemical Formulae 2 to 9will be exemplified below, but these materials are illustrative only and are not for limiting the scope of the present application, and may be replaced with materials publicly known in the art.

As a positive electrode material, materials having a relatively high work function may be used, and a transparent conductive oxide, a metal or a conductive polymer, and the like may be used. Specific examples of the positive electrode material include: a metal such as vanadium, chromium, copper, zinc, and gold, or an alloy thereof; a metal oxide such as zinc oxide, indium oxide, indium tin oxide (ITO), and indium zinc oxide (IZO); a combination of a metal and an oxide, such as ZnO:Al or SnO₂:Sb; a conductive polymer such as poly(3-methylthiophene), poly[3,4-(ethylene-1,2-dioxy)thiophene] (PEDT), polypyrrole, and polyaniline; and the like, but are not limited thereto.

As a negative electrode material, materials having a relatively low work function may be used, and a metal, a metal oxide, or a conductive polymer, and the like may be used. Specific examples of the negative electrode material include: a metal such as magnesium, calcium, sodium, potassium, titanium, indium, yttrium, lithium, gadolinium, aluminum, silver, tin, and lead, or an alloy thereof; a multi-layer structured material, such as LiF/Al or LiO₂/Al; and the like, but are not limited thereto.

As a hole injection material, a publicly-known hole injection material may also be used, and it is possible to use, for example, a phthalocyanine compound such as copper phthalocyanine disclosed in US Patent No. 4,356,429 or starburst-type amine derivatives described in the document [Advanced Material, 6, p. 677 (1994)], for example, tris(4-carbazoyl-9-ylphenyl)amine (TCTA), 4,4',4"-tri[phenyl(m-tolyl)amino]triphenylamine (m-MTDATA), 1,3,5-tris[4-(3-methylphenylphenylamino)phenyl]benzene (m-MTDAPB), polyaniline/dodecylbenzenesulfonic acid or poly(3,4-ethylenedioxythiophene)/poly(4-styrenesulfonate), which is a soluble conductive polymer, polyaniline/camphor sulfonic acid or polyaniline/poly(4-styrene-sulfonate), and the like.

As a hole transporting material, a pyrazoline derivative, an arylamine-based derivative, a stilbene derivative, a triphenyldiamine derivative, and the like may be used, and a low-molecular weight or polymer material may also be used.

As an electron transporting material, it is possible to use an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyldicyanoethylene and a derivative thereof, a diphenoquinone derivative, a metal complex of 8-hydroxyquinoline and a derivative thereof, and the like, and a low-molecular weight material and a polymer material may also be used.

As an electron injection material, for example, LiF is representatively used in the art, but the present application is not limited thereto.

As a light emitting material, a red, green, or blue light emitting material may be used, and if necessary, two or more light emitting materials may be mixed and used. Further, a fluorescent material may also be used as the light emitting material, but may also be used as a phosphorescent material. As the light emitting material, it is also possible to use alone a material which emits light by combining holes and electrons each injected from a positive electrode and a negative electrode, but materials in which a host material and a dopant material are involved in light emission together may also be used.

The organic light emitting device according to an exemplary embodiment of the present application may be a top emission type, a bottom emission type, or a dual emission type according to the material to be used.

The heterocyclic compound according to an exemplary embodiment of the present application may act even in organic electronic devices including organic solar cells, organic photoconductors, organic transistors, and the like, based on the principle similar to those applied to organic light emitting devices.

Hereinafter, the present specification will be described in more detail through Examples, but these Examples are provided only for exemplifying the present application, and are not intended to limit the scope of the present application.

### <Preparation Example 1>

### <Preparation Example 1> Preparation of Compound 1-1

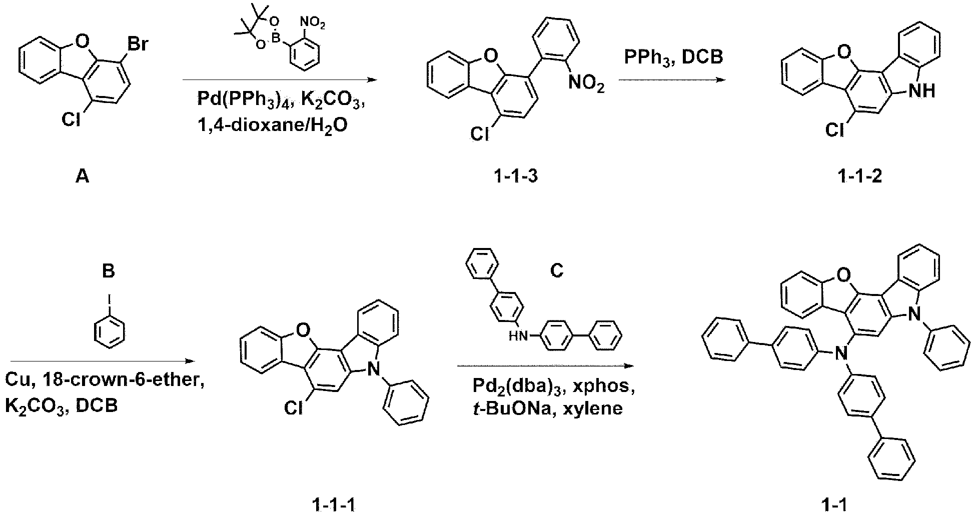

### 1) Preparation of Intermediate 1-1-3

After 20.0 g (71.0 mM) of a compound 4-bromo-1-chlorodibenzo[b,d]furan, 17.7 g (45.3 mM) of 4,4,5,5-tetramethyl-2-(2-nitrophenyl)-1,3,2-dioxaborolane, 4.1 g (3.6 mM) of Pd(PPh₃)₄, and 19.6 g (142.0 mM) of K₂CO₃ were dissolved in 200 mL/40 mL of 1,4-dioxane/distilled water, the resulting solution was refluxed for 6 hours. After the reaction was completed, distilled water and dichloromethane (hereinafter, referred to as DCM) were added thereto at room temperature, extraction was performed, the organic layer was dried over MgSO₄, and then the solvent was removed by a rotary evaporator. The reaction product was purified by column chromatography (DCM:Hex = 1:5) and recrystallized with methanol to obtain 20.7 g (90%) of Intermediate 1-1-3.

Here, Hex means hexane.

### 2) Preparation of Intermediate 1-1-2

After 20.0 g (61.8 mM) of Intermediate 1-1-3 and 40.5 g (154.5 mM) of PPh₃ were dissolved in 200 mL of dichlorobenzene (hereinafter, referred to as DCB), the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hex = 1:4) and recrystallized with methanol to obtain 14.2 g (79%) of Intermediate 1-1-2.

### 3) Preparation of Intermediate 1-1-1

After 14.0 g (48.0 mM) of Intermediate 1-1-12, 10.7 mL (96.0 mM) of iodobenzene, 0.3 g (4.8 mM) of Cu, 1.3 g (4.8 mM) of 18-crown-6-ether, and 13.3 g (96.0 mM) of K₂CO₃ were dissolved in 140 mL of DCB, the resulting solution was refluxed for 48 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hex = 1:4) and recrystallized with methanol to obtain 14.3 g (81%) of Intermediate 1-1-1.

### 4) Preparation of Compound 1-1

After 14.0 g (38.1 mM) of Intermediate 1-1-1, 12.2 g (38.1 mM) of di([1,1'-biphenyl]-4-yl)amine, Pd₂(dba)₃, 1.7 g (1.9 mM) of tris(dibenzylideneacetone)dipalladium(0), XPhos, 1.8 g (3.8 mM) of 2-(dicyclohexylphosphino)-2',4',6'-triisopropylbiphenyl and 5.5 g (57.2 mM) of sodium t-butoxide (hereinafter, referred to as t-BuONa) were dissolved in 140 mL of xylene, the resulting solution was refluxed for 6 hours (h). After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hex = 1:4) to obtain 21.2 g (85%) of Target Compound 1-1.

Target Compound A in the following Table 1 was synthesized in the same manner as in the preparation method in Preparation Example 1, except that Compound A in the following Table 1 was used instead of 4-bromo-1-chlorodibenzo[b,d]furan, Compound B in the following Table 1 was used instead of iodobenzene, and Compound C in the following Table 1 was used instead of di([1,1'-biphenyl]-4-yl)amine, in Preparation Example 1.

**[Table 1]**

| **compound No.** | **Compound A** | **Compound 8** | **Compound C** | **Target Compound A** | **Yield** |
|---|---|---|---|---|---|
| 1-9 | 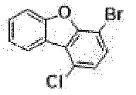 | 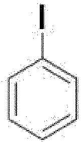 | 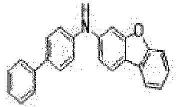 | 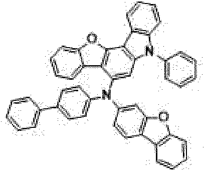 | 50% |
| 1-20 | 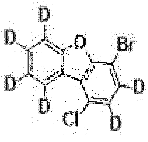 | | 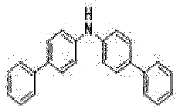 | 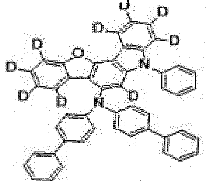 | 48% |
| 1-22 | 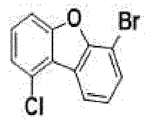 | | 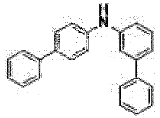 | 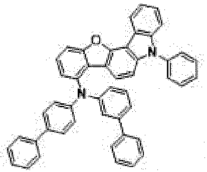 | 49% |
| 1-30 | | | 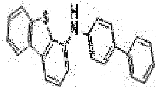 | 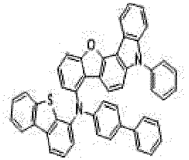 | 51% |
| 1-37 | | 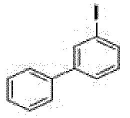 | 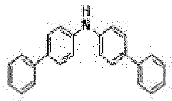 | 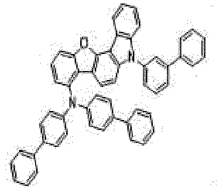 | 48% |
| 1-43 | 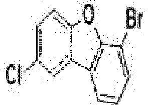 | 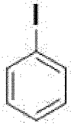 | 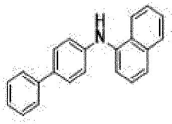 | 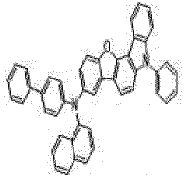 | 49% |
| 1-51 | | | 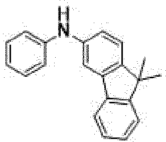 | 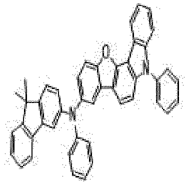 | 52% |
| 1-58 | 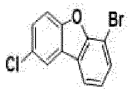 | 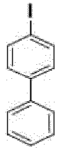 | 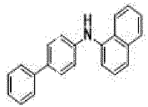 | 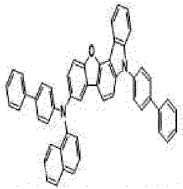 | 50% |
| 1-65 | 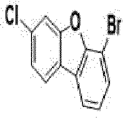 | 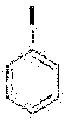 | 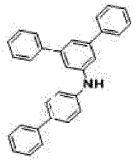 | 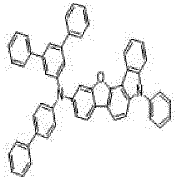 | 51% |
| 1-72 | | | 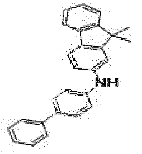 | 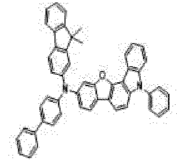 | 53% |
| 1-79 | | 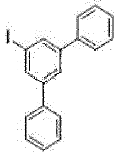 | 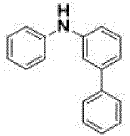 | 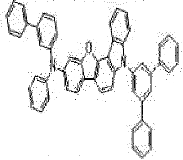 | 49% |

### <Preparation Example 2> Preparation of Compound 1-13

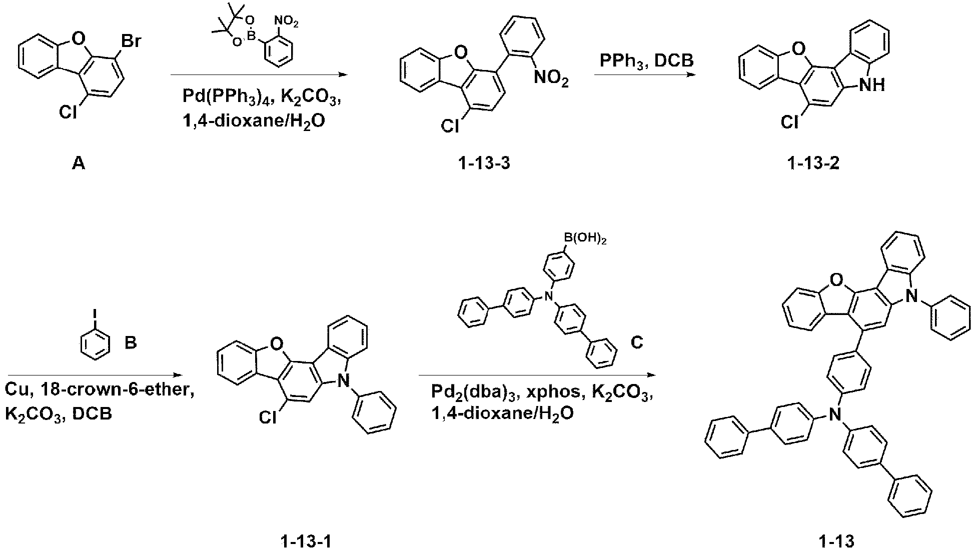

### 1) Preparation of Intermediate 1-13-3

After 20.0 g (71.0 mM) of a compound 4-bromo-1-chlorodibenzo[b,d]furan, 17.7 g (45.3 mM) of 4,4,5,5-tetramethyl-2-(2-nitrophenyl)-1,3,2-dioxaborolane, 4.1 g (3.6 mM) of Pd(PPh₃)₄, and 19.6 g (142.0 mM) of K₂CO₃ were dissolved in 200 mL/40 mL of 1,4-dioxane/water, the resulting solution was refluxed for 6 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hex = 1:5) and recrystallized with methanol to obtain 20.7 g (90%) of Intermediate 1-13-3.

### 2) Preparation of Intermediate 1-13-2

After 20.0 g (61.8 mM) of Intermediate 1-13-3 and 40.5 g (154.5 mM) of PPh₃ were dissolved in 200 mL of DCB, the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hex = 1:4) and recrystallized with methanol to obtain 14.2 g (79%) of Intermediate 1-13-2.

### 3) Preparation of Intermediate 1-13-1

After 14.0 g (48.0 mM) of Intermediate 1-13-12, 10.7 mL (96.0 mM) of iodobenzene, 0.3 g (4.8 mM) of Cu, 1.3 g (4.8 mM) of 18-crown-6-ether, and 13.3 g (96.0 mM) of K₂CO₃ were dissolved in 140 mL of DCB, the resulting solution was refluxed for 48 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hex = 1:4) and recrystallized with methanol to obtain 14.3g (81%) of Intermediate 1-13-1.

### 4) Preparation of Compound 1-13

After 14.0 g (38.1 mM) of Intermediate 1-13-1, 16.8 g (38.1 mM) of 4-(di([1,1'-biphenyl]-4-yl)amino)phenyl)boronic acid, Pd₂(dba)₃, 1.7 g (1.9 mM) of tris(dibenzylideneacetone)dipalladium(0), XPhos, 1.8 g (3.8 mM) of 2-(dicyclohexylphosphino)-2',4',6'-triisopropylbiphenyl and 15.8 g (114.3 mM) of K₂CO₃ were dissolved in 140 mL/30 mL of 1,4-dioxane/distilled water, the resulting solution was refluxed for 6 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hex = 1:5) to obtain 24.2 g (87%) of Target Compound 1-13.

Target Compound A in the following Table 2 was synthesized in the same manner as in the preparation method in Preparation Example 2, except that Compound A in the following Table 2 was used instead of 4-bromo-1-chlorodibenzo[b,d]furan, Compound B in the following Table 2 was used instead of iodobenzene, and Compound C in the following Table 2 was used instead of 4-(di([1,1'-biphenyl]-4-yl)amino)phenyl)boronic acid, in Preparation Example 2.

**[Table 2]**

| **Compound No.** | **Compound A** | **Compound B** | **Compound C** | **Target Compound A** | **Yield** |
|---|---|---|---|---|---|
| 1-34 | 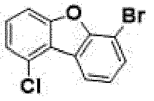 | 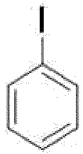 | 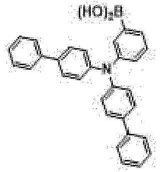 | 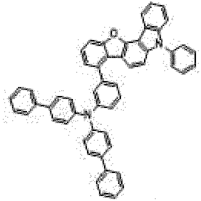 | 50% |
| 1-55 | 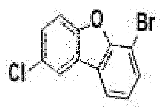 | | 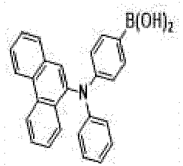 | 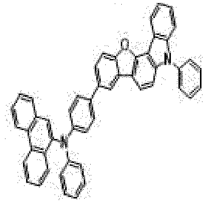 | 51% |
| 1-75 | 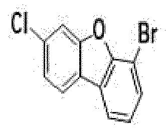 | | 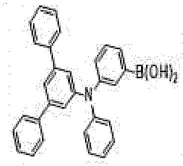 | 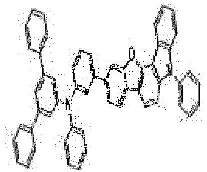 | 48% |

### <Preparation Example 3> Preparation of Compound 2-8

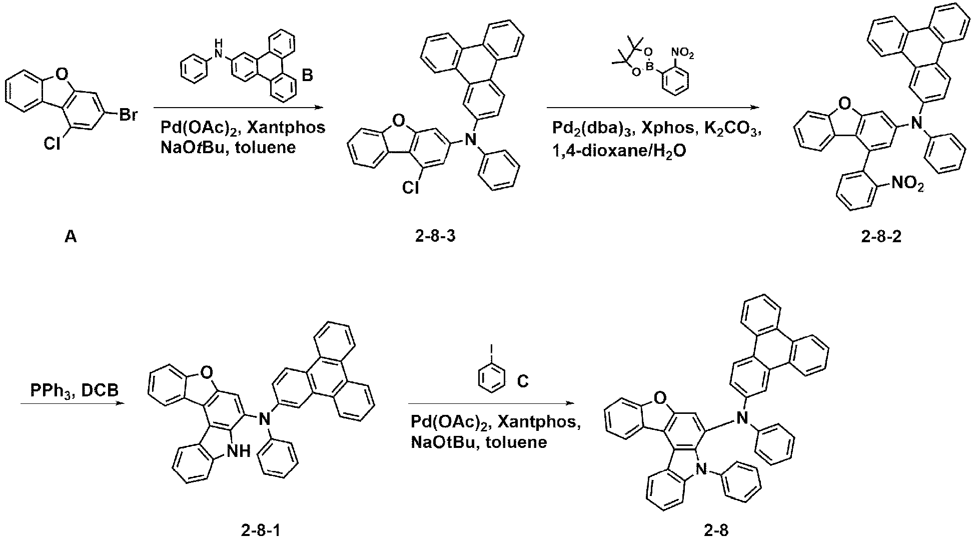

### 1) Preparation of Intermediate 2-8-3

After 20.0 g (71.0 mM) of a compound 3-bromo-1-chlorodibenzo[b,d]furan), 22.7 g (71.0 mM) of N-phenyltriphenylen-2-amine, 0.8 g (3.6 mM) of Pd(OAc)₂, 4.1 g (7.1 mM) of Xantphos, and 13.6 g (142.0 mM) of t-BuONa were dissolved in 200 mL of toluene, the resulting solution was refluxed for 6 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hex = 1:4) and recrystallized with methanol to obtain 32.1 g (87%) of Intermediate 2-8-3.

### 2) Preparation of Intermediate 2-8-2

After 32.0 g (61.5 mM) of Intermediate 2-8-3, 15.3 g (61.5 mM) of 4,4,5,5-tetramethyl-2-(2-nitrophenyl)-1,3,2-dioxaborolane, Pd₂(dba)₃, 2.8 g (3.1 mM) of tris(dibenzylideneacetone)dipalladium(0), XPhos, 2.9 g (6.2 mM) of 2-(dicyclohexylphosphino)-2',4',6'-triisopropylbiphenyl and 25.5 g (184.5 mM) of K₂CO₃ were dissolved in 320 mL/60 mL of 1,4-dioxane/distilled water, the resulting solution was refluxed for 6 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hex = 1:5) and recrystallized with methanol to obtain 29.8 g (80%) of Intermediate 2-8-2.

### 3) Preparation of Intermediate 2-8-1

After 29.0 g (47.8 mM) of Intermediate 2-8-2 and 31.3 g (119.5 mM) of PPh₃ were dissolved in 300 mL of DCB, the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hex = 1:4) and recrystallized with methanol to obtain 21.5 g (78%) of Intermediate 2-8-1.

### 4) Preparation of Compound 2-8

After 21.0 g (36.5 mM) of Intermediate 2-8-1, 8.1 mL (73.0 mM) of iodobenzene, 0.2 g (3.7 mM) of Cu, 1.0 g (3.7 mM) of 18-crown-6-ether, and 10.1 g (73.0 mM) of K₂CO₃ were dissolved in 210 mL of DCB, the resulting solution was refluxed for 48 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hex = 1:3) to obtain 20.2 g (85%) of Target Compound 2-8.

Target Compound A in the following Table 3 was synthesized in the same manner as in the preparation method in Preparation Example 3, except that Compound A in the following Table 3 was used instead of 3-bromo-1-chlorodibenzo[b,d]furan, Compound B in the following Table 3 was used instead of 4,4,5,5-tetramethyl-2-(2-nitrophenyl)-1,3,2-dioxaborolane, and Compound C in the following Table 3 was used instead of iodobenzene, in Preparation Example 3.

**[Table 3]**

| **Compound No.** | **Compound A** | **Compound B** | **Compound C** | **Target Compound A** | **Yield** |
|---|---|---|---|---|---|
| 2-12 | 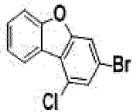 | 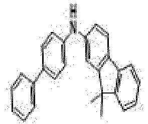 | 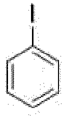 | 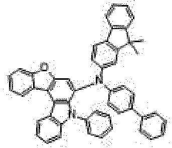 | 48% |
| 2-19 | | 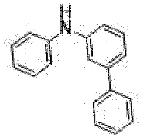 | 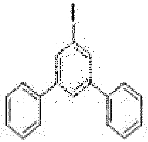 | 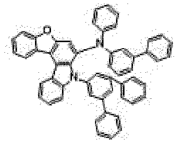 | 49% |
| 2-21 | 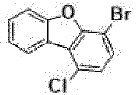 | 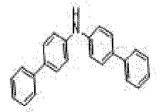 | 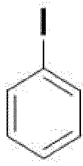 | 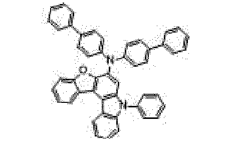 | 46% |
| 2-29 | | 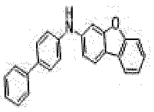 | | 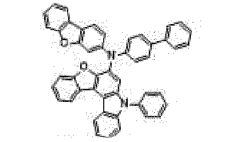 | 45% |
| 2-40 | | 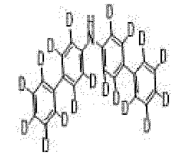 | | 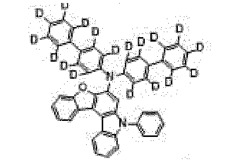 | 47% |
| 2-46 | 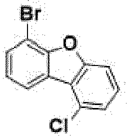 | 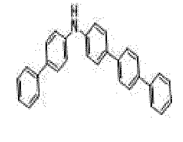 | | 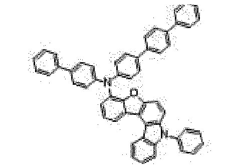 | 45% |
| 2-50 | | 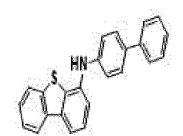 | | 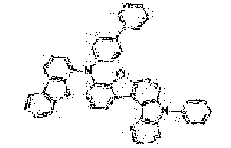 | 46% |
| 2-57 | | 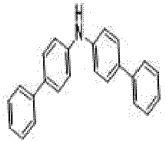 | 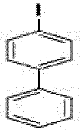 | 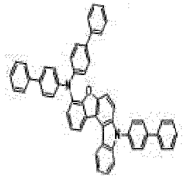 | 45% |
| 2-67 | 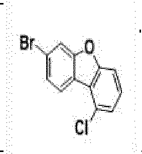 | 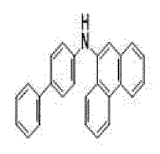 | 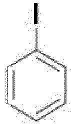 | 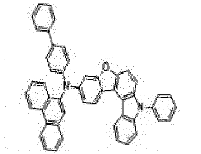 | 49% |
| 2-71 | | 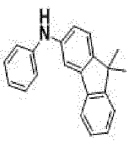 | | 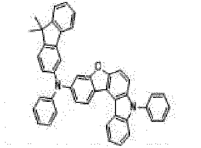 | 44% |
| 2-78 | | 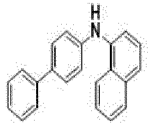 | 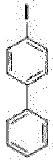 | 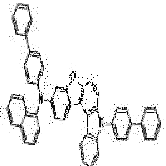 | 50% |

### <Preparation Example 4> Preparation of Compound 2-16

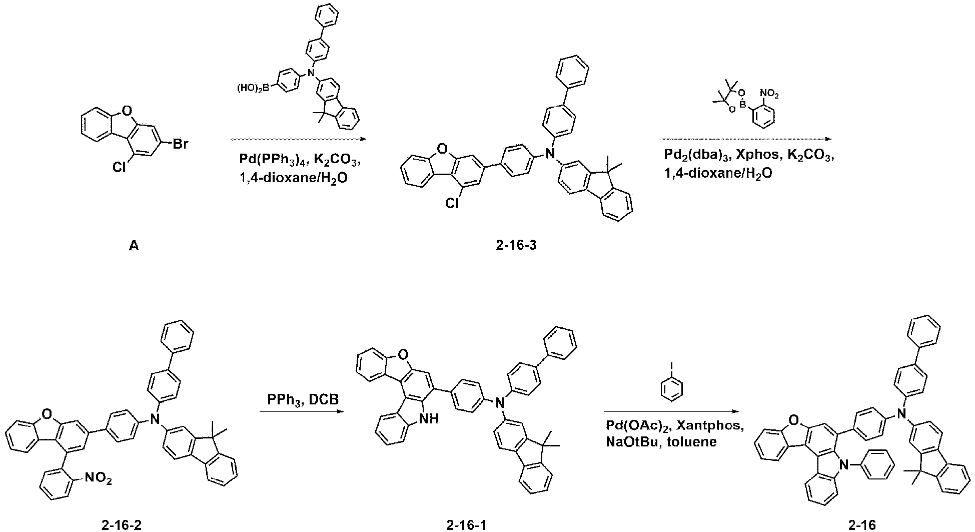

### 1) Preparation of Intermediate 2-16-3

After 20.0 g (71.0 mM) of a compound 3-bromo-1-chlorodibenzo[b,d]furan, 34.2 g (71.0 mM) of 4-([1,1'-biphenyl]-4-yl(9,9-dimethyl-9H-fluoren-2-yl)amino)phenyl)boronic acid, 4.1 g (3.6 mM) of Pd(PPh₃)₄, and 19.6 g (142.0 mM) of K₂CO₃ were dissolved in 200 mL/40 mL of 1,4-dioxane/distilled water, the resulting solution was refluxed for 6 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hex = 1:4) and recrystallized with methanol to obtain 39.9 g (88%) of Intermediate 2-16-3.

### 2) Preparation of Intermediate 2-16-2

After 39.0 g (61.1 mM) of Intermediate 2-16-3, 15.2 g (61.5 mM) of 4,4,5,5-tetramethyl-2-(2-nitrophenyl)-1,3,2-dioxaborolane, Pd₂(dba)₃, 2.8 g (3.1 mM) of tris(dibenzylideneacetone)dipalladium(0), XPhos, 2.9 g (6.1 mM) of 2-(dicyclohexylphosphino)-2',4',6'-triisopropylbiphenyl and 25.3 g (183.3 mM) of K₂CO₃ were dissolved in 390 mL/80 mL of 1,4-dioxane/distilled water, the resulting solution was refluxed for 6 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hex = 1:4) and recrystallized with methanol to obtain 34.1 g (77%) of Intermediate 2-16-2.

### 3) Preparation of Intermediate 2-16-1

After 34.0 g (46.9 mM) of Intermediate 2-16-2 and 30.8 g (117.3 mM) of PPh₃ were dissolved in 340 mL of DCB, the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hex = 1:5) and recrystallized with methanol to obtain 26.0 g (80%) of Intermediate 2-16-1.

### 4) Preparation of Compound 2-16

After 26.0 g (37.5 mM) of Intermediate 2-16-1, 8.4 mL (75.0 mM) of iodobenzene, 0.2 g (3.8 mM) of Cu, 1.0 g (3.8 mM) of 18-crown-6-ether, and 10.4 g (75.0 mM) of K₂CO₃ were dissolved in 260 mL of DCB, the resulting solution was refluxed for 48 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hex = 1:5) to obtain 23.6 g (82%) of Target Compound 2-16.

Target Compound A in the following Table 4 was synthesized in the same manner as in the preparation method in Preparation Example 4, except that Compound A in the following Table 4 was used instead of 3-bromo-1-chlorodibenzo[b,d]furan, Compound B in the following Table 4 was used instead of 4-([1,1'-biphenyl]-4-yl(9,9-dimethyl-9H-fluoren-2-yl)amino)phenyl)boronic acid, and Compound C in the following Table 4 was used instead of iodobenzene, in Preparation Example 4.

**[Table 4]**

| **Compound No.** | **Compound A** | **Compound B** | **Compound C** | **Target Compound A** | **Yield** |
|---|---|---|---|---|---|
| 2-33 | 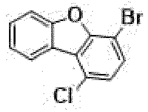 | 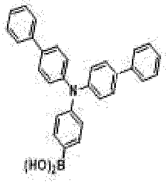 | 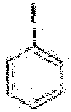 | 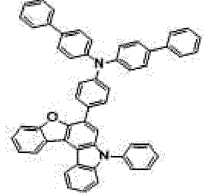 | 45% |
| 2-54 | 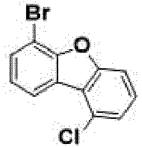 | 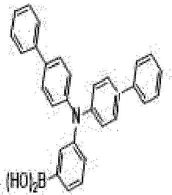 | | 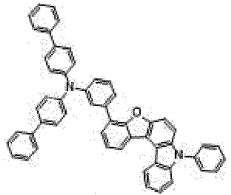 | 43% |
| 2-75 | 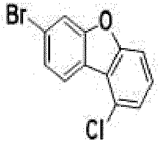 | 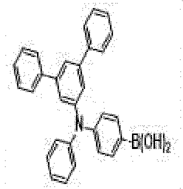 | | 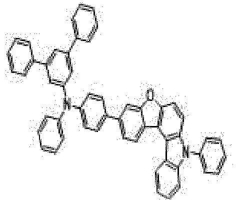 | 46% |

### <Preparation Example 5> Preparation of Compound 1-80

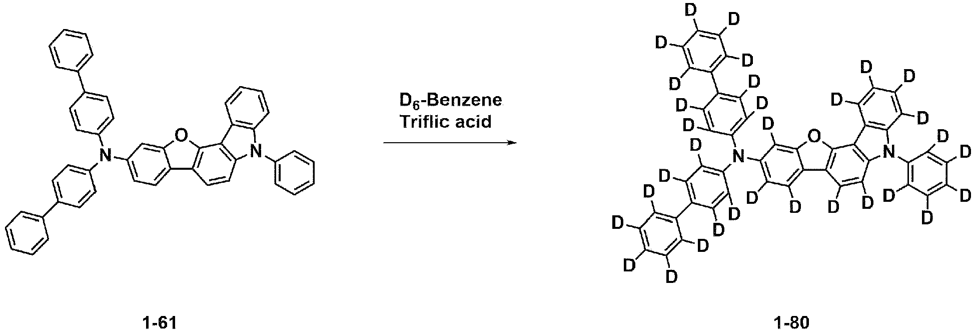

### 1) Preparation of Intermediate 1-61

Intermediate 1-61 was obtained by preparing a compound in the same manner as in the preparation method of Intermediate 1-1-3 of Preparation Example 1, except that 6-bromo-3-chlorodibenzo[b,d]furan was used instead of 4-bromo-1-chlorodibenzo[b,d]furan in the preparation method of Intermediate 1-1-3 in Preparation Example 1.

### 2) Preparation of Compound 1-80

After 30.0 g (46.0 mM) of Intermediate 1-61 and 28.5 ml (322 mM) of triflic acid were dissolved in 300 ml of benzene-D₆, the resulting solution was refluxed for 6 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hex = 1:4) and recrystallized with methanol to obtain 29.9 g (95%) of Target Compound 1-80.

### <Preparation Example 6> Preparation of Compound 2-60

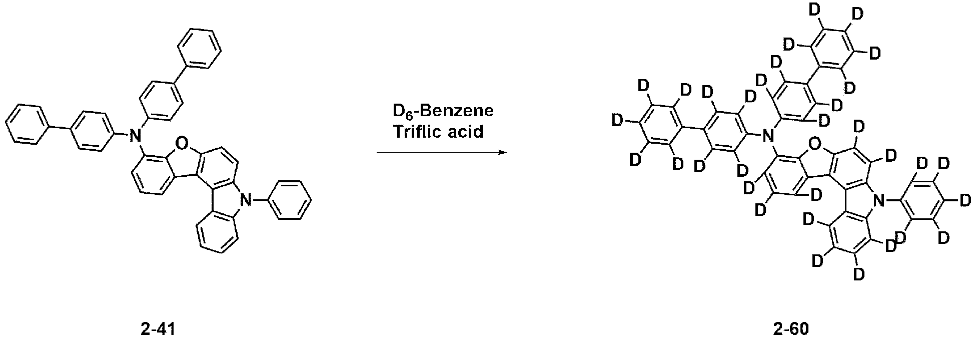

### 1) Preparation of Intermediate 2-41

Intermediate 2-41 was obtained by preparing a compound in the same manner as in the preparation method of Intermediate 1-1-3 of Preparation Example 1, except that 6-bromo-1-chlorodibenzo[b,d]furan was used instead of 3-bromo-1-chlorodibenzo[b,d]furan in the preparation method of Compound 2-8-3 in Preparation Example 3.

### 2) Preparation of Compound 2-60

After 30.0 g (46.0 mM) of Intermediate 2-41 and 28.5 ml (322 mM) of triflic acid were dissolved in 300 ml of benzene-D₆, the resulting solution was refluxed for 6 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hex = 1:5) and recrystallized with methanol to obtain 29.3 g (93%) of Target Compound 2-60.

The compounds other than the compounds described in Tables 1 to 4 were also prepared in the same manner as in the above-described Preparation Examples.

The synthesis confirmation results of the compounds produced above are shown in Tables 5 and 6. Table 5 shows the measured values of ¹H NMR(CDCl₃, 200 Mz), and Table 6 shows the measured values of field desorption mass spectrometry (FD-MS).

**[Table 5]**

| Compound No. | ¹H NMR(CDCl₃, 200Mz) |
|---|---|
| 1-1 | δ = 8.55(1H, d), 7.94(1H, d), 7.89(1H, d), 7.25-7.66(24H, m), 6.69(4H, d), 6.33 (1H, s) |
| 1-9 | δ = 8.55(1H, d), 7.94(1H, d), 7.89(2H, d), 7.25-7.66(22H, m), 6.69(2H, d), 6.33(2H, d) |
| 1-13 | δ = 8.55(1H, d), 7.94(1H, d), 7.89(1H, d), 7.25-7.66(27H, m), 6.69(6H, d) |
| 1-20 | δ = = 7.41-7.58(19H, m), 6.69(4H, d) |
| 1-22 | δ = 8.55(1H, d), 7.94(1H, d), 7.89(1H, d), 7.25-7.54 (20H, m), 7.13(2H, t), 7.02(1H, d), 6.89(1H, s), 6.88(1H, d), 6.69(2H, d), 6.59(1H, d), 6.33(1H, d) |
| 1-30 | δ = 8.55(1H, d), 8.45(1H, d), 7.89-7.98 (3H, m), 7.81(1H, d), 7.25-7.58(17H, m), 7.13(2H, t), 7.02(1H, d), 6.86(1H, d), 6.69(2H, d), 6.33(1H, d) |
| 1-34 | δ = 8.55(1H, d), 7.94 (1H, d), 7.89(1H, d), 7.75(1H, d), 7.25-7.62(24H, m), 7.13(1H, d), 6.89(1H, s), 6.88(1H, d), 6.69(4H, d), 6.59(1H, d) |
| 1-37 | δ = 8.55(1H, d), 8.09(1H, s), 7.94(1H, d), 7.89(1H, d), 7.25-7.54(24H, m), 7.13(2H, t), 7.02(1H, d), 6.69(4H, d), 6.33(1H, d) |
| 1-43 | δ = 8.55(1H, d), 8.07(1H, d), 8.02(1H, d), 7.94(1H, d), 7.89(1H, d), 7.25-7.65(20H, m), 7.13(1H, d), 6.98(1H, d), 6.69(2H, d), 6.39(1H, d) |
| 1-51 | δ = 8.55(1H, d), 7.87-7.94(3H, m), 7.13-7.65(16H, m), 7.04(1H, s), 6.81(1H, t), 6.63(2H, d), 6.48(1H, d), 6.39(1H, d), 1.72(6H, s) |
| 1-55 | δ = 8.93(2H, d), 8.55(1H, d), 8.12(2H, d), 7.71-7.94(9H, m), 7.45-7.58(7H, m), 7.13-7.33(5H, m), 6.91(1H, s), 6.81(1H, t), 6.69(2H, d), 6.63(2H, d) |
| 1-58 | δ = 8.55(1H, d), 8.07(1H, d), 8.02(1H, d), 7.94 (1H, d), 7.89(1H, d), 7.79(2H, d), 7.68(2H, d), 7.65(1H, s), 7.25-7.57(19H, m), 7.13(1H, d), 6.98(1H, d), 6.69(2H, d), 6.39(1H, d) |
| 1-65 | δ = 8.55(1H, d), 7.94(1H, d), 7.89(1H, d), 7.25-7.64(26H, m), 7.13(1H, d), 7.06(1H, s), 6.85(2H, s), 6.69(2H, d), 6.33(1H, d) |
| 1-72 | δ = 8.55(1H, d), 7.87-7.94(3H, m), 7.25-7.64(20H, m), 7.13(1H, d), 6.75(1H, s), 6.69(2H, d), 6.58(1H, d), 6.33(1H, d), 1.72(6H, s) |
| 1-75 | δ = 8.55(1H, d), 7.89-7.95(3H, m), 7.75(1H, d), 7.13-7.64(22H, m), 7.06(1H, s), 6.81-6.89(5H, m), 6.63(2H, d), 6.59(1H, d) |
| 1-79 | δ = 8.55(1H, d), 8.05(2H, d), 7.88-7.94 (3H, m), 7.64(1H, d), 7.13-7.52(22H, m), 6.81-6.89(3H, m), 6.63(2H, d), 6.59(1H, d), 6.33(1H, d) |
| 2-8 | δ = 8.93(2H, d), 8.55(1H, d), 8.13(1H, s), 8.12(2H, d), 7.82-7.94(7H, m), 7.20-7.66(13H, m), 7.02(1H, d), 6.81(1H, t), 6.63(2H, d) |
| 2-12 | δ = 8.55(1H, d), 7.87-7.94(3H, m), 7.25-7.66(22H, m), 6.75(1H, s), 6.69(2H, d), 6.58(1H, d), 1.72(6H, s) |
| 2-16 | δ = 8.55(1H, d), 7.87-7.94(3H, m), 7.25-7.66(24H, m), 6.75(1H, d), 6.69(4H, d), 6.58(1H, d), 1.72(6H, s) |
| 2-19 | δ = 8.55(1H, d), 8.05(2H, s), 7.88-7.94(3H, m), 7.66(1H, d), 7.20-7.52(23H, m), 6.81-6.89(3H, m), 6.63(2H, d), 6.59(1H, d) |
| 2-21 | δ = 8.55(1H, d), 7.94 (1H, d), 7.89(1H, d), 7.25-7.66(24H, m), 6.69(4H, d), 6.39(1H, s) |
| 2-29 | δ = 8.55(1H, d), 7.94(1H, d), 7.89(2H, d), 7.25-7.66(22H, m), 6.69(2H, d), 6.39(1H, s), 6.33(1H, d) |
| 2-33 | δ = 8.55(1H, d), 7.94(1H, d), 7.89(1H, d), 7.25-7.66(27H, m), 6.69(6H, d) |
| 2-40 | δ = 8.55(1H, d), 7.94(1H, d), 7.89(1H, d), 7.25-7.66(10H, m), 6.39(1H, s) |
| 2-46 | δ = 8.55(1H, d), 7.94(1H, d), 7.25-7.66(28H, m), 7.07(1H, t), 6.69(4H, d), 6.39(1H, d) |
| 2-50 | δ = 8.55(1H, d), 8.45(1H, d), 7.98(1H, d), 7.94(1H, d), 7.81(1H, d), 7.19-7.66(20H, m), 7.07(1H, t), 6.86(1H, d), 6.69(2H, d), 6.39(1H, d) |
| 2-54 | δ = 8.55(1H, d), 7.94 (1H, d), 7.85(1H, d), 7.81(1H, d), 7.19-7.66(25H, m), 6.89(1H, d), 6.88(1H, d), 6.69(4H, d), 6.59(1H, d) |
| 2-57 | δ = 8.55(1H, d), 7.94(1H, d), 7.79(2H, d), 7.68(2H, d), 7.66(1H, d), 7.19-7.54(23H, m), 7.07(1H, t), 6.69(4H, d), 6.39(1H, d) |
| 2-67 | δ = 8.93(2H, d), 8.55(1H, d), 8.12(2H, d), 7.82-7.94(5H, m), 7.19-7.66(18H, m), 6.91(1H, s), 6.69(2H, d), 6.33(1H, d) |
| 2-71 | δ = 8.55(1H, d), 7.94 (1H, d), 7.87 (1H, d), 7.19-7.66(17H, m), 7.04(1H, s), 6.81 (1H, t), 6.63(2H, d), 6.48(1H, d), 6.33(1H, d), 1.72(6H, s) |
| 2-75 | δ = 8.55(1H, d), 7.95(1H, d), 7.94 (1H, d), 7.75(1H, d), 7.19-7.66(24H, m), 7.06(1H, s), 6.85(2H, s), 6.81(1H, t), 6.69(2H, d), 6.63(2H, d) |
| 2-78 | δ = 8.55(1H, d), 8.07(1H, d), 8.02(1H, d), 7.94(1H, d), 7.79(2H, d), 7.19-7.66(24H, m), 6.98(1H, d), 6.69(2H, d), 6.33(1H, d) |

**[Table 6]**

| Compound | FD-MS | Compound | FD-MS |
|---|---|---|---|
| 1-1 | m/z= 652.25 (C₄₈H₃₂N₂O=652.78) | 1-9 | m/z= 666.23 (C₄₈H₃₀N₂O₂=666.76) |
| 1-13 | m/z= 728.28 (C₅₄H₃₆N₂O=728.88) | 1-20 | m/z= 661.31 (C₄₈H₂₃D₉N₂O=661.84) |
| 1-22 | m/z= 652.25 (C₄₈H₃₂N₂O=652.78) | 1-30 | m/z= 682.21 (C₄₈H₃₀N₂OS=682.83) |
| 1-34 | m/z= 728.28 (C₅₄H₃₆N₂O=728.88) | 1-37 | m/z= 728.28 (C₅₄H₃₆N₂O=728.88) |
| 1-43 | m/z= 626.24 (C₄₆H₃₀N₂O=626.74) | 1-51 | m/z= 616.25 (C₄₅H₃₂N₂O=616.75) |
| 1-55 | m/z= 676.25 (C₅₀H₃₂N₂O=676.80) | 1-58 | m/z= 702.27 (C₅₂H₃₄N₂O=702.84) |
| 1-65 | m/z= 728.28 (C₅₄H₃₆N₂O=728.88) | 1-72 | m/z= 692.28 (C₅₁H₃₆N₂O=692.84) |
| 1-75 | m/z= 728.28 (C₅₄H₃₆N₂O=728.88) | 1-79 | m/z= 728.28 (C₅₄H₃₆N₂O=728.88) |
| 1-80 | m/z= 684.45 (C₄₈D₃₂N₂O=684.98) | 2-8 | m/z= 650.24 (C₄₈H₃₀N₂O=650.76) |
| 2-12 | m/z= 692.28 (C₅₁H₃₆N₂O=692.84) | 2-16 | m/z= 768.31 (C₅₇H₄₀N₂O=768.94) |
| 2-19 | m/z= 728.28 (C₅₄H₃₆N₂O=728.88) | 2-21 | m/z= 652.25 (C₄₈H₃₂N₂O=652.78) |
| 2-29 | m/z= 666.23 (C₄₈H₃₀N₂O₂=666.76) | 2-33 | m/z= 728.28 (C₅₄H₃₆N₂O=728.88) |
| 2-40 | m/z= 670.36 (C₄₈H₁₄D₁₈N₂O=670.89) | 2-46 | m/z= 728.28 (C₅₄H₃₆N₂O=728.88) |
| 2-50 | m/z= 682.21 (C₄₈H₃₀N₂OS=682.83) | 2-54 | m/z= 728.28 (C₅₄H₃₆N₂O=728.88) |
| 2-57 | m/z= 728.28 (C₅₄H₃₆N₂O=728.88) | 2-60 | m/z= 684.45 (C₄₈D₃₂N₂O=684.98) |
| 2-67 | m/z= 676.25 (C₅₀H₃₂N₂O=676.80) | 2-71 | m/z= 616.25 (C₄₅H₃₂N₂O=616.75) |
| 2-75 | m/z= 728.28 (C₅₄H₃₆N₂O=728.88) | 2-78 | m/z= 702.27 (C₅₂H₃₄N₂O=702.84) |

### <Experimental Example 1>

### 1) Manufacture of organic light emitting device

### Comparative Example 1

A glass substrate, in which ITO was thinly coated to have a thickness of 1,500 Å, was ultrasonically washed with distilled water. When the washing with distilled water was finished, the glass substrate was ultrasonically washed with a solvent such as acetone, methanol, and isopropyl alcohol, dried and then was subjected to UVO treatment for 5 minutes using UV in a UV cleaning machine. Thereafter, the substrate was transferred to a plasma washing machine (PT), and then was subjected to plasma treatment in a vacuum state for an ITO work function and in order to remove a residual film, and was transferred to a thermal deposition apparatus for organic deposition.

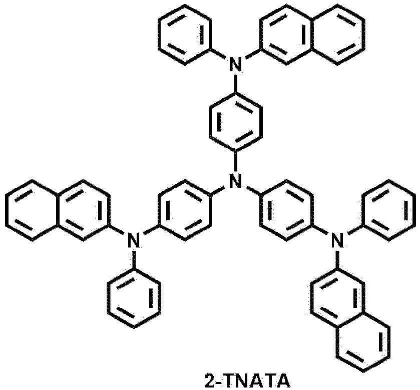

Subsequently, air in the chamber was evacuated until the degree of vacuum in the chamber reached 10⁻⁶ torr, and then a hole injection layer having a thickness of 600 Å was deposited on the ITO substrate by applying current to the cell to evaporate 2-TNATA. A hole transport layer having a thickness of 300 Å was deposited on the hole injection layer by placing the following N,N'-bis(α-naphthyl)-N,N'-diphenyl-4,4'-diamine (NPB) in another cell in the vacuum deposition apparatus and applying current to the cell to evaporate NPB.

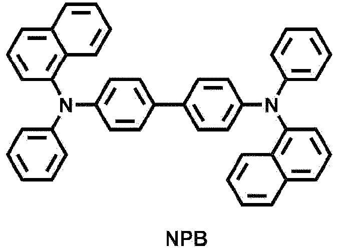

A light emitting layer was thermally vacuum deposited thereon as follows. The light emitting layer was deposited by depositing a compound of 9-[4-(4,6-diphenyl-1,3,5-triazin-2-yl)phenyl]-9'-phenyl-3,3'-Bi-9H-carbazole as a host to have a thickness of 400 Å and doping the deposited layer with a green phosphorescent dopant Ir(ppy)₃ at 7% of the thickness of the host. Thereafter, BCP as a hole blocking layer was deposited to have a thickness of 60 Å, and Alq₃ as an electron transport layer was deposited to have a thickness of 200 Å thereon. Finally, an organic light emitting device of Comparative Example 1 was manufactured by depositing lithium fluoride (LiF) to have a thickness of 10 Å on the electron transport layer to form an electron injection layer, and then depositing an aluminum (Al) negative electrode to have a thickness of 1,200 Å on the electron injection layer to form a negative electrode.

Meanwhile, all the organic compounds required for manufacturing an organic light emitting device were subjected to vacuum sublimed purification under 10⁻⁸ to 10⁻⁶ torr for each material, and used for the manufacture of the organic light emitting device.

### Examples 1 to 34

Organic light emitting devices of Examples 1 to 34 were manufactured in the same manner as in the manufacturing method of the organic light emitting device of Comparative Example 1, except that the compounds of Examples 1 to 34 in the following Table 7 were used instead of Compound NPB used during the forming of the hole transport layer in Comparative Example 1.

### Comparative Examples 2 to 9

Organic light emitting devices of Comparative Examples 2 to 9 were manufactured in the same manner as in the manufacturing method of the organic light emitting device of Comparative Example 1, except that the compounds of M1 to M8 in the following Table 7 were used instead of Compound NPB used during the forming of the hole transport layer in Comparative Example 1.
In this case, Compounds M1 to M8 used in Comparative Examples 2 to 9 are as follows.

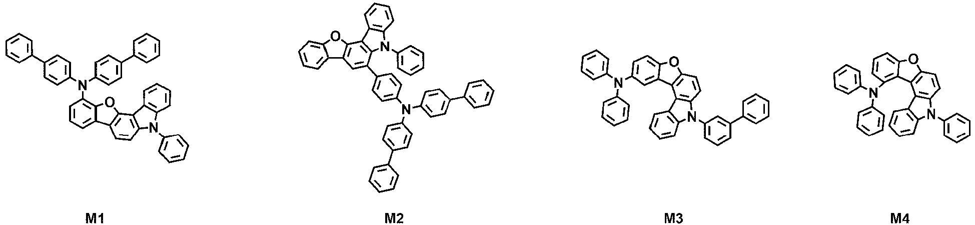

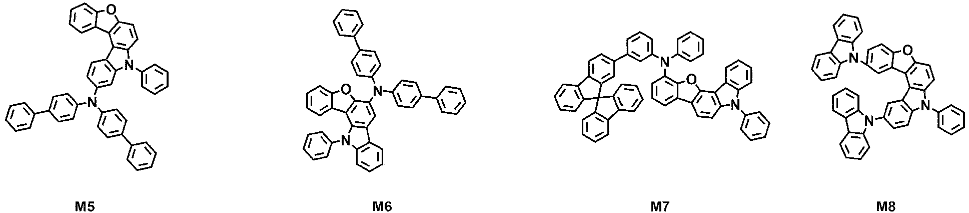

### (2) Driving voltage and light emitting efficiency of organic light emitting device

For the organic light emitting devices of Examples 1 to 34 and Comparative Examples 1 to 9 manufactured as above, electroluminescent light emission (EL) characteristics were measured using M7000 manufactured by McScience Inc., and with the measurement results, a service life T₉₀ (unit: h, hour), which was the time when the luminance became 90% compared to the initial luminance when the standard luminance was 6,000 cd/m², was measured using a service life measurement apparatus (M6000) manufactured by McScience Inc.

The characteristics of the organic light emitting devices of the present invention shown with the measurement results are shown in the following Table 7.

**[Table 7]**

| | Compound | Driving voltage (V) | Light emitting efficiency (cd/A) | Service life (T₉₀) |
|---|---|---|---|---|
| Example 1 | 1-1 | 3.74 | 132.08 | 269 |
| Example 2 | 1-9 | 3.80 | 131.70 | 265 |
| Example 3 | 1-13 | 3.87 | 129.84 | 261 |
| Example 4 | 1-20 | 3.55 | 138.27 | 271 |
| Example 5 | 1-22 | 4.10 | 127.89 | 245 |
| Example 6 | 1-30 | 4.15 | 127.62 | 241 |
| Example 7 | 1-34 | 4.19 | 127.48 | 240 |
| Example 8 | 1-37 | 4.12 | 127.72 | 244 |
| Example 9 | 1-43 | 4.31 | 125.87 | 227 |
| Example 10 | 1-51 | 4.35 | 125.32 | 221 |
| Example 11 | 1-55 | 4.37 | 125.12 | 220 |
| Example 12 | 1-58 | 4.32 | 125.52 | 225 |
| Example 13 | 1-65 | 4.40 | 123.85 | 209 |
| Example 14 | 1-72 | 4.44 | 122.76 | 206 |
| Example 15 | 1-75 | 4.47 | 122.69 | 199 |
| Example 16 | 1-79 | 4.41 | 123.02 | 208 |
| Example 17 | 1-80 | 3.52 | 139.05 | 275 |
| Example 18 | 2-8 | 4.21 | 126.92 | 239 |
| Example 19 | 2-12 | 4.27 | 126.79 | 235 |
| Example 20 | 2-16 | 4.29 | 126.57 | 231 |
| Example 21 | 2-19 | 4.22 | 126.34 | 237 |
| Example 22 | 2-21 | 3.81 | 131.70 | 259 |
| Example 23 | 2-29 | 3.92 | 130.78 | 255 |
| Example 24 | 2-33 | 3.97 | 128.89 | 251 |
| Example 25 | 2-40 | 3.61 | 137.44 | 265 |
| Example 26 | 2-46 | 4.44 | 121.59 | 207 |
| Example 27 | 2-50 | 4.49 | 120.84 | 195 |
| Example 28 | 2-54 | 4.47 | 120.76 | 191 |
| Example 29 | 2-57 | 4.45 | 121.29 | 206 |
| Example 30 | 2-60 | 3.57 | 138.29 | 269 |
| Example 31 | 2-67 | 4.30 | 124.99 | 218 |
| Example 32 | 2-71 | 4.38 | 124.62 | 212 |
| Example 33 | 2-75 | 4.38 | 124.52 | 211 |
| Example 34 | 2-78 | 4.35 | 124.75 | 215 |
| Comparative Example 1 | NPB | 4.51 | 110.59 | 150 |
| Comparative Example 2 | M1 | 4.61 | 117.92 | 178 |
| Comparative Example 3 | M2 | 4.63 | 117.75 | 177 |
| Comparative Example 4 | M3 | 4.68 | 117.52 | 172 |
| Comparative Example 5 | M4 | 4.65 | 117.65 | 175 |
| Comparative Example 6 | M5 | 4.69 | 117.38 | 171 |
| Comparative Example 7 | M6 | 4.55 | 118.93 | 183 |
| Comparative Example 8 | M7 | 4.69 | 116.30 | 165 |
| Comparative Example 9 | M8 | 4.65 | 116.49 | 166 |

As shown in Table 7, the organic light emitting devices of Examples 1 to 34, in which a hole transport layer was formed using the heterocyclic compound of any of Chemical Formulae 2 to 9 according to the present invention, have characteristics of long service life, low voltage and high efficiency.

Specifically, the organic light emitting devices of Examples 1 to 34 have a structure in which benzofurocarbazole is substituted with two types of substituents, that is, an amine group and an aryl group to delocalize the highest occupied molecular orbital (HOMO) energy level, thereby increasing the hole transport ability and stabilizing the HOMO energy.

Therefore, when the heterocyclic compound of any of Chemical Formulae 2 to 9 is used as a material for the hole transport layer in the organic light emitting device, an appropriate energy level and an appropriate band gap are formed to increase excitons in the light emitting region. Increasing excitons in the light emitting region means having an effect of reducing the driving voltage of the device and an effect of increasing efficiency. In the case of the organic light emitting devices of Comparative Examples 1 to 9 in which the compound according to the present application is not used during the formation of the hole transport layer, it can be confirmed that the light emitting efficiency and the service life are lower than those of Examples 1 to 34.

In particular, in the cases of Examples 4, 17, 25 and 30 using the compound substituted with deuterium, it was possible to confirm an effect of further improving the driving voltage, light emitting efficiency and service life. Specifically, Compound 1-1 of Example 1 and Compound 1-20 of Example 4 have the same compound structure, but have a difference in the presence or absence of deuterium substitution, and it could be seen that the driving voltage, light emitting efficiency and service life of Example 4 with deuterium substitution are better than those of Example 1.

## Claims

1. A heterocyclic compound represented by any one of the following Chemical Formulae 2 to 9: 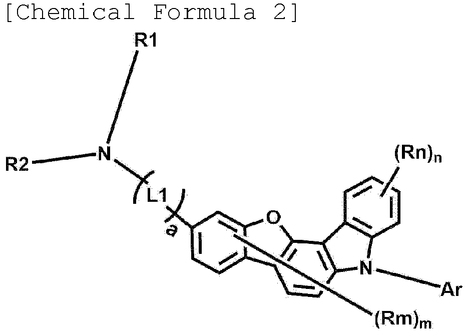 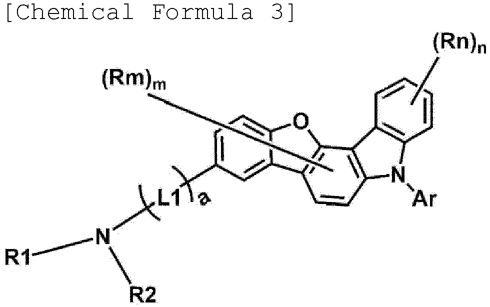 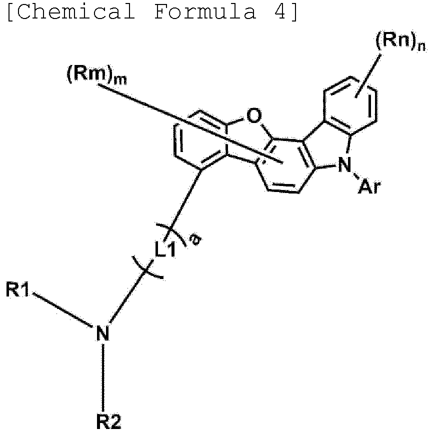 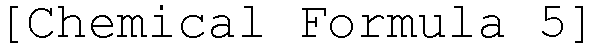 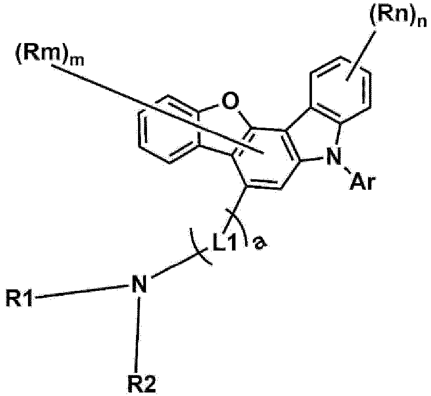 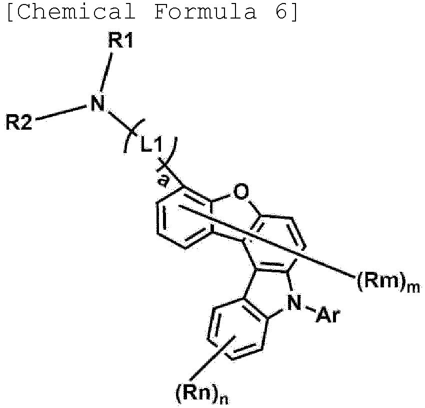 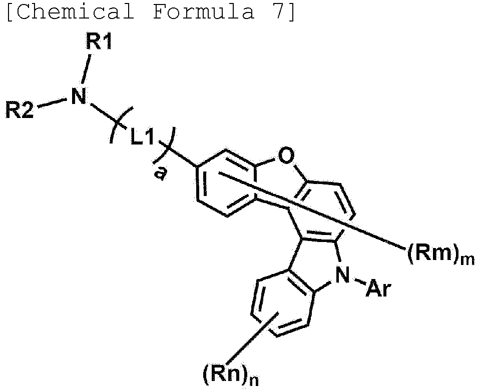 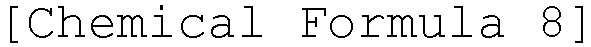 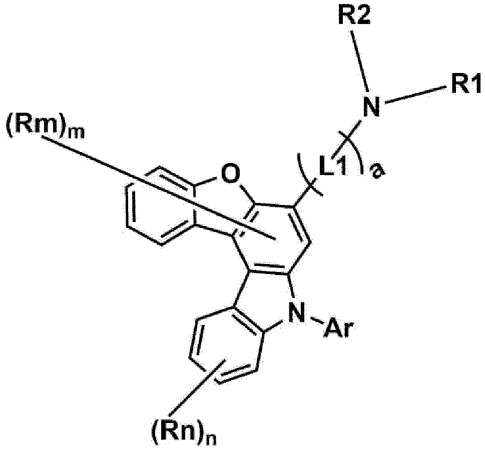 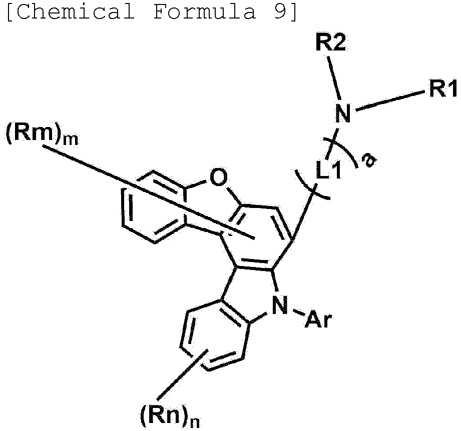 wherein, in Chemical Formulae 2 to 9,
L1 is a direct bond; or a substituted or unsubstituted arylene group having 6 to 60 carbon atoms, a is an integer from 0 to 3, and when a is 2 or higher, L1's in the parenthesis are the same as or different from each other,
R1 is a phenyl group unsubstituted or substituted with one or more deuteriums; a biphenyl group unsubstituted or substituted with one or more deuteriums; a terphenyl group unsubstituted or substituted with one or more deuteriums; a naphthyl group unsubstituted or substituted with one or more deuteriums; a phenanthrene group unsubstituted or substituted with one or more deuteriums; a pyrene group; a fluorene group unsubstituted or substituted with one or more substituents selected from the group consisting of deuterium and a methyl group; a triphenylenyl group unsubstituted or substituted with one or more deuteriums; a dibenzofuran group unsubstituted or substituted with one or more deuteriums; or a dibenzothiophene group unsubstituted or substituted with one or more deuteriums,
R2 is a phenyl group unsubstituted or substituted with one or more deuteriums; a biphenyl group unsubstituted or substituted with one or more deuteriums; a terphenyl group unsubstituted or substituted with one or more deuteriums; a naphthyl group unsubstituted or substituted with one or more deuteriums; a phenanthrene group unsubstituted or substituted with one or more deuteriums; a dibenzofuran group unsubstituted or substituted with one or more deuteriums; or a dibenzothiophene group unsubstituted or substituted with one or more deuteriums,
Rm and Rn are the same as or different from each other, and are each independently hydrogen; or deuterium,
m is an integer from 0 to 5, and when m is 2 or higher, Rm's in the parenthesis are the same as or different from each other,
n is an integer from 0 to 4, and when n is 2 or higher, Rn's in the parenthesis are the same as or different from each other, and
Ar is a substituted or unsubstituted aryl group having 6 to 60 carbon atoms.

2. The heterocyclic compound of claim 1, wherein a deuterium content of the heterocyclic compound represented by any one of Chemical Formulae 2 to 9 is 10% to 100%.

3. The heterocyclic compound of claim 1, wherein Ar is a substituted or unsubstituted aryl group having 6 to 40 carbon atoms.

4. The heterocyclic compound of claim 1, wherein L1 is a direct bond; or a substituted or unsubstituted arylene group having 6 to 40 carbon atoms.

5. The heterocyclic compound of claim 1, wherein the heterocyclic compound is represented by any one of the following compounds:

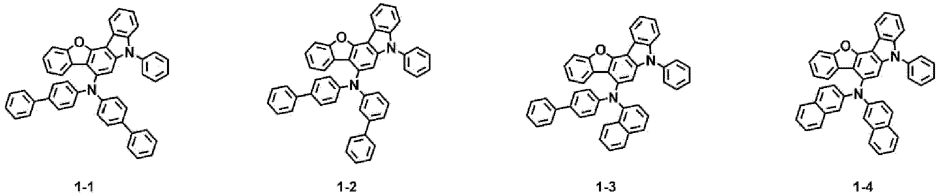

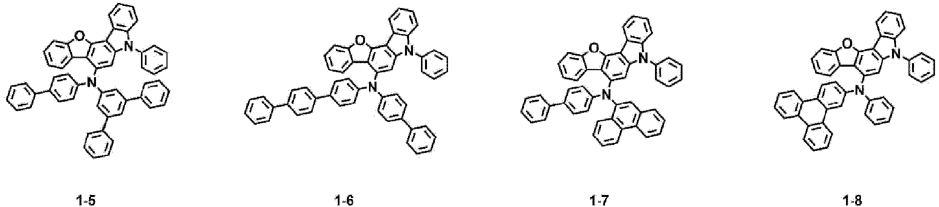

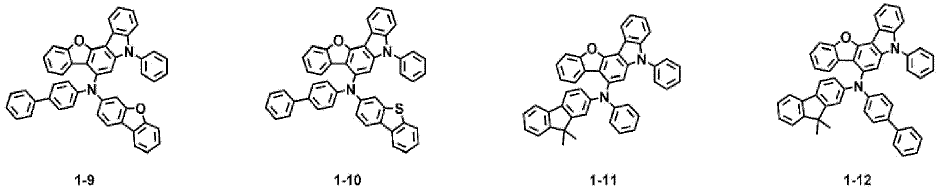

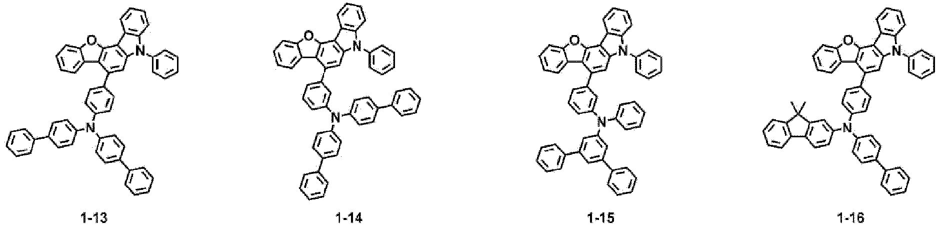

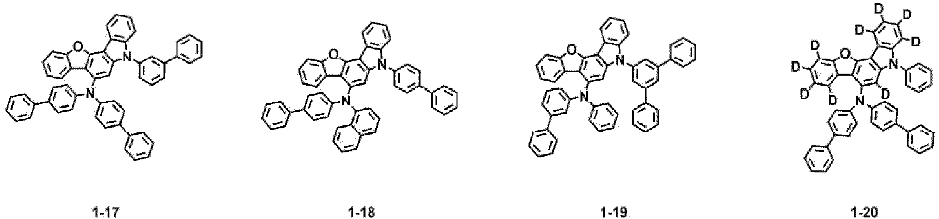

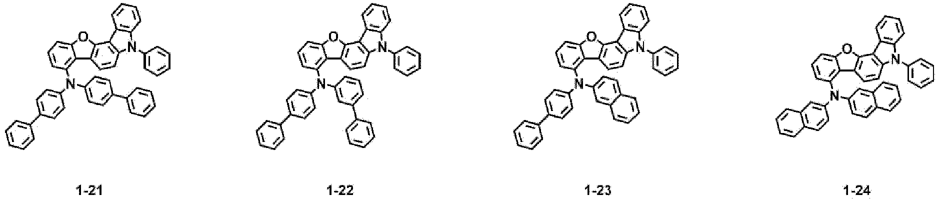

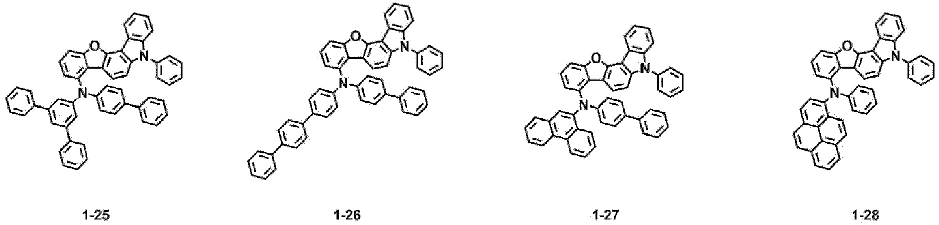

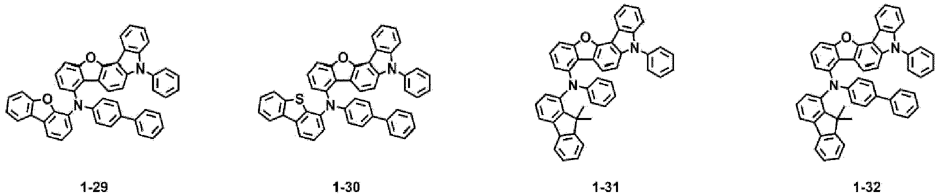

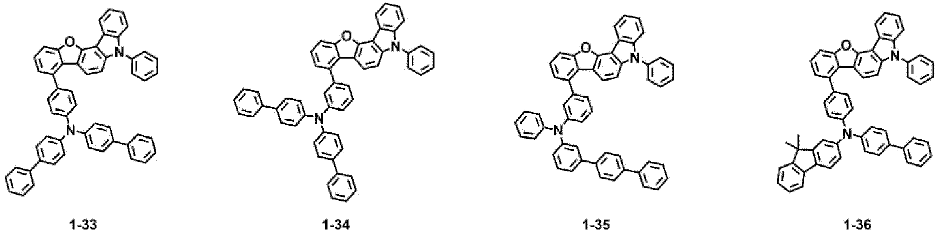

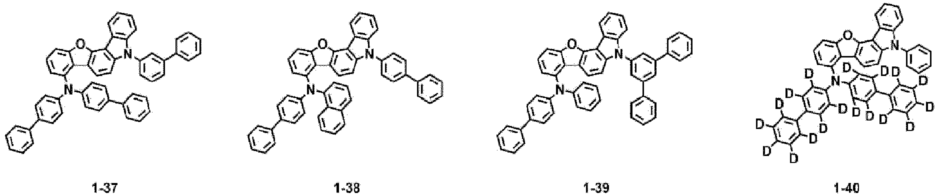

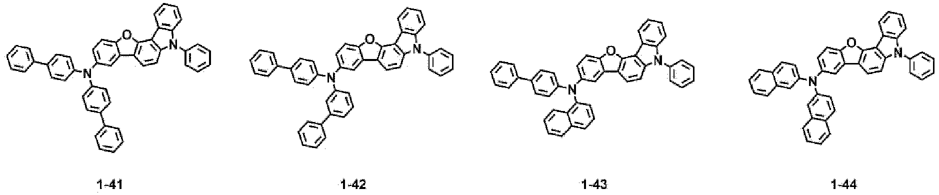

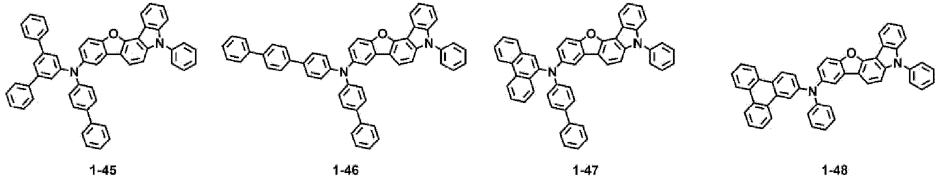

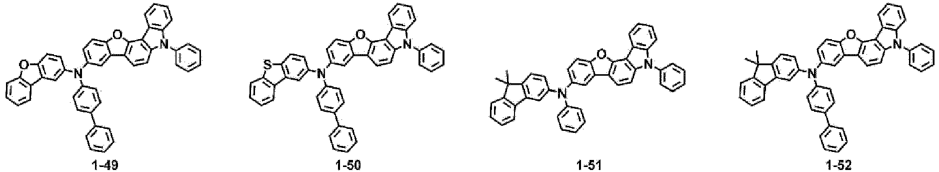

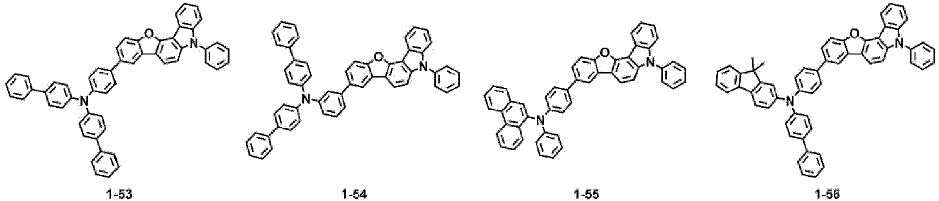

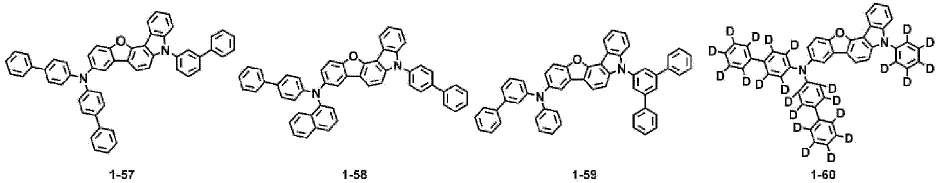

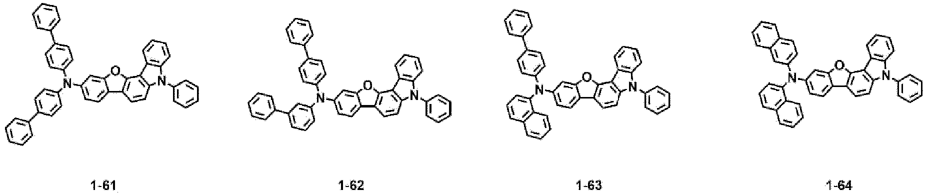

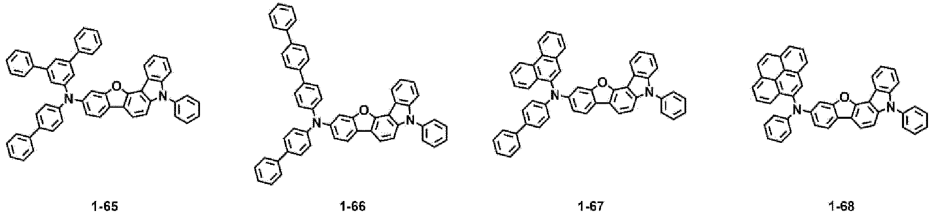

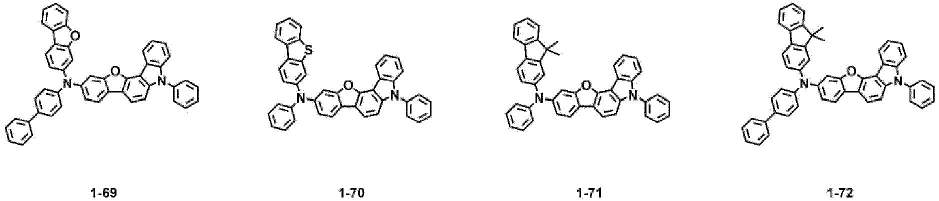

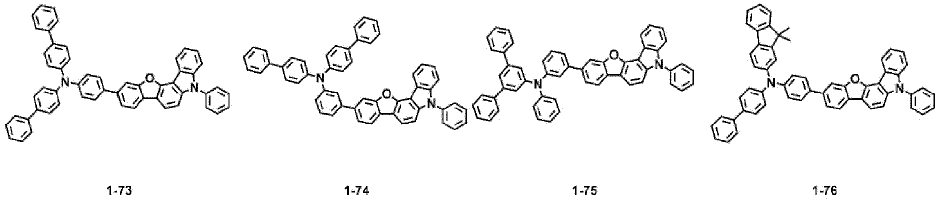

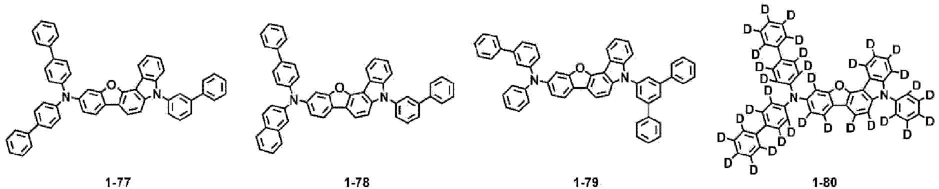

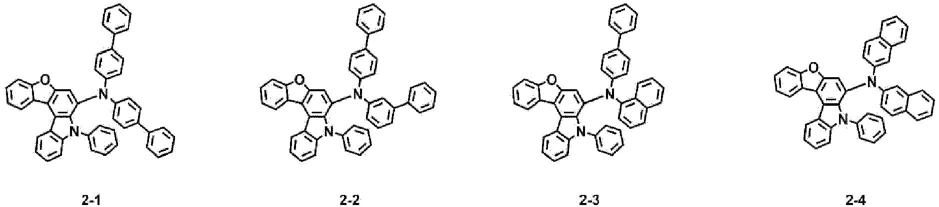

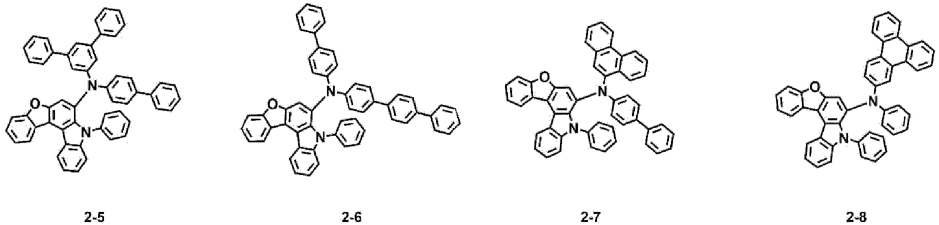

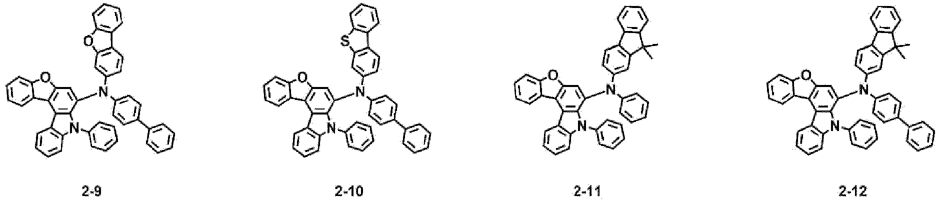

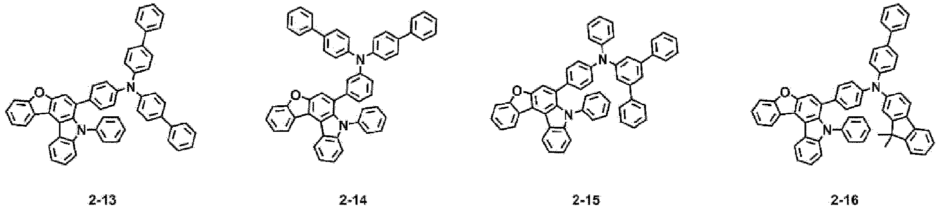

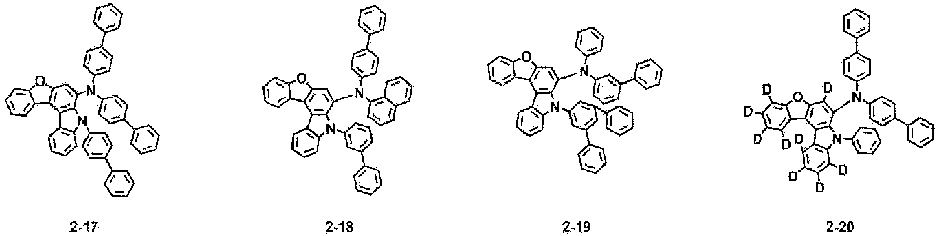

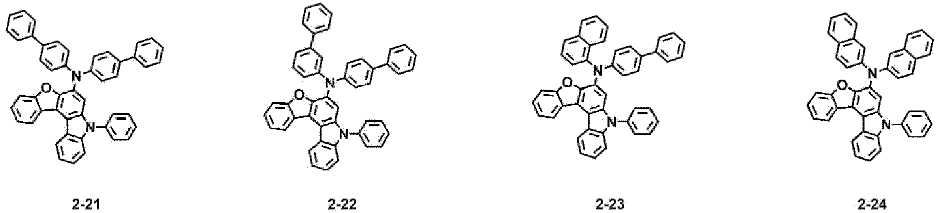

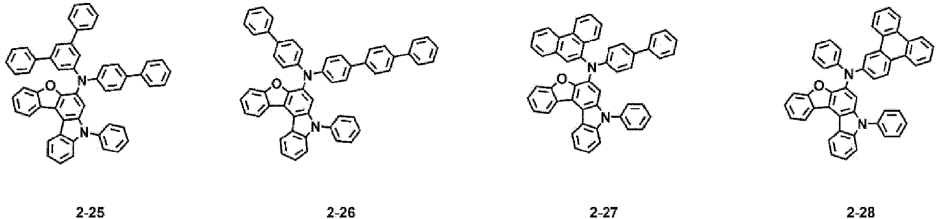

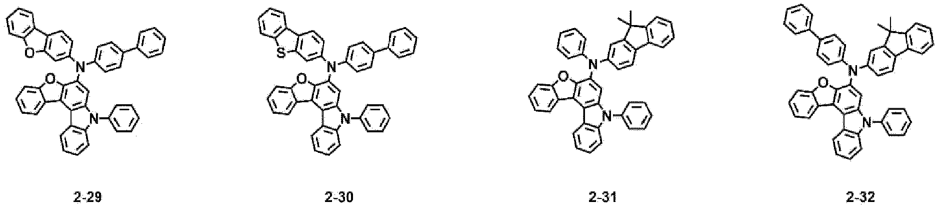

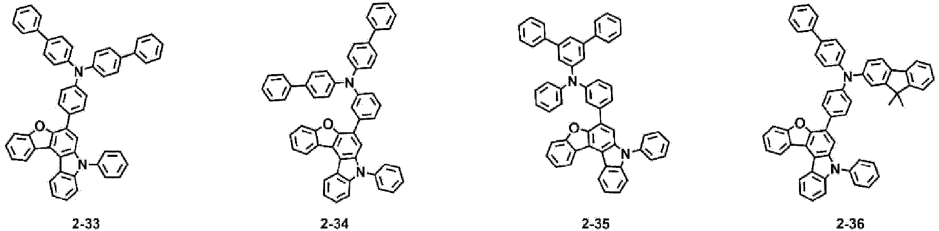

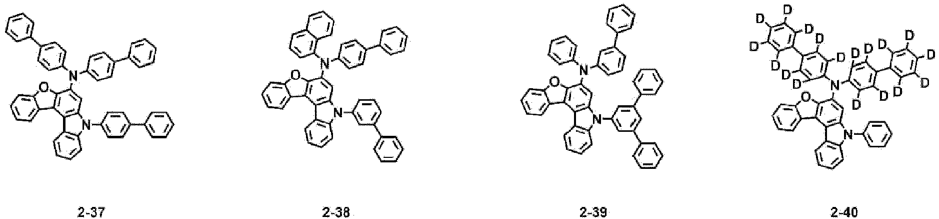

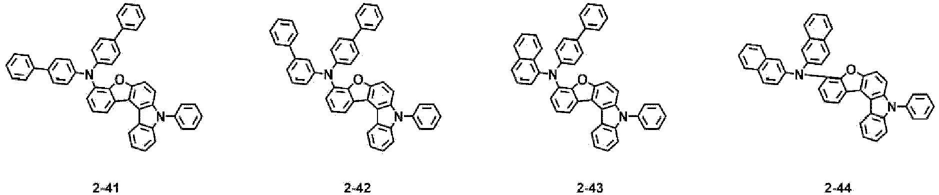

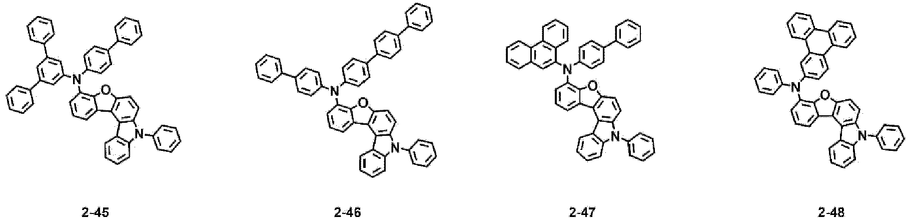

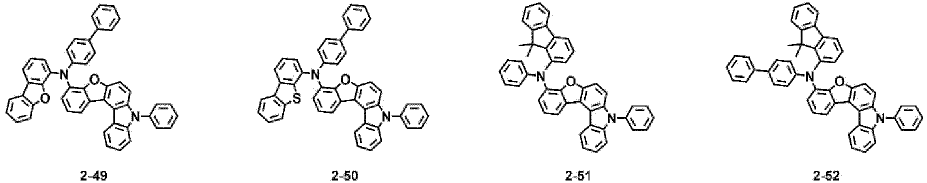

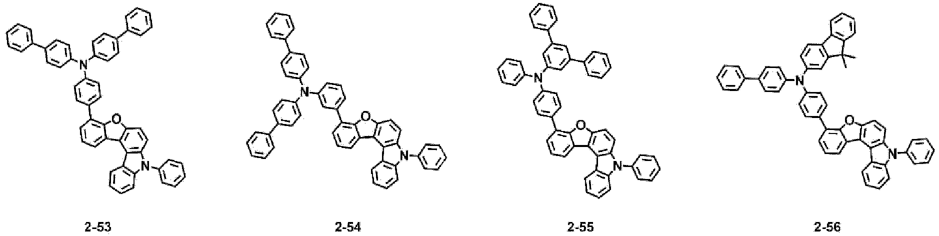

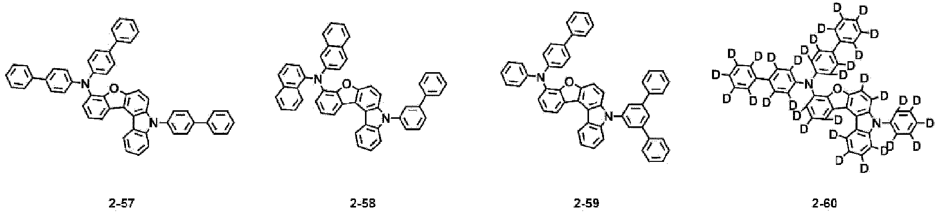

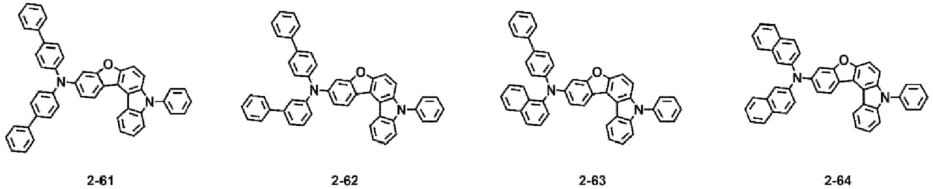

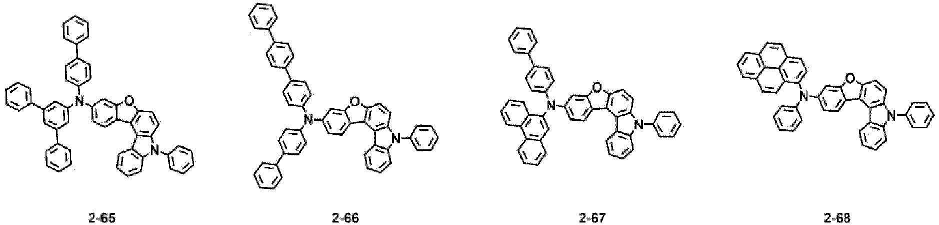

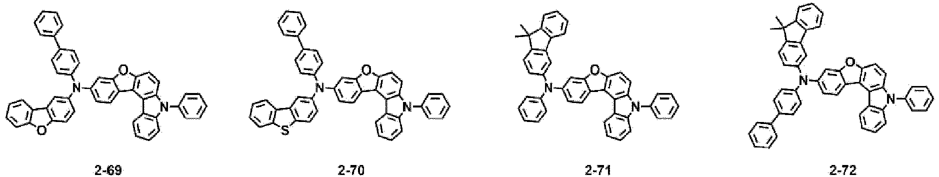

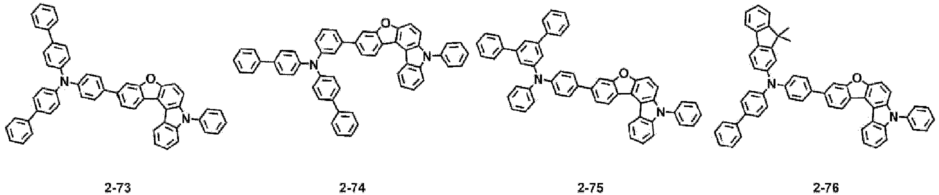

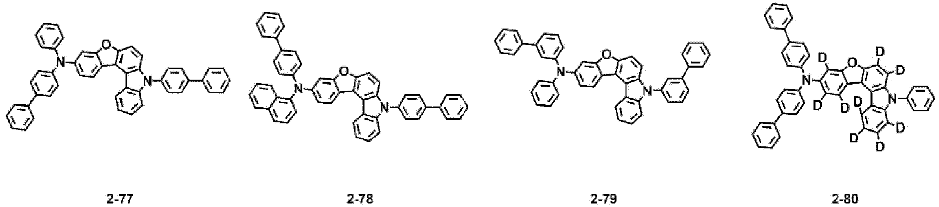

6. An organic light emitting device comprising a first electrode, a second electrode, and an organic material layer having one or more layers provided between the first electrode and the second electrode, wherein one or more layers of the organic material layer comprise the heterocyclic compound according to any one of claims 1 to 5.

7. The organic light emitting device of claim 6, wherein the organic material layer comprises a hole transport layer having one or more layers, and the hole transport layer comprises the heterocyclic compound represented by any one of Chemical Formulae 2 to 9.

8. The organic light emitting device of claim 6, wherein the organic material layer further comprises one layer or two or more layers selected from the group consisting of a light emitting layer, a hole injection layer, a hole transport layer, an electron injection layer, an electron transport layer, a hole auxiliary layer and a hole blocking layer.

## Patentansprüche

1. Heterocyclische Verbindung, dargestellt durch eine der folgenden chemischen Formeln 2 bis 9: 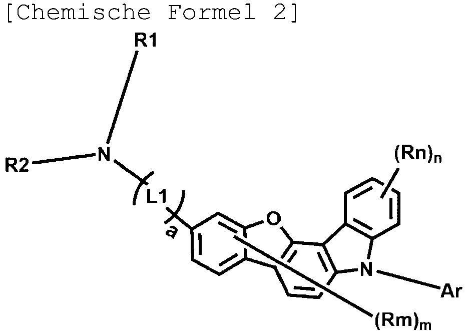 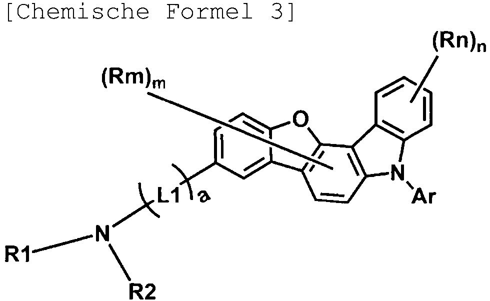 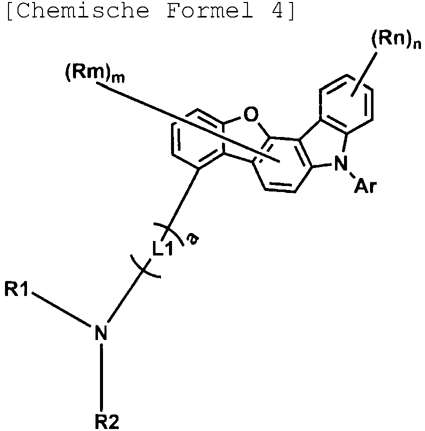 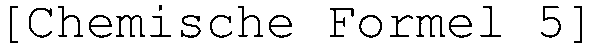 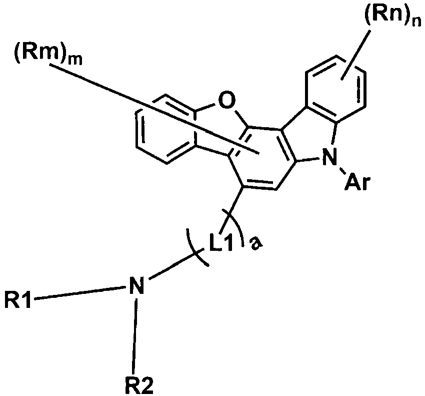 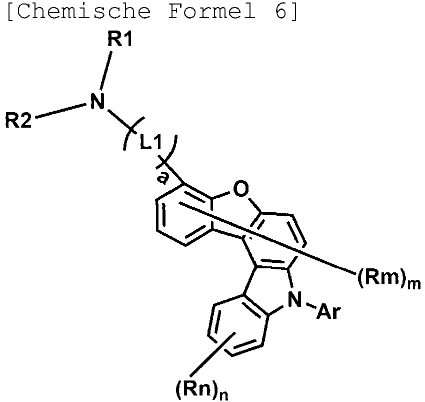 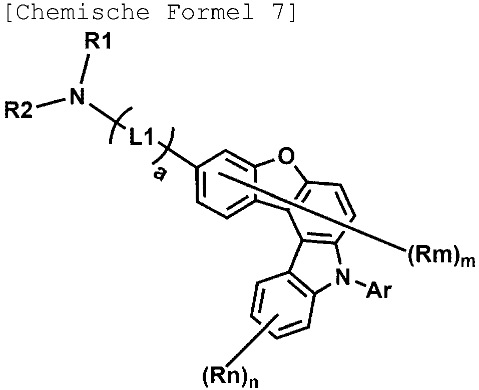 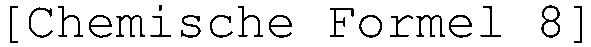 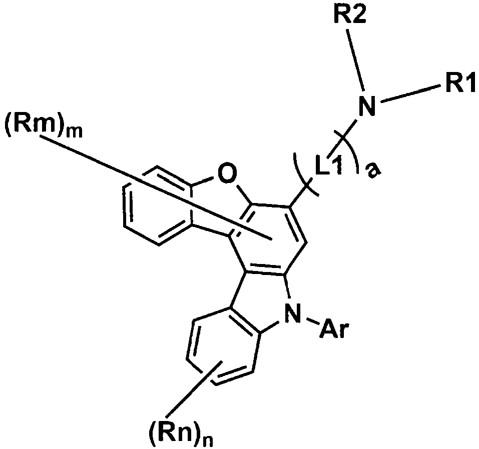 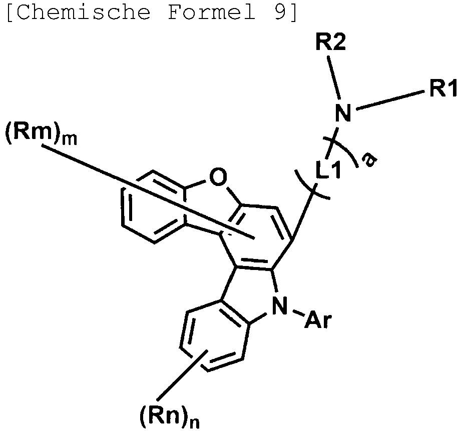 wobei in den chemischen Formeln 2 bis 9
L1 eine direkte Bindung oder eine substituierte oder unsubstituierte Arylengruppe mit 6 bis 60 Kohlenstoffatomen ist, a eine ganze Zahl von 0 bis 3 ist und, wenn a 2 oder höher ist, die L1 in den Klammern gleich oder verschieden voneinander sind,
R1 eine Phenylgruppe, unsubstituiert oder substituiert mit einem oder mehreren Deuterium(en); eine Biphenylgruppe, unsubstituiert oder substituiert mit einem oder mehreren Deuterium(en); eine Terphenylgruppe, unsubstituiert oder substituiert mit einem oder mehreren Deuterium(en); eine Naphthylgruppe, unsubstituiert oder substituiert mit einem oder mehreren Deuterium(en); eine Phenanthrengruppe, unsubstituiert oder substituiert mit einem oder mehreren Deuterium(en); eine Pyrengruppe; eine Fluorengruppe, unsubstituiert oder substituiert mit einem oder mehreren Substituenten, ausgewählt aus der Gruppe bestehend aus Deuterium und einer Methylgruppe; eine Triphenylenylgruppe, unsubstituiert oder substituiert mit einem oder mehreren Deuterium(en); eine Dibenzofurangruppe, unsubstituiert oder substituiert mit einem oder mehreren Deuterium(en); oder eine Dibenzothiophengruppe, unsubstituiert oder substituiert mit einem oder mehreren Deuterium(en), ist,
R2 eine Phenylgruppe, unsubstituiert oder substituiert mit einem oder mehreren Deuterium(en); eine Biphenylgruppe, unsubstituiert oder substituiert mit einem oder mehreren Deuterium(en); eine Terphenylgruppe, unsubstituiert oder substituiert mit einem oder mehreren Deuterium(en); eine Naphthylgruppe, unsubstituiert oder substituiert mit einem oder mehreren Deuterium(en); eine Phenanthrengruppe, unsubstituiert oder substituiert mit einem oder mehreren Deuterium(en); eine Dibenzofurangruppe, unsubstituiert oder substituiert mit einem oder mehreren Deuterium(en); oder eine Dibenzothiophengruppe, unsubstituiert oder substituiert mit einem oder mehreren Deuterium(en), ist,
Rm und Rn gleich oder verschieden voneinander sind und jeweils unabhängig Wasserstoff oder Deuterium sind,
m eine ganze Zahl von 0 bis 5 ist und, wenn m 2 oder höher ist, die Rm in den Klammern gleich oder verschieden voneinander sind,
n eine ganze Zahl von 0 bis 4 ist und, wenn n 2 oder höher ist, die Rn in den Klammern gleich oder verschieden voneinander sind und
Ar eine substituierte oder unsubstituierte Arylgruppe mit 6 bis 60 Kohlenstoffatomen ist.

2. Heterocyclische Verbindung nach Anspruch 1, wobei ein Deuteriumgehalt der heterocyclischen Verbindung, dargestellt durch eine der chemischen Formeln 2 bis 9, 10% bis 100% beträgt.

3. Heterocyclische Verbindung nach Anspruch 1, wobei Ar eine substituierte oder unsubstituierte Arylgruppe mit 6 bis 40 Kohlenstoffatomen ist.

4. Heterocyclische Verbindung nach Anspruch 1, wobei L1 eine direkte Bindung oder eine substituierte oder unsubstituierte Arylengruppe mit 6 bis 40 Kohlenstoffatomen ist.

5. Heterocyclische Verbindung nach Anspruch 1, wobei die heterocyclische Verbindung durch eine der folgenden Verbindungen dargestellt ist:

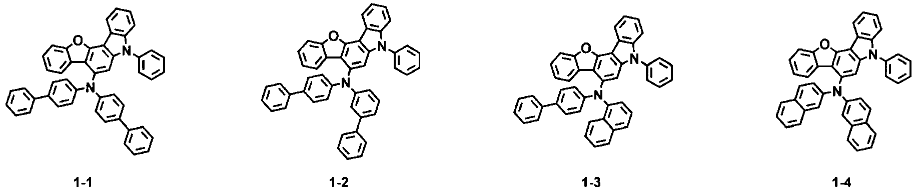

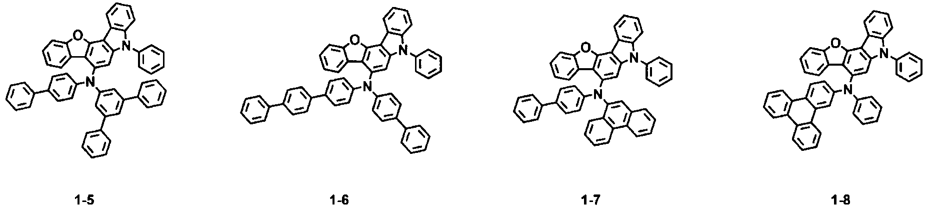

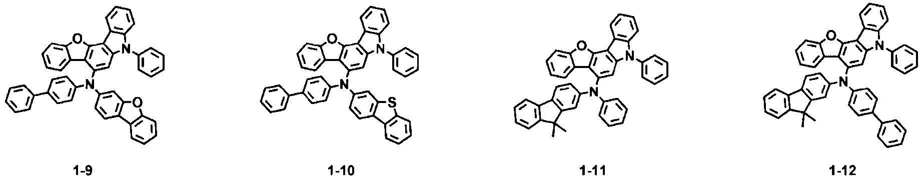

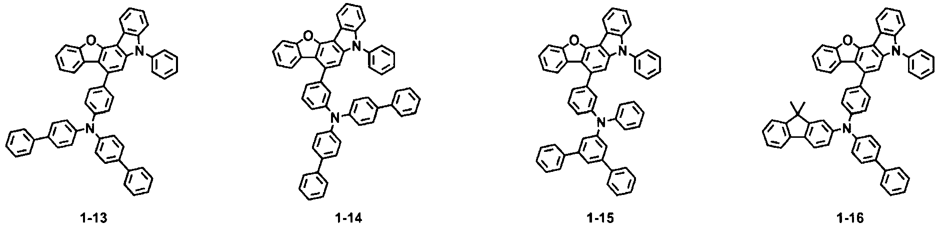

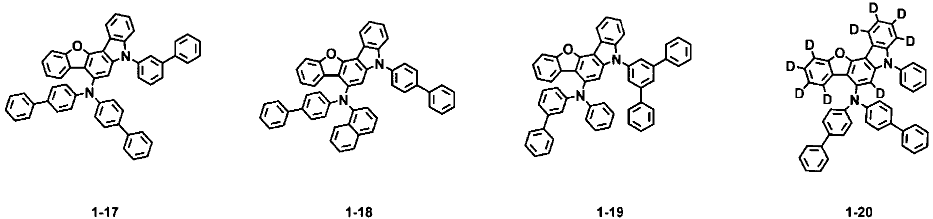

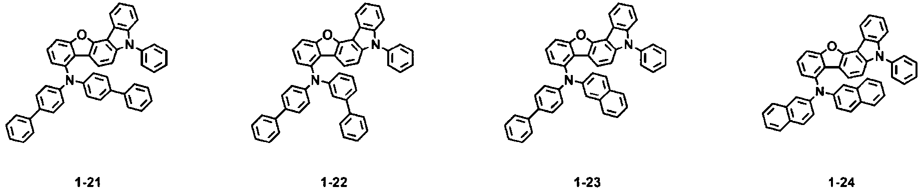

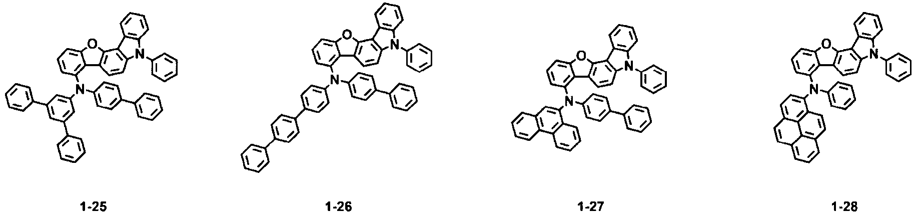

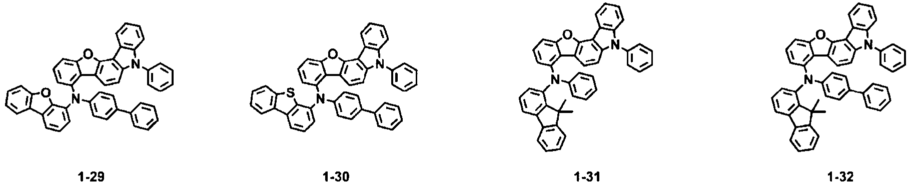

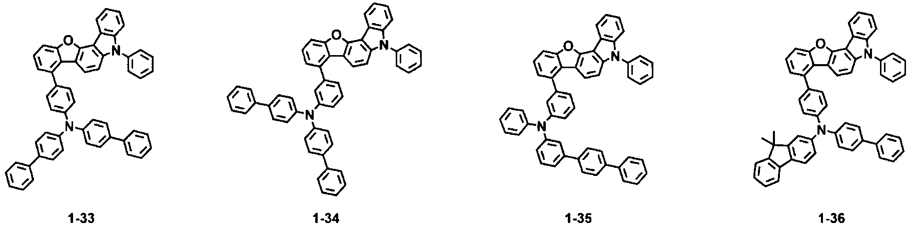

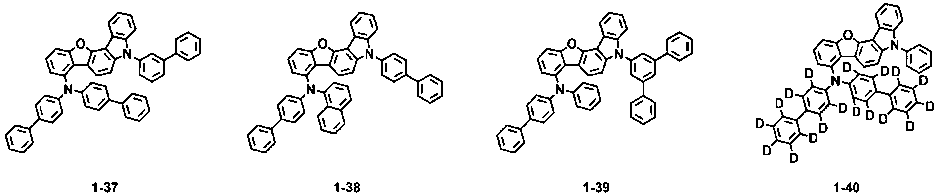

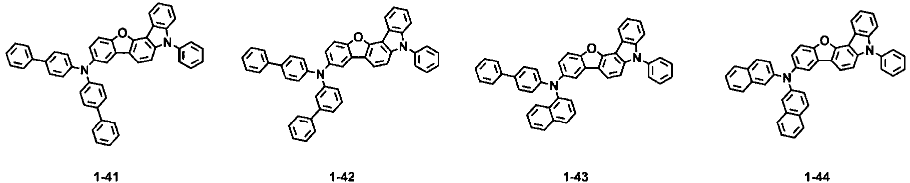

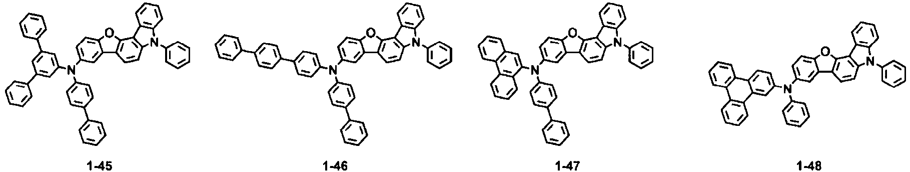

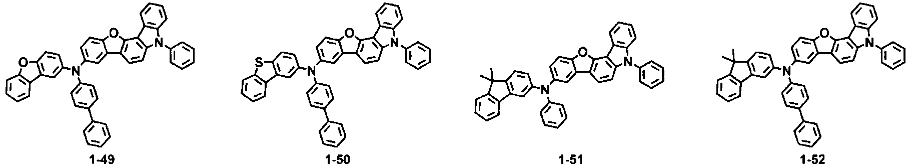

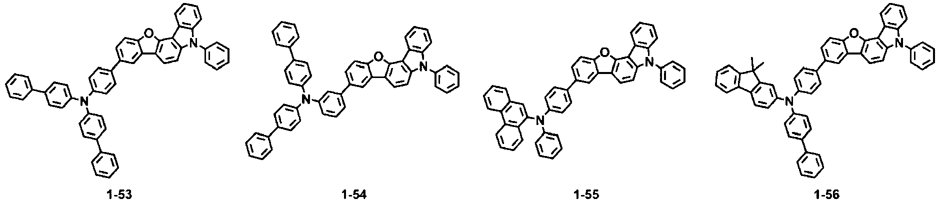

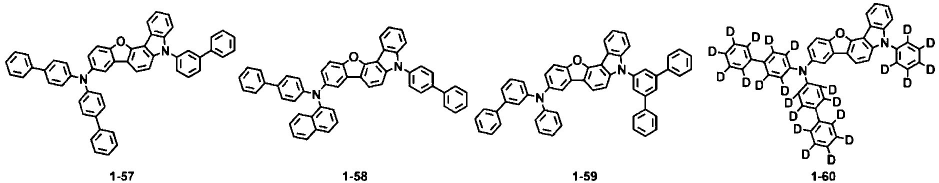

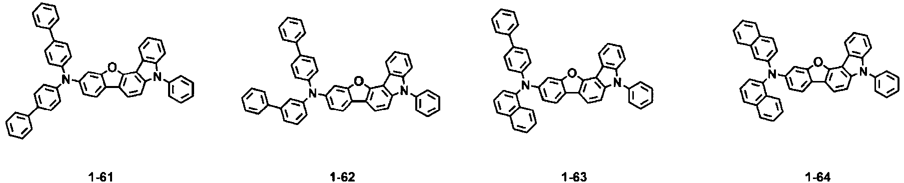

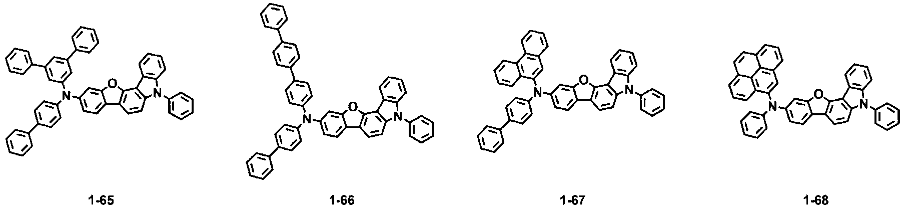

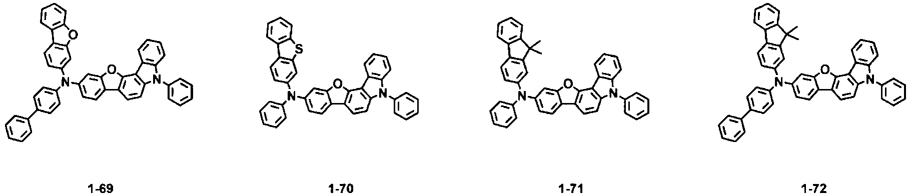

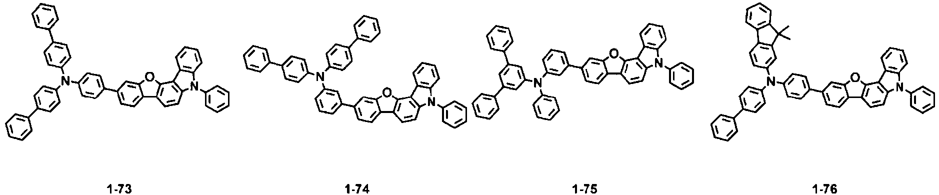

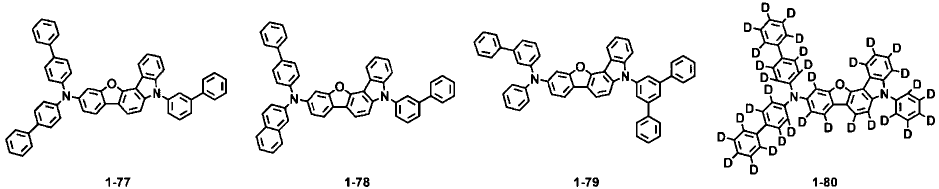

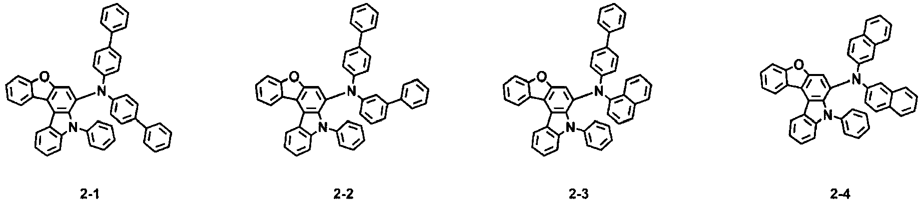

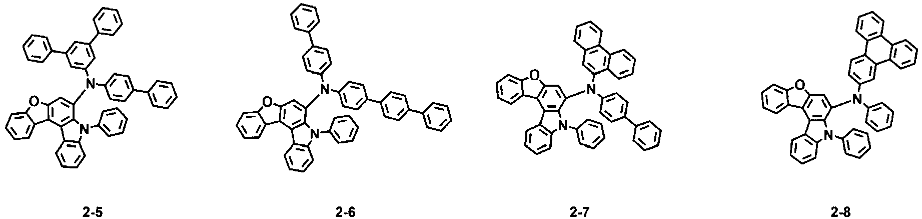

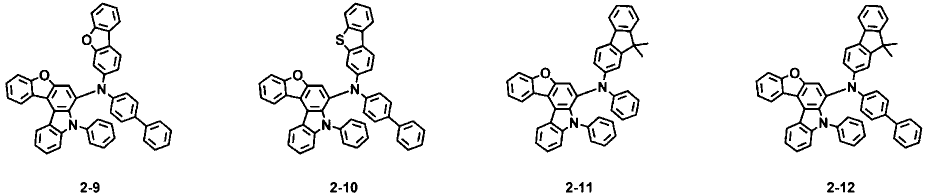

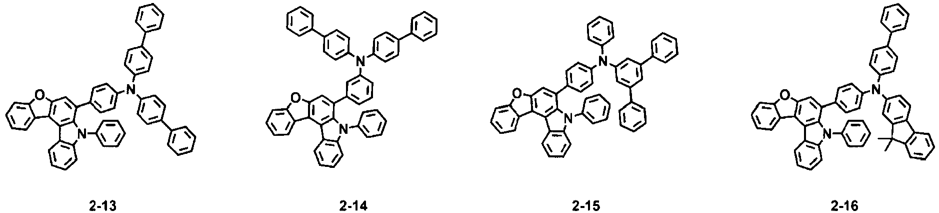

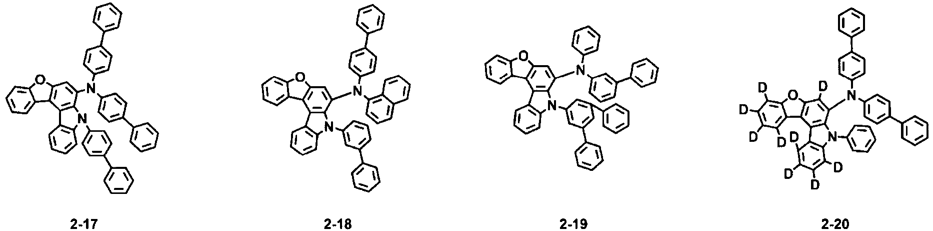

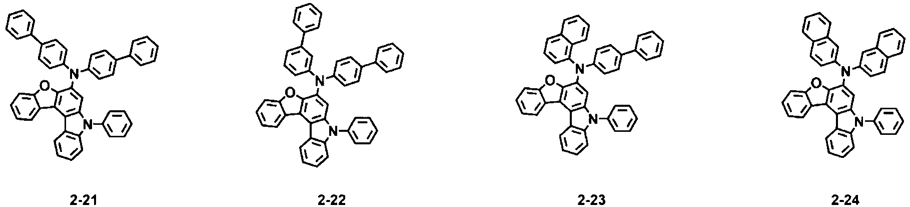

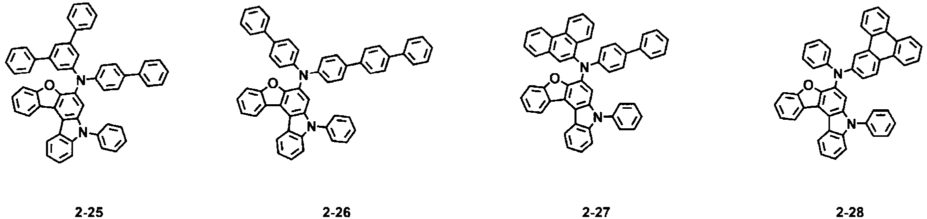

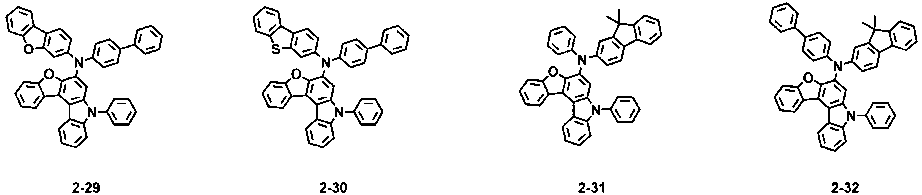

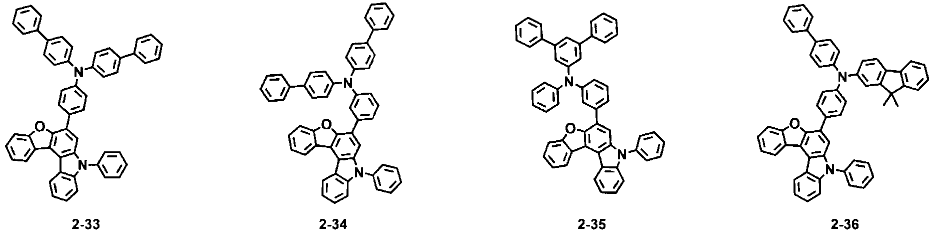

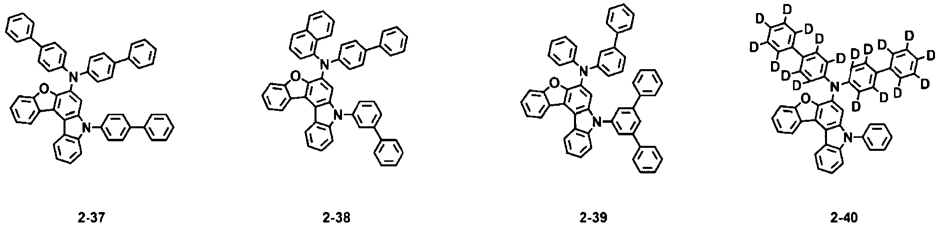

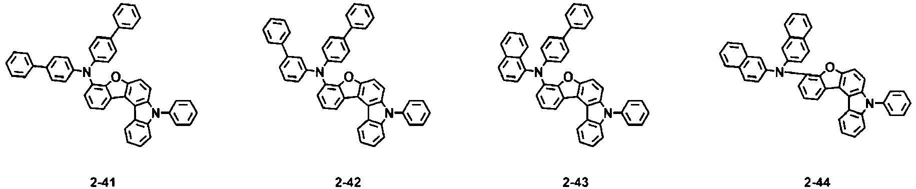

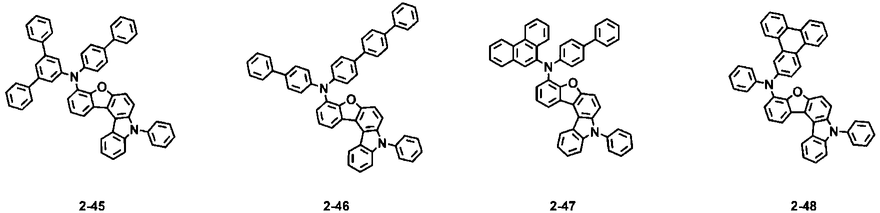

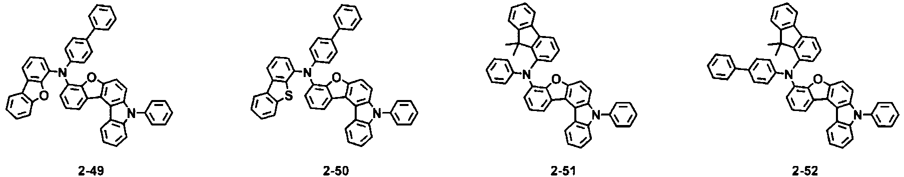

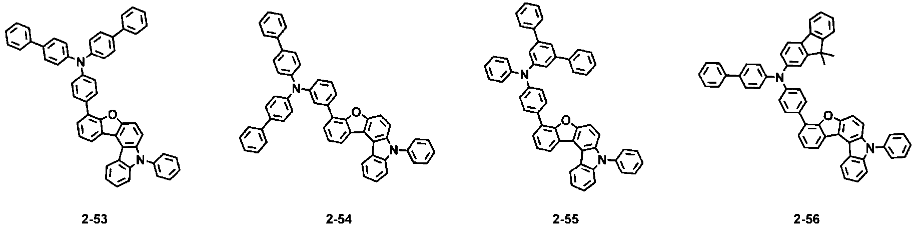

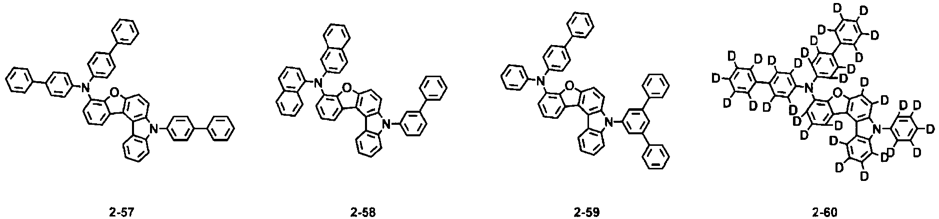

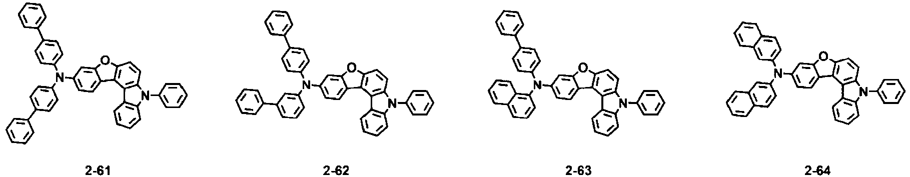

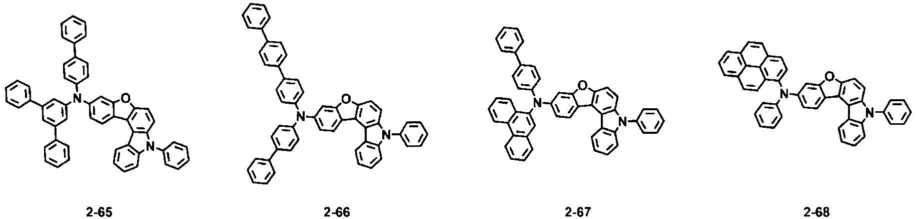

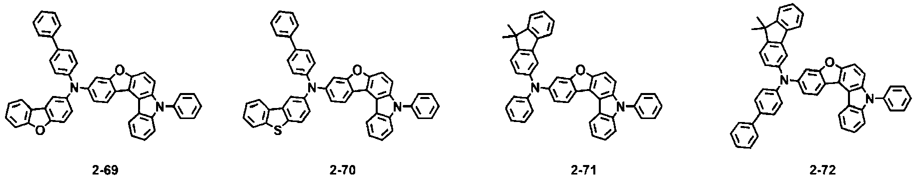

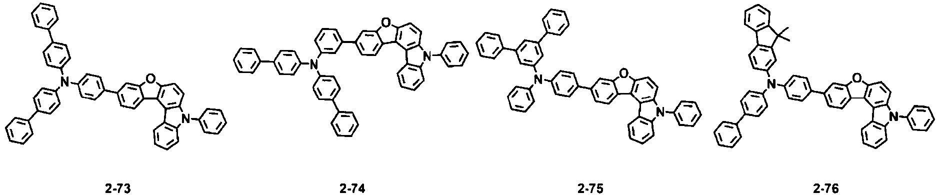

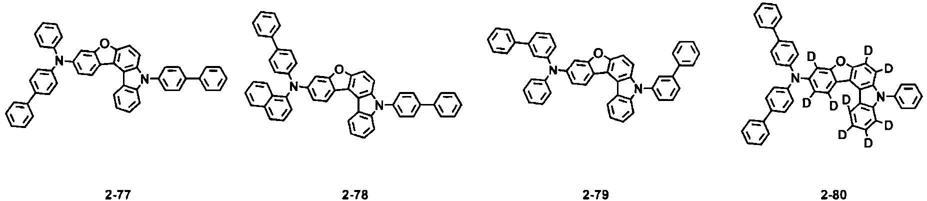

6. Organische lichtemittierende Vorrichtung, umfassend eine erste Elektrode, eine zweite Elektrode und eine organische Materialschicht mit einer oder mehreren Schichten, die zwischen der ersten Elektrode und der zweiten Elektrode bereitgestellt sind, wobei eine oder mehrere Schichten der organischen Materialschicht die heterocyclische Verbindung nach einem der Ansprüche 1 bis 5 umfassen.

7. Organische lichtemittierende Vorrichtung nach Anspruch 6, wobei die organische Materialschicht eine Lochtransportschicht mit einer oder mehreren Schichten umfasst und die Lochtransportschicht die heterocyclische Verbindung, dargestellt durch eine der chemischen Formeln 2 bis 9, umfasst.

8. Organische lichtemittierende Vorrichtung nach Anspruch 6, wobei die organische Materialschicht ferner eine Schicht oder zwei oder mehr Schichten, ausgewählt aus der Gruppe bestehend aus einer lichtemittierenden Schicht, einer Lochinjektionsschicht, einer Lochtransportschicht, einer Elektroneninjektionsschicht, einer Elektronentransportschicht, einer Lochhilfsschicht und einer Lochblockierschicht, umfasst.

## Revendications

1. Composé hétérocyclique représenté par l'une quelconque des Formules chimiques 2 à 9 ci-après : 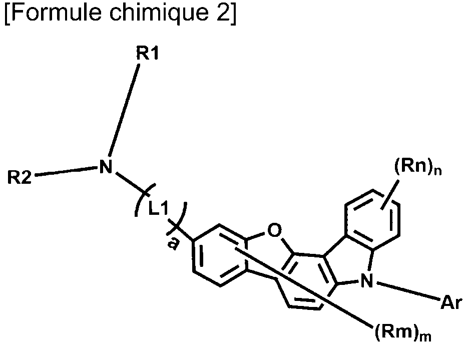 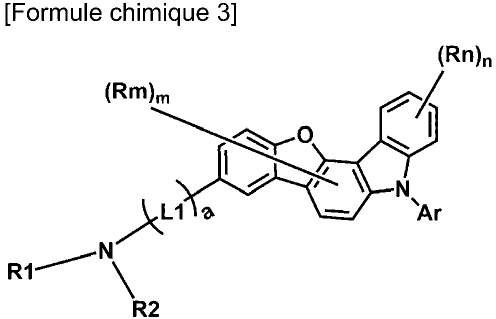 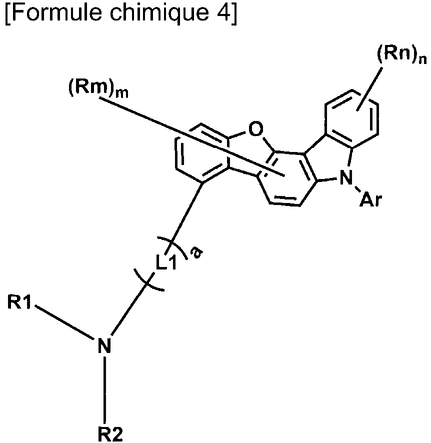 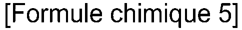 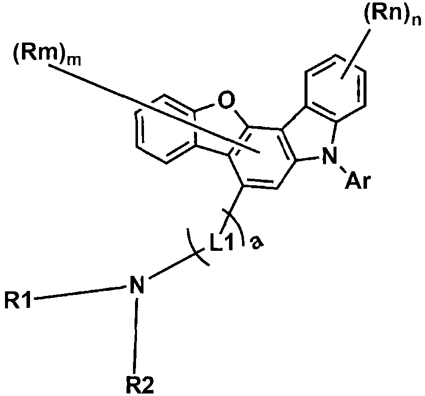 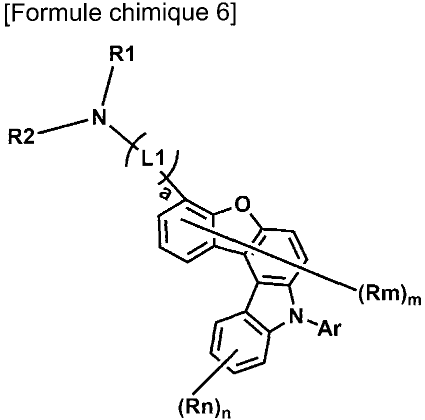 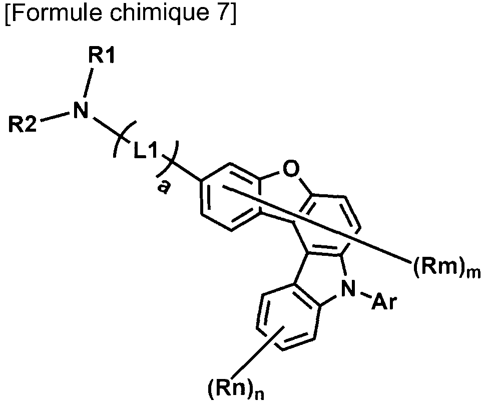 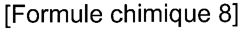 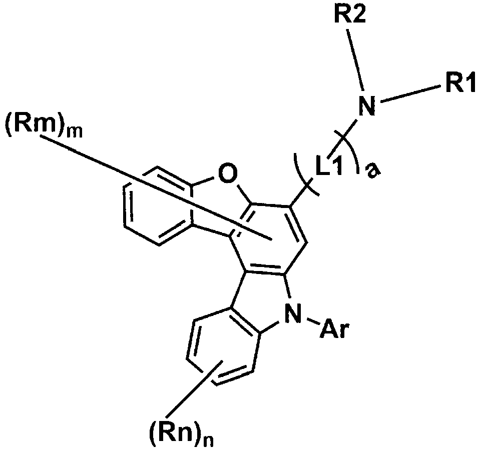 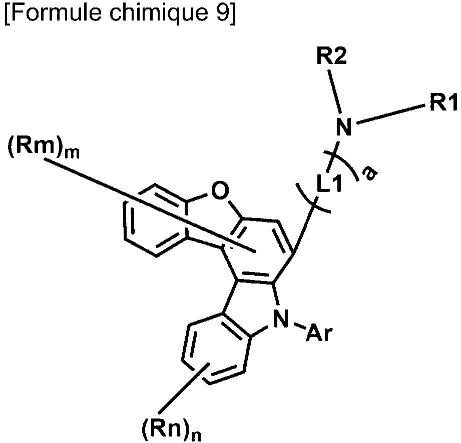 dans lequel, dans les Formules chimiques 2 à 9,
L1 est une liaison directe ; ou un groupe arylène non substitué ou substitué ayant 6 à 60 atomes de carbone, a est un nombre entier de 0 à 3 et, quand a est égal ou supérieur à 2, les L1 entre parenthèses sont identiques ou différents l'un de l'autre,
R1 est un groupe phényle non substitué ou substitué par un ou plusieurs deutériums ; un groupe biphényle non substitué ou substitué par un ou plusieurs deutériums ; un groupe terphényle non substitué ou substitué par un ou plusieurs deutériums ; un groupe naphtyle non substitué ou substitué par un ou plusieurs deutériums ; un groupe phénanthrène non substitué ou substitué par un ou plusieurs deutériums ; un groupe pyrène ; un groupe fluorène non substitué ou substitué par un ou plusieurs substituants sélectionnés dans le groupe constitué d'un deutérium et un groupe méthyle ; un groupe triphénylényle non substitué ou substitué par un ou plusieurs deutériums ; un groupe dibenzofurane non substitué ou substitué par un ou plusieurs deutériums ; ou un groupe dibenzothiophène non substitué ou substitué par un ou plusieurs deutériums,
R2 est un groupe phényle non substitué ou substitué par un ou plusieurs deutériums ; un groupe biphényle non substitué ou substitué par un ou plusieurs deutériums ; un groupe terphényle non substitué ou substitué par un ou plusieurs deutériums ; un groupe naphtyle non substitué ou substitué par un ou plusieurs deutériums ; un groupe phénanthrène non substitué ou substitué par un ou plusieurs deutériums ; un groupe dibenzofurane non substitué ou substitué par un ou plusieurs deutériums ; ou un groupe dibenzothiophène non substitué ou substitué par un ou plusieurs deutériums,
Rm et Rn sont identiques ou différents l'un de l'autre, et sont chacun indépendamment un hydrogène ; ou un deutérium,
m est un nombre entier de 0 à 5 et, quand m est égal ou supérieur à 2, les Rm entre parenthèses sont identiques ou différents l'un de l'autre,
n est un nombre entier de 0 à 4 et, quand n est égal ou supérieur à 2, les Rn entre parenthèses sont identiques ou différents l'un de l'autre, et
Ar est un groupe aryle non substitué ou substitué ayant 6 à 60 atomes de carbone.

2. Composé hétérocyclique selon la revendication 1, dans lequel une teneur en deutérium du composé hétérocyclique représenté par l'une quelconque des Formules chimiques 2 à 9 est 10 % à 100 %.

3. Composé hétérocyclique selon la revendication 1, dans lequel Ar est un groupe aryle non substitué ou substitué ayant 6 à 40 atomes de carbone.

4. Composé hétérocyclique selon la revendication 1, dans lequel L1 est une liaison directe ; ou un groupe arylène non substitué ou substitué ayant 6 à 40 atomes de carbone.

5. Composé hétérocyclique selon la revendication 1, dans lequel le composé hétérocyclique est représenté par l'un quelconque des composés ci-après :

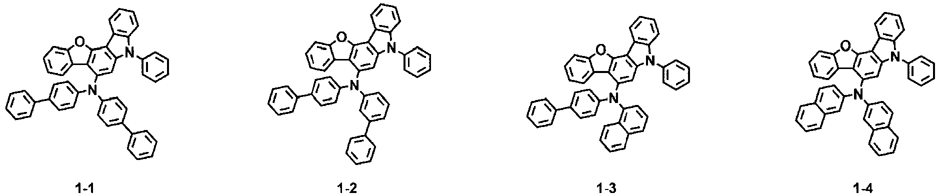

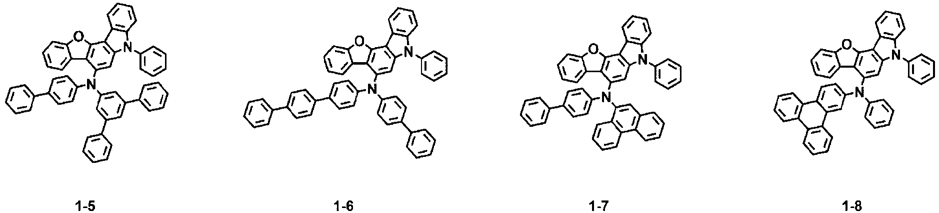

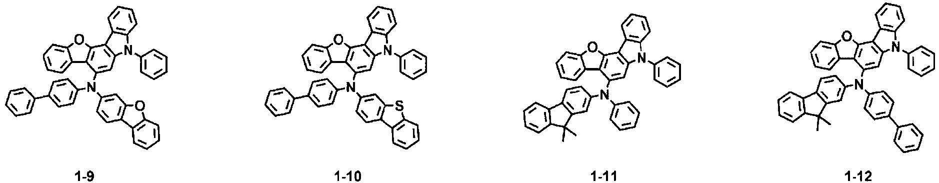

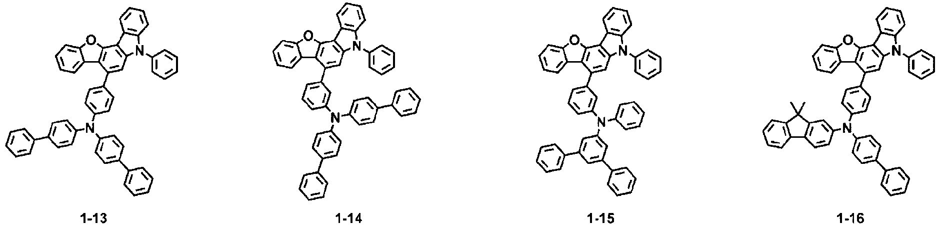

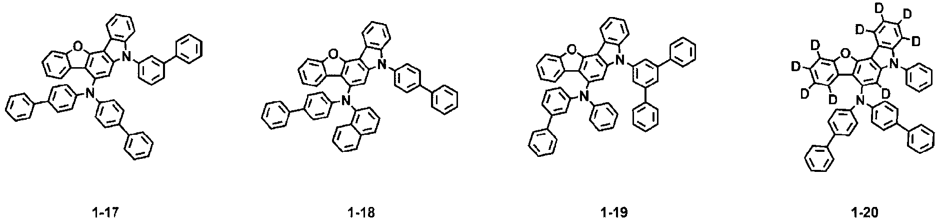

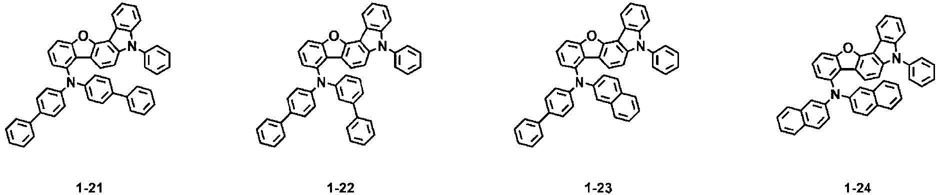

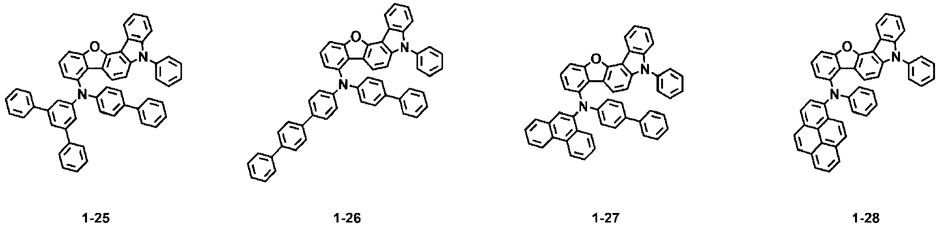

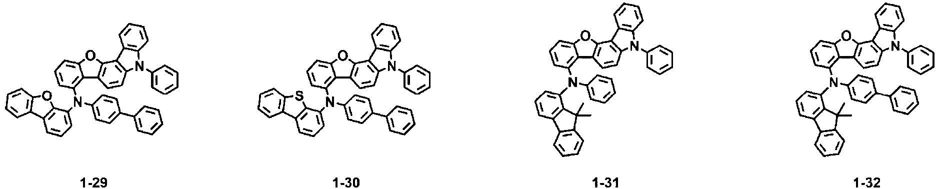

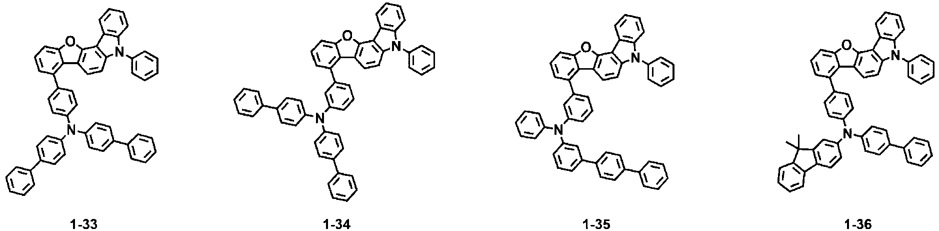

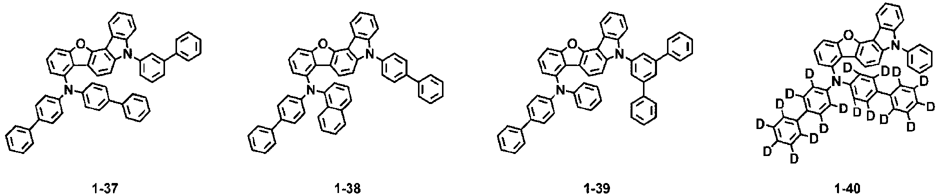

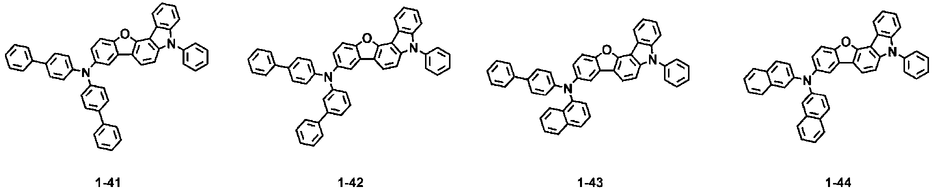

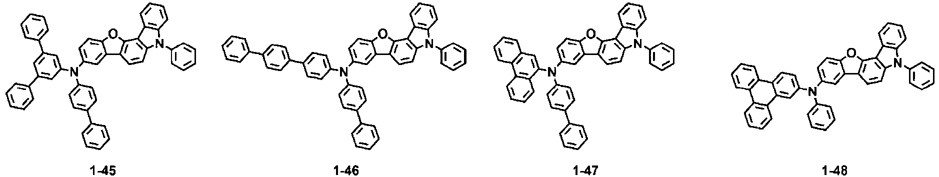

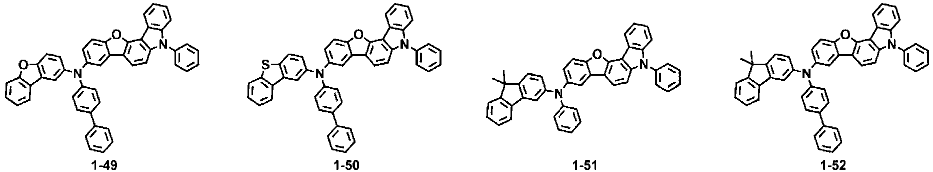

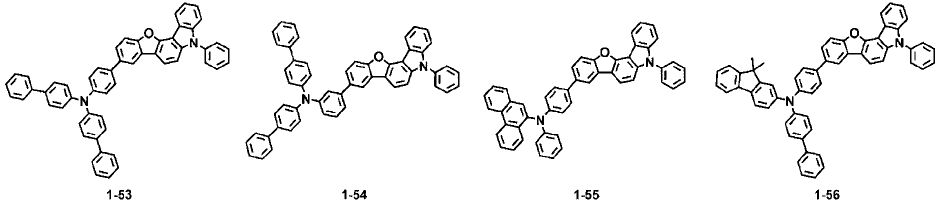

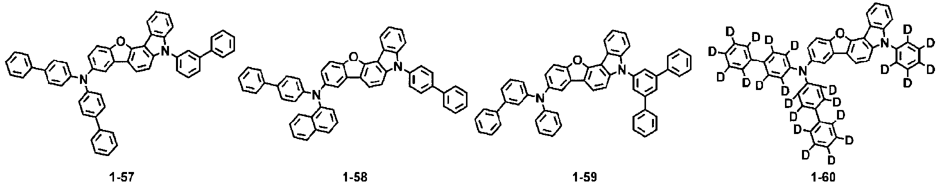

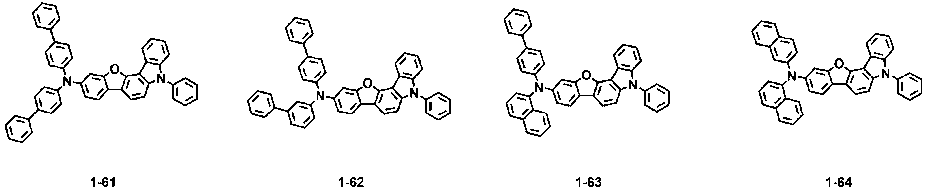

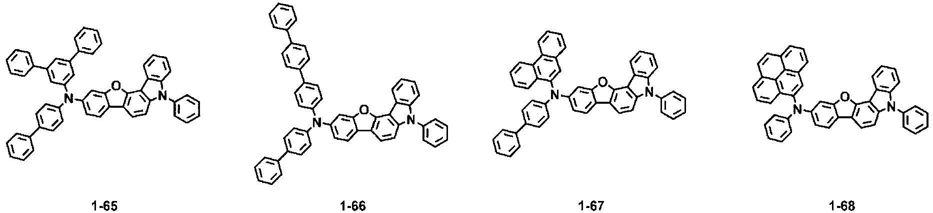

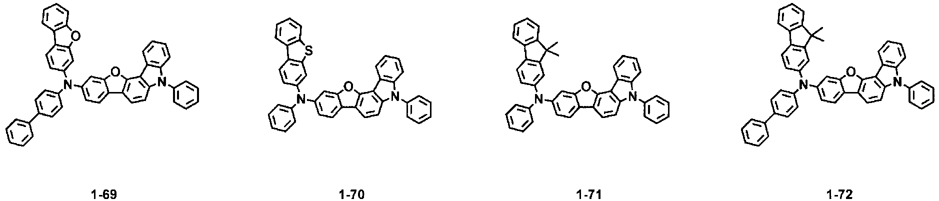

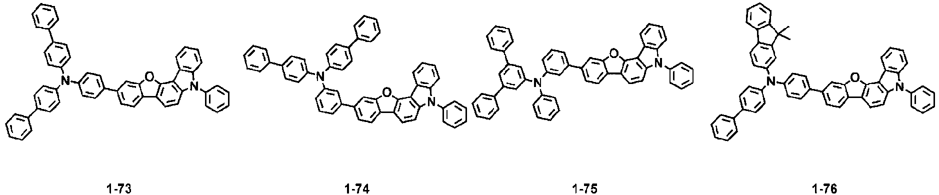

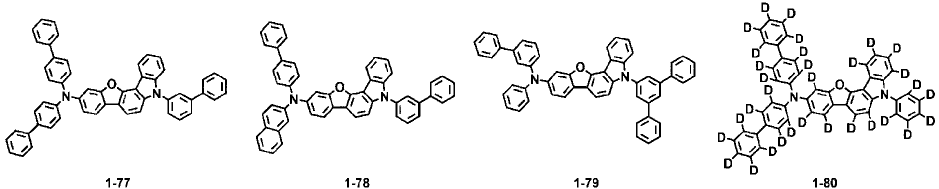

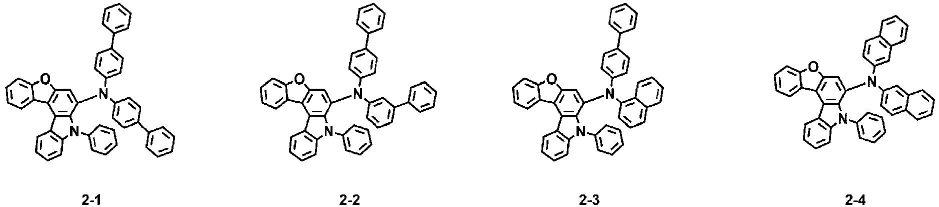

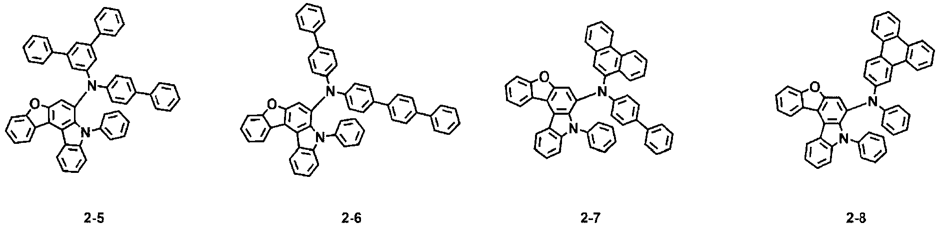

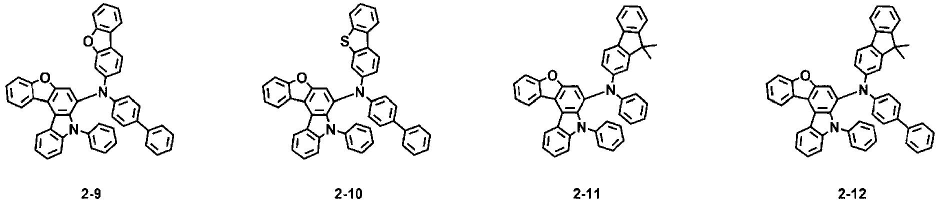

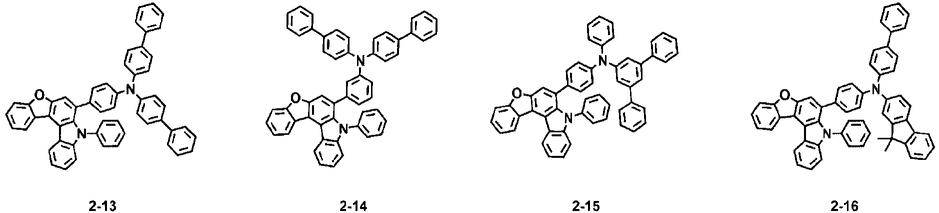

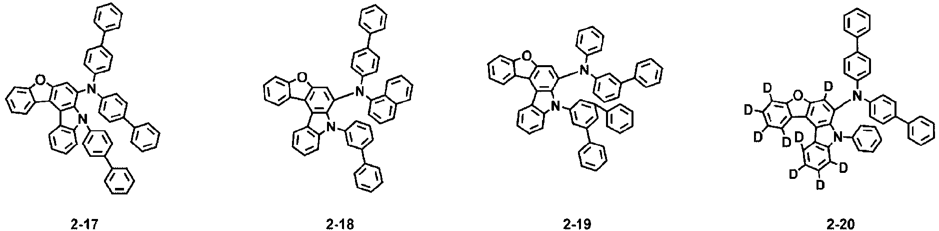

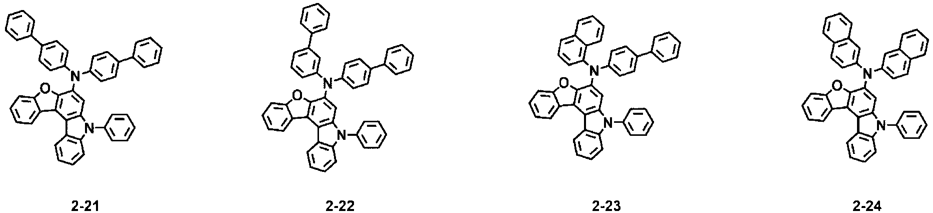

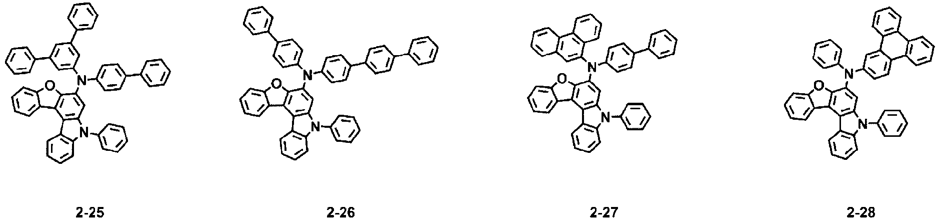

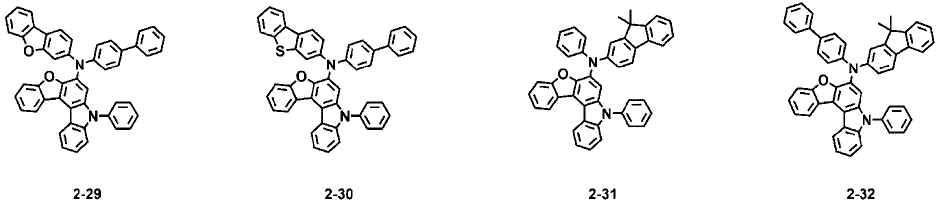

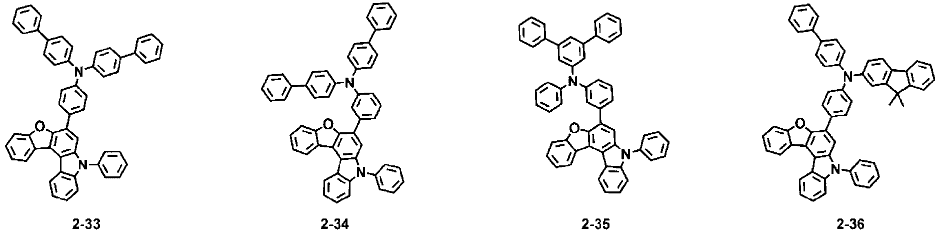

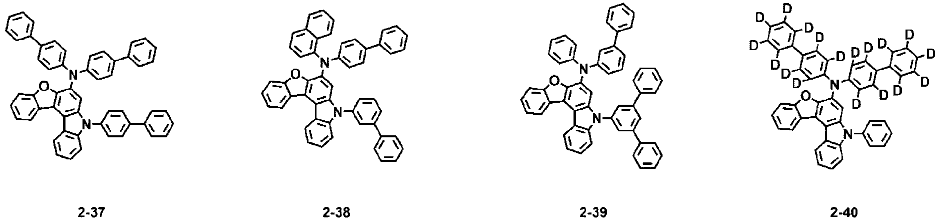

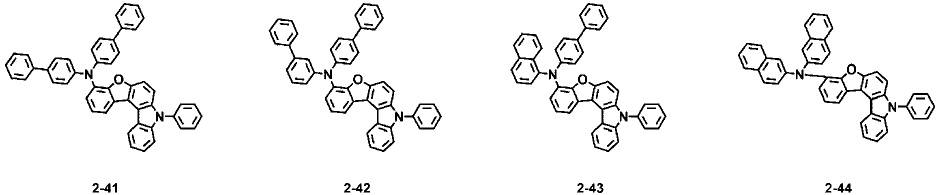

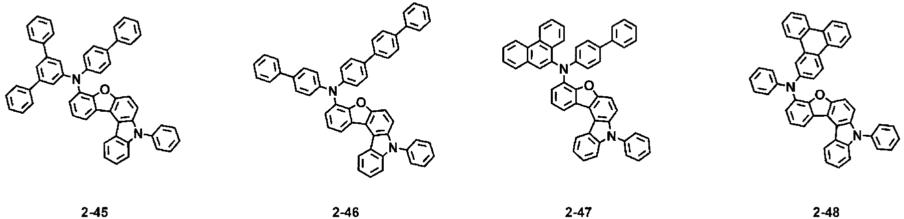

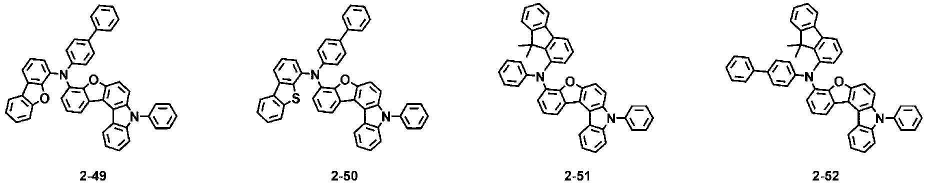

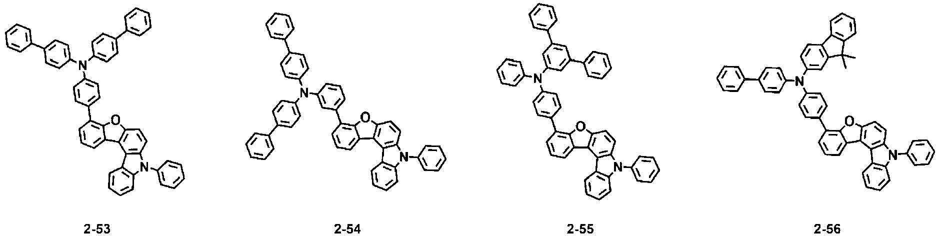

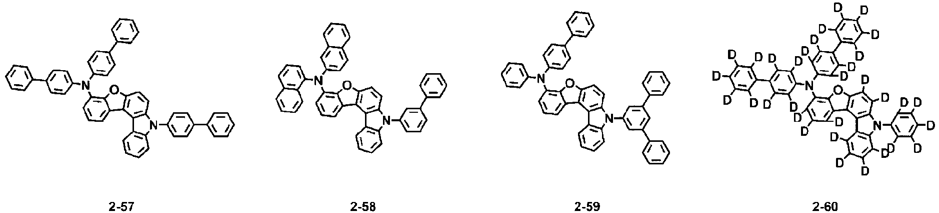

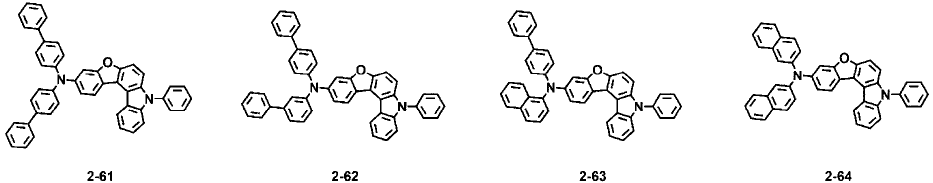

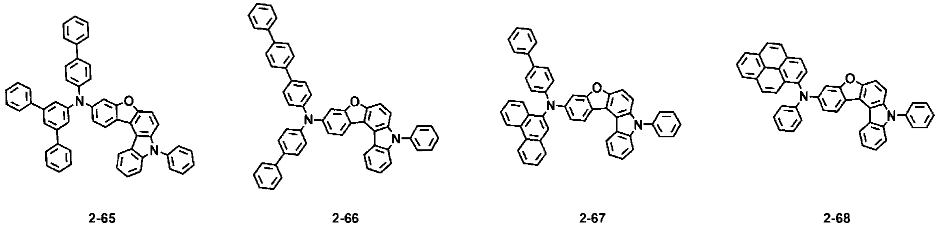

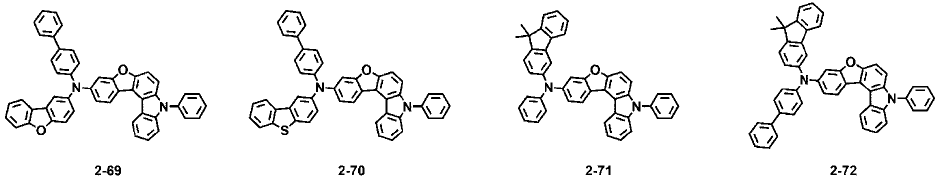

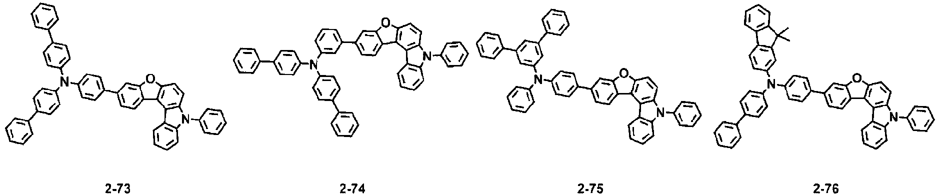

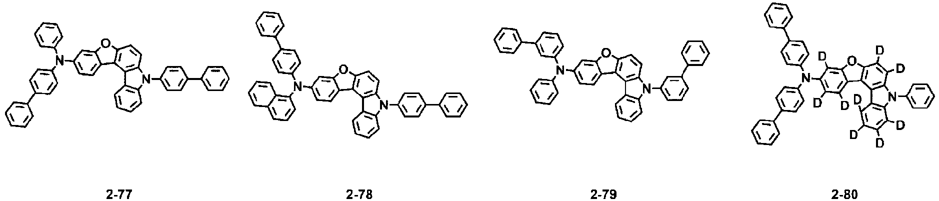

6. Dispositif électroluminescent organique comprenant une première électrode, une deuxième électrode et une couche de matériau organique ayant une ou plusieurs couches fournies entre la première électrode et la deuxième électrode, dans lequel une ou plusieurs couches de la couche de matériau organique comprennent le composé hétérocyclique selon l'une quelconque des revendications 1 à 5.

7. Dispositif électroluminescent organique selon la revendication 6, dans lequel la couche de matériau organique comprend une couche de transfert de trous ayant une ou plusieurs couches, et la couche de transfert de trous comprend le composé hétérocyclique représenté par l'une quelconque des Formules chimiques 2 à 9.

8. Dispositif électroluminescent organique selon la revendication 6, dans lequel la couche de matériau comprend en outre une ou deux ou plus de deux couches sélectionnées dans le groupe constitué d'une couche électroluminescente, une couche d'injection de trous, une couche de transfert de trous, une couche d'injection d'électrons, une couche de transfert d'électrons, une couche de trous auxiliaire et une couche de blocage de trous.
